(19) Europäisches Patentamt
European Patent Office
Office européen des brevets

(11) **EP 3 064 133 B1**

(12) **EUROPEAN PATENT SPECIFICATION**

(45) Date of publication and mention
of the grant of the patent:
**13.03.2019 Bulletin 2019/11**

(51) Int Cl.:
***A61B 5/11*** *(2006.01)*    *A61B 5/00* *(2006.01)*

(21) Application number: **16153287.4**

(22) Date of filing: **29.01.2016**

(54) **SPECIFYING APPARATUS AND SPECIFYING METHOD**

SPEZIFIZIERUNGSVORRICHTUNG UND SPEZIFIZIERUNGSVERFAHREN

APPAREIL DE SPÉCIFICATION ET PROCÉDÉ DE SPÉCIFICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.03.2015 JP 2015040412**

(43) Date of publication of application:
**07.09.2016 Bulletin 2016/36**

(73) Proprietor: **FUJITSU LIMITED
Kawasaki-shi,
Kanagawa 211-8588 (JP)**

(72) Inventors:
• **SASAMOTO, Yuki
Kawasaki-shi, Kanagawa 211-8588 (JP)**
• **INOMATA, Akihiro
Kawasaki-shi, Kanagawa 211-8588 (JP)**
• **HOTTA, Shinji
Kawasaki-shi, Kanagawa 211-8588 (JP)**
• **MAEDA, Kazuho
Kawasaki-shi, Kanagawa 211-8588 (JP)**

(74) Representative: **Haseltine Lake LLP
Lincoln House, 5th Floor
300 High Holborn
London WC1V 7JH (GB)**

(56) References cited:
**WO-A1-2011/055255    WO-A1-2013/001411
WO-A1-2013/030703    US-A1- 2010 010 392
US-A1- 2011 245 629    US-A1- 2014 330 172
US-B1- 8 206 325**

**Description**

FIELD

The embodiment discussed herein is related to a measuring apparatus and a measuring method.

BACKGROUND

A technology in which data indicating a movement of a body is acquired by a sensor and pattern matching is performed between a definition for a value indicated by a parameter in a case of a certain posture or movement such as walking or sitting, and the acquired data, and thus a posture or a movement is determined has been known,. For example, the technology is disclosed in Japanese Laid-open Patent Publication No. 2011-8612, Japanese Laid-open Patent Publication No. 2012-113753, International Publication Pamphlet No. WO 2013/046510, Japanese Laid-open Patent Publication No. 2014-44510, Japanese Laid-open Patent Publication No. 2005-304942, Japanese Laid-open Patent Publication No. 2014-36764, "Real-world Gyroscope-based Gait Event Detection and Gait Feature Extraction", Fraccaro, et al., eTELEMED 2014: The Sixth International Conference on eHealth, Telemedicine, and Social Medicine, "Analysis of gait initiation action of the young and the elderly", SHIMAMURA, et al., Congress of the Japanese Physical Therapy Association 2012(0), 48101585-48101585, 2013, Japanese Physical Therapy Association. Reference may be made to US 2014/0330172 A1, which relates to systems and methods for automatically quantifying mobility, and to WO 2011/055255 A1, which relates to revoking a fall alarm of a user after the fall is detected.

SUMMARY

TECHNICAL PROBLEM

In the method of the related art, a movement and the like having a specific pattern may be determined. However, it is difficult to specify a transitional period when transition to the movement or the posture is performed, or a transitional period when transition from a movement or a posture to another movement or another posture is performed.

According to an embodiment of one aspect of the invention, it is desirable to specify a transitional period when transition to a predetermined movement or posture is performed, or a transitional period when transition from a predetermined movement or posture to another movement or posture is performed.

SOLUTION TO PROBLEM

The present invention is defined by the independent claims, to which reference should now be made. Specific embodiments are defined in the dependent claims.

ADVANTAGEOUS EFFECTS OF INVENTION

According to the technology discussed in an embodiment of one aspect of the invention, it is possible to specify a transitional period when transition to a predetermined movement or posture is performed, or a transitional period when transition from a predetermined movement or posture to another movement or posture is performed.

BRIEF DESCRIPTION OF DRAWINGS

The invention is described, by way of example only, with reference to the following drawings, in which:

FIG. 1 is a diagram illustrating the entire configuration of a measurement system which includes a measuring apparatus according to an example;
FIG. 2 is a diagram illustrating a form in which a gyro sensor is attached;
FIG. 3 is a diagram illustrating an example of time series data of the gyro sensor.
FIG. 4 is a diagram illustrating a transitional state.
FIGs. 5A and 5B are diagrams illustrating examples of time series data of the sensor 2 when the body has a stationary posture and when the body is in a gait stable state.
FIG. 6 is a diagram illustrating an example of a relationship between time series data of the sensor 2 obtained during walking and a movement of a person at this time;
FIG. 7 is a diagram illustrating a difference of characteristics between a plurality of periods which have different closeness to a period of a stable state;

FIG. 8 is a diagram illustrating a transitional period corresponding to a difference between a stable period and a second period;

FIG. 9 is a diagram illustrating a case of including only the transitional state before the stable state of gait;

FIG. 10 is a diagram illustrating a case of including an action other than the transitional state, before the stable state of the gait;

FIG. 11 is a diagram illustrating an example of a configuration of a measuring apparatus;

FIG. 12 is a diagram illustrating an example of a hardware configuration of a processing device of a measuring apparatus;

FIG. 13 is a flowchart illustrating an example of processing performed by the measuring apparatus 10 according to an example illustrated in FIG. 11;

FIG. 14 is a flowchart illustrating an example of a process of Step S1308 in FIG. 13;

FIGs. 15A and 15B are diagrams illustrating an example of a stable period detection algorithm;

FIG. 16 is a diagram illustrating an example in which a plurality of stable periods is detected;

FIG. 17 is a schematic diagram illustrating plural types of parameters;

FIG. 18 is a diagram illustrating an example of enumerated semi-stable periods;

FIG. 19 is a diagram illustrating an example of data in a semi-stable period selection index database 22;

FIGs. 20A to 20G are diagrams illustrating examples of a feature value;

FIG. 21 is a diagram illustrating an example of data (values of second threshold candidates) in a second threshold candidate database 21;

FIGs. 22A and 22B are diagrams illustrating examples of a period length of the semi-stable period for each eased value;

FIGs. 23A and 23B are diagrams illustrating examples of a period length of the semi-stable period for each eased value;

FIGs. 24A and 24B are diagrams illustrating examples of a period length of the semi-stable period for each eased value;

FIGs. 25A and 25B are diagrams illustrating examples of time series data of a sensor value stored in a sensor data storage unit 34;

FIG. 26 is a diagram illustrating an example of the stable period detection algorithm stored in a stable period detection algorithm database 20;

FIG. 27 is a diagram illustrating an example of the stable period detection algorithm stored in the stable period detection algorithm database 20;

FIG. 28 is a diagram illustrating a detection method of the stable state of the gait by the stable period detection unit 11;

FIG. 29 is a diagram illustrating a detection method of the stable state of the gait by the stable period detection unit 11;

FIGs. 30A and 30B are diagrams illustrating examples of the eased value relating to various parameters used in the stable period detection algorithm;

FIGs. 31A to 31C are diagram illustrating results obtained by detecting the semi-stable period and the period length thereof using the eased value relating to the various parameters;

FIG. 32 is a diagram illustrating an example of calculation results of an index value (evaluation value) of a monotonous increase tendency, which are obtained based on the detection results illustrated in FIGs. 31A to 31C;

FIG. 33 is a diagram illustrating an example of enumeration results of the semi-stable period by a semi-stable period enumeration unit;

FIG. 34 is a diagram illustrating an example of a selection method by a semi-stable period selection unit;

FIG. 35 is a diagram illustrating another example of the selection method by the semi-stable period selection unit;

FIG. 36 is a diagram illustrating yet another example of the selection method by the semi-stable period selection unit;

FIG. 37 is a diagram illustrating yet another example of the selection method by the semi-stable period selection unit;

FIG. 38 is a flowchart illustrating an example of a process of Step S1312 in FIG. 13;

FIG. 39 is an example of time series data including a focused stable period relating to the gait;

FIG. 40 is a diagram illustrating an example of the plurality of semi-stable periods enumerated in Step S1310, based on the time series data illustrated in FIG. 39;

FIG. 41 is a diagram illustrating a relationship between a semi-stable period ID = 44 and a semi-stable period ID =45, and the time series data.

FIGs. 42A and 42B are diagrams illustrating a specifying method of a first one step for the semi-stable period;

FIGs. 43A and 43B are diagrams illustrating a specifying method of a second step for the semi-stable period;

FIG. 44 is a diagram illustrating an example of a calculation method of a changed angle of a leg;

FIGs. 45A and 45B are diagrams illustrating the changed angle of the leg on the first step;

FIGs. 46A and 46B are diagrams illustrating the changed angle of the leg on the second step;

FIG. 47 is a diagram illustrating an example of each of calculation results in Steps S3804 and S3806 and illustrating an example of a determination result of a constraint condition at gait initiation;

FIG. 48 is a diagram illustrating a selection method based on the period length and the constraint condition at the gait initiation;

FIG. 49 is a diagram illustrating another example of the configuration of the measuring apparatus;

FIG. 50 is a flowchart illustrating an example of processing performed by a measuring apparatus 10B according to the example illustrated in FIG. 49;

FIG. 51 is a flowchart illustrating an example of a process of Step S5008 in FIG. 50;

FIG. 52 is a flowchart illustrating an example of processing performed by a data acquisition device 300 and a processing device 100B;

FIG. 53 is a diagram illustrating the transitional state for a standing position;

FIG. 54 is a diagram illustrating an example of time series data of the sensor 2 obtained during the movement illustrating in FIG. 53;

FIG. 55 is a diagram illustrating an example of a detection algorithm of the stable period at the standing position;

FIG. 56 is a diagram illustrating an example of the plurality of enumerated semi-stable periods;

FIG. 57 is a diagram illustrating the transitional state for a sitting position;

FIG. 58 is a diagram illustrating an example of time series data of the sensor 2 obtained during the movement illustrating in FIG. 57;

FIG. 59 is a diagram illustrating an example of the detection algorithm of the stable period at the sitting position;

FIG. 60 is a diagram illustrating an example of the plurality of enumerated semi-stable periods;

FIGs. 61A and 61B are diagrams illustrating an example of a movement having no relationship with the transitional state at the standing position or the sitting position;

FIG. 62 is a diagram illustrating the constraint condition relating to the standing position;

FIG. 63 is a diagram illustrating the constraint condition relating to the sitting position;

FIG. 64 is a diagram illustrating the transitional state for a standing action;

FIG. 65 is a diagram illustrating an example of time series data of the sensor 2 obtained during the movement illustrated in FIG. 64;

FIG. 66 is a diagram illustrating an example of the detection algorithm of a strong action period for the standing action;

FIGs. 67A and 67B are flowcharts illustrating an example of processing performed by the measuring apparatus 10 when the transitional period relating to an action which is not stably repeated is specified;

FIG. 68 is a diagram illustrating an example of a plurality of enumerated weak action periods;

FIG. 69 is a diagram illustrating an example of the plurality of weak action periods which are enumerated based on a plurality of eased values of a third threshold candidate; and

FIG. 70 is a diagram illustrating the transitional period of the standing action.

DESCRIPTION OF EMBODIMENT

Hereinafter, an embodiment will be described in detail with reference to the accompanying drawings.

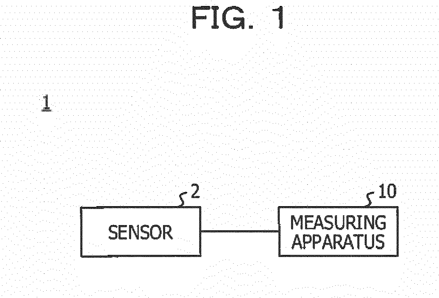

FIG. 1 is a diagram illustrating the entire configuration of a measurement system including a measuring apparatus according to an example.

A measurement system 1 includes a sensor 2 and a measuring apparatus 10. "A person" indicates a person to be measured below.

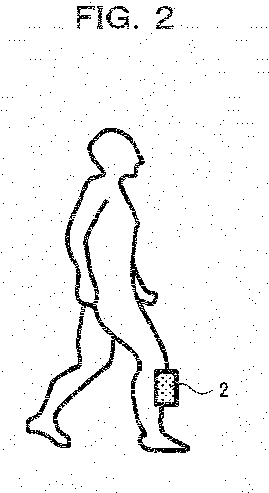

The sensor 2 detects a movement of a person. The movement of a person may randomly occur and may be a movement of a certain portion of the body of a person, for example. For example, the movement of a person may include a movement of the hand or the leg, a movement of a line of sight, a movement of internal organs, a movement of the brain (brainwave), and the like. The sensor 2 may be single or a group of a plurality of sensors. The sensor 2 may be attached to a person by using the hand and the like, so as to be difficult to relatively move against the person, may be held by the person, and may be disposed at a position separated from a person to be measured. An example of the sensor of the type which is attached to a person includes a gyro sensor, an acceleration sensor, and the like. An example of the sensor of the type which is disposed at a position separated from a person includes an image sensor (camera), a distance image sensor, and the like. In the following descriptions, as an example, the sensor 2 is set to be a gyro sensor attached to the leg of a person, as schematically illustrated in FIG. 2, as long as the sensor is not particularly mentioned.

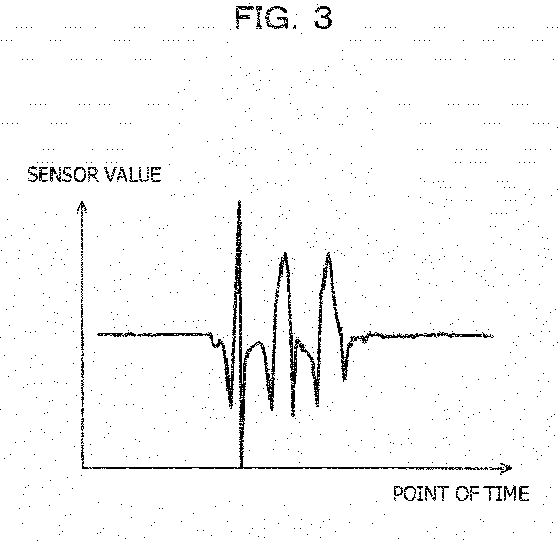

The measuring apparatus 10 specifies a period for which the person performs a predetermined movement or a predetermined posture, based on time series data (see FIG. 3) of sensor values obtained from the sensor 2. The time series data of the sensor values may be raw data of the sensor values or may be data subjected to certain preprocessing, conversion processing, or the like. A configuration, an operation, and the like of the measuring apparatus 10 will be described later. The predetermined movement or the predetermined posture may be anything. Here, a movement is set to have a dynamic state and a posture is set to have a static state without the movement. However, the movement and the posture may or may be not distinguished from each other. For example, the movement may be gait, running, and the like. For example, the posture may be a sitting position, a standing position, and the like. The movement or the posture has a transitional state before and/or after reaching a stable state (fixed state). Whether the movement or the posture has the transitional state only before reaching the stable state or has the transitional state both before and after the stable state depends on the movement or the posture.

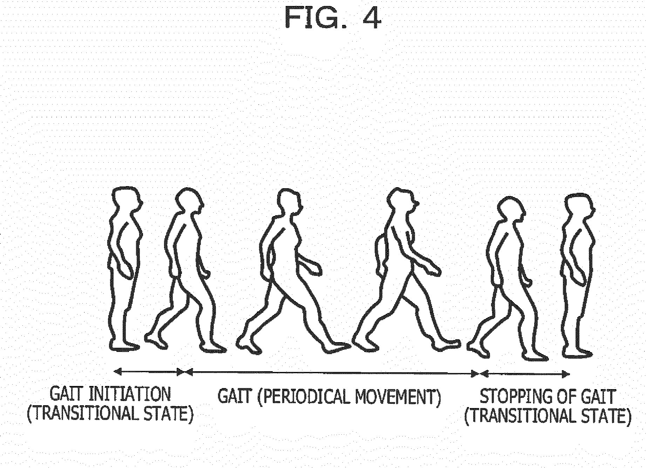

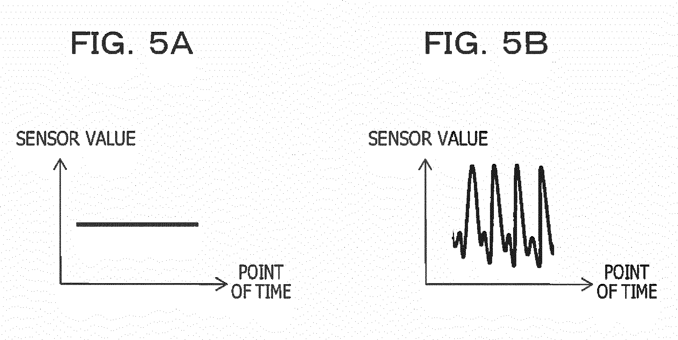

FIG. 4 is a diagram illustrating the transitional state. FIG. 5A illustrates an example of the time series data of the sensor 2 when the movement or the posture is in a stationary state (stationary posture) as the stable state. FIG. 5B illustrates an example of the time series data of the sensor 2 when the movement is in the stable state of gait.

The example illustrated in FIG. 4 relates to the gait. For example, in a case of the gait, a state from a moment at which a first step starts from a stationary posture at gait initiation until the movement is transitioned so as to be in the stable state of gait corresponds to the transitional state before the stable state of the gait. The stable state of the gait corresponds to a state where a stride, a pace, or the like is periodically substantially unchanged (see FIG. 5B). A state from the stable state before gait is stopped until the movement is transitioned to a stationary posture corresponds to the transitional state after the stable state of the gait.

As illustrated in FIGs. 5A and 5B, generally, in the stable state, a predetermined parameter (for example, amplitude of a peak or an interval between peaks) obtained based on the time series data of the sensor values is periodically substantially unchanged. Thus, the stable state is easier to be detected than the transitional state.

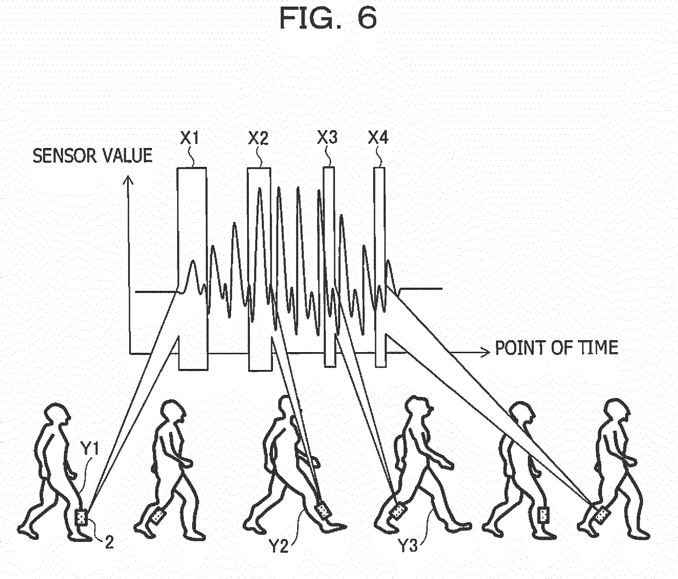

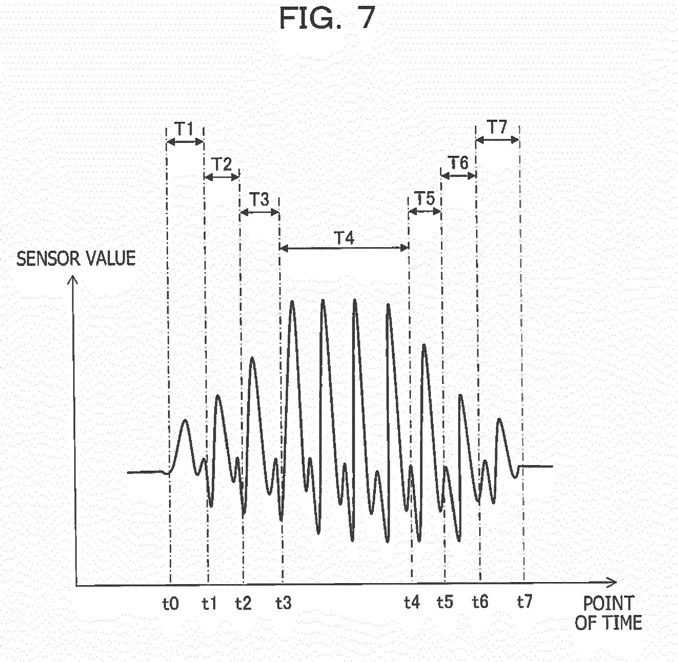

Next, a detection principle of the transitional state, which is used in the measuring apparatus 10 will be described with reference to FIGs. 6 and 7. In the following descriptions, as an example, a case using the gait as the predetermined movement will be described.

FIG. 6 is a diagram illustrating an example of a relationship between the time series data of the sensor 2 obtained at a time of gait and a movement of the person at this time. FIG. 6 schematically illustrates the movement of the person at the time of the gait, in association with the time series data. Specifically, data of a period X1 corresponds to data when the leg (right leg in this example) to which the sensor 2 is attached steps forward during gait initiation (when the right leg is raised). Data of a period X2 corresponds to data when the right leg steps forward in the stable state of the gait. Data of a period X3 corresponds to data when the left leg steps forward in the stable state of the gait (when the right leg comes into contact with the ground). Data of a period X4 corresponds to data when the left leg steps forward before the gait stops (when the right leg comes into contact with the ground).

As illustrated in FIG. 6, a pattern (feature based on the sensor values) of the sensor value in the transitional state is similar to a pattern of the sensor values in the stable state of the gait. For example, a pattern of the sensor values during the period X1 is similar to the sensor values during the period X2. A pattern of the sensor values during the period X3 is similar to the sensor values during the period X4. That is, data indicating a movement of the body in the transitional state before and after the stable state is not an extent of being considered (included) as the stable state, by pattern matching, but the data is similar to sensor values in the stable state to some degrees. Thus, it is understood that it is possible to detect the transitional state with high accuracy by paying attention to such similarity.

FIG. 7 is a diagram illustrating a difference of characteristics between a plurality of periods which have different closeness to a period of the stable state. FIG. 7 illustrates time series data the same as the time series data illustrated in FIG. 6, as time series data of the sensor 2 obtained during the gait.

In the example illustrated in FIG. 7, a period T4 is a period of a point t3 of time to a point of time t4 and is a period corresponding to the stable state of the gait. In the following descriptions, the period corresponding to the stable state is also referred to as a "stable period". A period T1 is a period of a point t0 of time to a point t1 of time, and is a period separated from the stable period T4 by $\Delta T1(= t3 - t1)$. A period T2 is a period of the point t1 of time to a point t2 of time, and is a period separated from the stable period T4 by $\Delta T2(= t3 - t2)$. A period T3 is a period of the point t2 of time to the point t3 of time, and is a period just before the stable period T4 (that is, period adjacent to the stable period T4). A period T5 is a period of the point t4 of time to a point t5 of time, and is a period just after the stable period T4 (that is, period adjacent to the stable period T4). A period T6 is a period of the point t5 of time to a point t6 of time, and is a period separated from the stable period T4 by $\Delta T6(= t5 - t4)$. A period T7 is a period of the point t6 of time to a point t7 of time, and is a period separated from the stable period T4 by $\Delta T7(= t6 - t4)$.

As illustrated in FIG. 7, the transitional state has a tendency that the pattern (feature based on the sensor values) of the sensor value is proximate to the corresponding pattern in the stable period with being (timely) close to the stable period. For example, when a parameter (feature based on the sensor values) such as amplitude of a peak attracts attention, as illustrated in FIG. 7, the amplitude of a peak in the period T2, not in the period T1 is proximate to amplitude of a peak in the period T4. As illustrated in FIG. 7, the amplitude of a peak in the period T3, not in the period T2 is proximate to the amplitude of the peak in the period T4. Similarly, the amplitude of a peak in the period T6, not in the period T7 is proximate to the amplitude of the peak in the period T4. The amplitude of a peak in the period T5, not in the period T6 is proximate to the amplitude of the peak in the period T4. Thus, it is understood that it is possible to detect the transitional state with high accuracy by paying attention to the tendency of such similarity.

In this example, the measuring apparatus 10 detects the transitional state by using the following method.

First, the measuring apparatus 10 specifies a first period as the period (stable period) of the stable state. During the first period, it is determined that the gait is being performed when a first threshold is applied to the time series data of the sensor 2. The first threshold is any value and depends on a detection method for the stable state. For example, when the stable state is detected by pattern matching, the first threshold may be a threshold for a degree of similarity to a mask pattern, which is calculated. The first threshold may be defined as a lower limit value for a parameter or as an upper limit value, depending on the parameter which is being used. In addition, the first threshold may be defined as a range. The first threshold may be used in combination of a plurality of thresholds.

Then, the measuring apparatus 10 detects a second period for which it is determined that the gait is being performed when a second threshold which is eased compared to the first threshold is applied to the time series data of the sensor 2. At this time, the measuring apparatus 10 determines the second threshold as follows. The measuring apparatus 10 determines the second threshold based on a parameter having a tendency (this tendency is referred to as a "similarity increase tendency" below) that a value based on the time series data of the sensor 2 becomes closer to a value within the stable period with being close to the stable period. This is because a tendency of similarity in the transitional state illustrated in FIG. 7 is used.

Here, "the second threshold which is eased compared to the first threshold" means a relative relationship, that is, that when the first threshold and the second threshold are applied to the time series data of the sensor 2, the second threshold is easier to be determined that the gait is being performed than the first threshold. When the first threshold and the second threshold are thresholds relating to the same type of parameter, the second threshold is a threshold eased compared to the first threshold. In this case, the second period includes the whole stable period and includes a period which is not included in the stable period. "The second threshold which is eased compared to the first threshold" means a relative relationship, that is, that the second period based on the second threshold includes at least a portion of the stable period based on the first threshold and includes a period which is not included in the stable period, when the first threshold and the second threshold are not thresholds relating to the same type of parameter.

As described above, the second threshold may relate to a parameter which has the same type as a parameter relating to the first threshold, or may relate to a parameter which has a different type from a parameter relating to the first threshold. For example, the second threshold may be determined as a threshold relating to amplitude of a peak of the sensor values. In this case, an occurrence period of a peak of which the amplitude continuously exceeds the first threshold may be specified as the stable period, and an occurrence period of a peak of which the amplitude continuously exceeds the second threshold may be specified as the second period. The second threshold may be used in combination of a plurality of thresholds.

The measuring apparatus 10 preferably specifies a parameter having the strongest tendency that a value based on the time series data of the sensor 2 becomes closer to a value within the stable period with being close to the stable period, among the plural types of parameters as a parameter for the second threshold. In this case, the measuring apparatus 10 determines the second threshold based on the parameter for the second threshold. Each of the plural types of parameters may be a parameter which may be used for specifying the first period, that is, a parameter appropriate for specifying the stable state of the gait (for example, amplitude of a peak, interval between peaks, autocorrelation coefficient (for detecting periodicity), and the like).

Then, the measuring apparatus 10 specifies the transitional period based on a difference between the second period and the stable period. For example, the measuring apparatus 10 specifies a period obtained by excluding the stable period from the second period, as the transitional period. When a plurality of stable periods is detected from the time series data of the sensor 2, the second period may be detected and the transitional period may be specified for each of the stable periods.

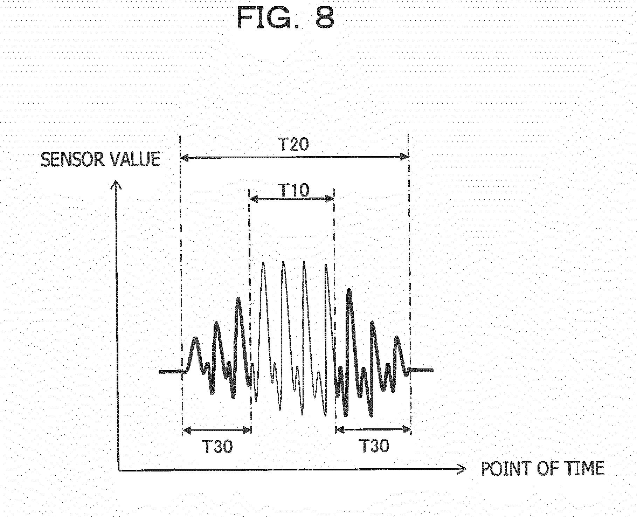

In the example illustrated in FIG. 8, the specified stable period is indicated by T10 and the specified second period is indicated by T20. In the example illustrated in FIG. 8, the second period T20 includes the whole stable period T10 and includes a period (period T30) which is not included in the stable period T10. The measuring apparatus 10 specifies the period T30 (two periods T30 before and after the stable period T10) which is a period obtained by excluding the stable period T10 from the second period T20, as the transitional period relating to the stable period T10.

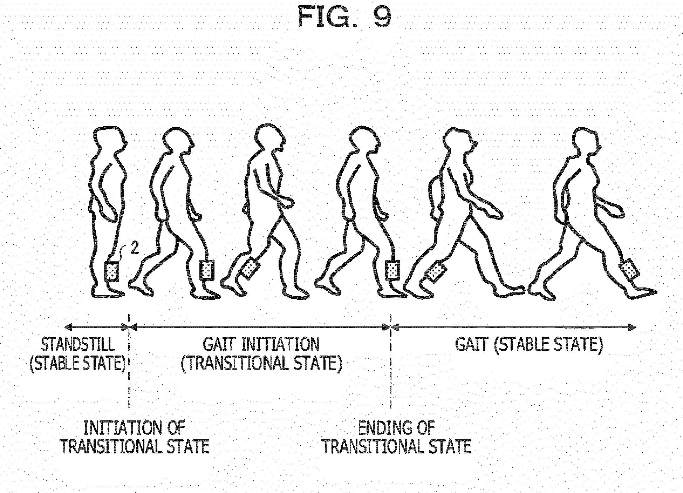

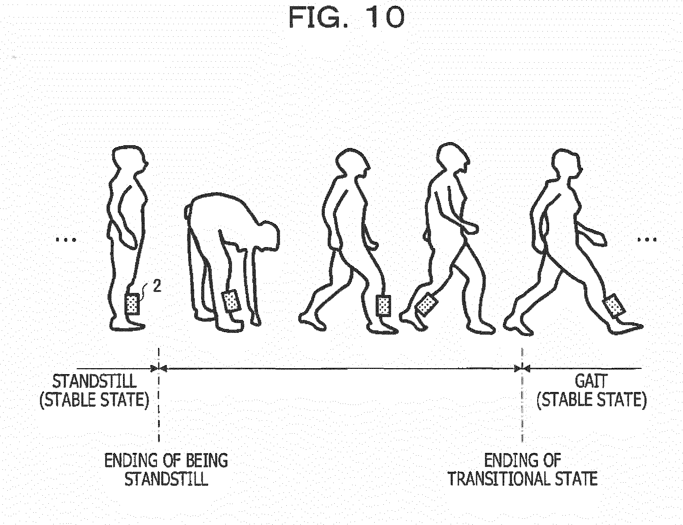

In the medical fields and the like, a symptom and the like when a certain posture or a certain movement is performed may be determined, and knowing of a state of activity and the like of the body or the brain before a certain posture or a certain movement is performed or after certain posture or a certain movement is performed is desirable (for example, NPL 2). The transitional state is a state before or after a certain posture or a certain movement is performed, and is an unstable state in which control is difficult. Thus, specifying of a unique pattern is difficult and various patterns are generated. This is because control when a command of doing a certain movement is transmitted from the brain and a muscle is caused to react by the command is difficult, and a change or abnormality which is not viewed in a periodic movement easily occurs. Since a person does various postures or various movements, defining of a mask pattern for all of the postures or all of the movements is difficult. At a time between the stable state, and the separate posture or separate movement before and after the stable state, various postures or various movements are included in addition to a movement of the transitional state which is originally desired to be extracted. For example, as illustrated in FIG. 9, if only the transitional state which is originally desired to be extracted is included at a time between the stationary posture and the stable state of the gait, as illustrated in FIG. 10, a movement state (movement state having no relationship with gait initiation) of which determination as the transitional state is difficult may be included. In the example illustrated in FIG. 10, a movement state of picking an object up is included at a time between the stationary posture and the stable state of the gait, in addition to the transitional state toward the stable state of the gait. Thus, detecting of the transitional state with high accuracy may be difficult by simply using a method of specifying the whole period between an ending point of time of the stationary posture and initiation time of the stable state as the transitional state, or using a method of specifying a predetermined period (fixed period) before and after the stable state of the gait as the transitional state.

From this point, according to this example, since the second threshold is determined based on the parameter having the similarity increase tendency, it is possible to detect the transitional state with high accuracy by using the tendency of similarity illustrated in FIGs. 6 and 7. The transitional state may be detected with high accuracy when the parameter having the strongest similarity increase tendency is specified from the plural types of parameters in comparison to a case of using only single parameter. This is because the transitional state relating to a certain stable state is not necessarily allowed to be detected with high accuracy by using the parameter which is used for detecting the stable state. For example, this is because the value of the parameter may not be close to a value of the same parameter within the period of the stable state due to an influence of noises and the like before and after the stable state.

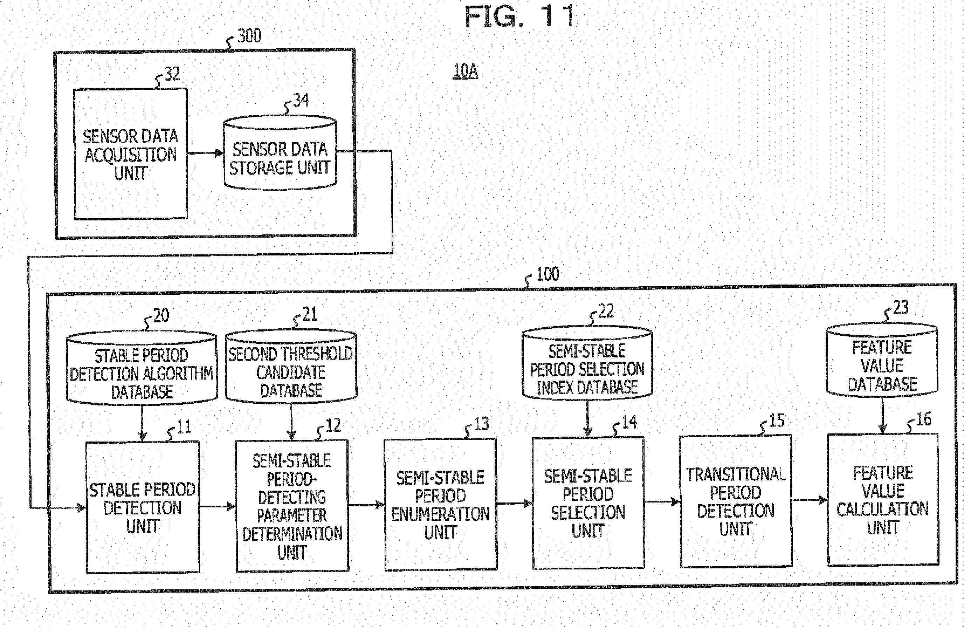

Next, a configuration example and an operation example of a measuring apparatus 10A as an example of the measuring apparatus 10 will be described with reference to FIGs. 11 to 48.

FIG. 11 is a diagram illustrating an example of a configuration of the measuring apparatus 10A.

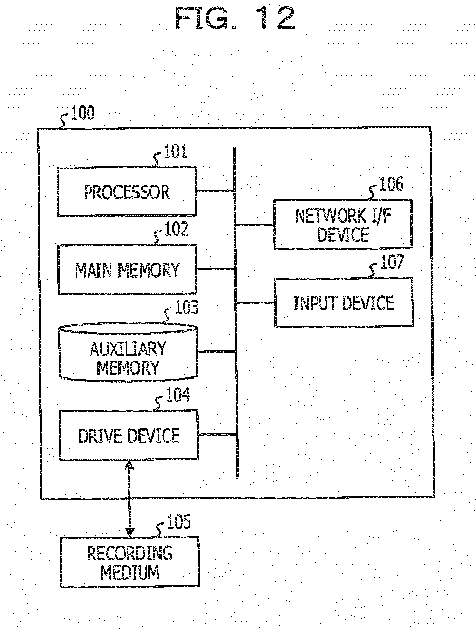

FIG. 12 is a diagram illustrating an example of a hardware configuration of a processing device 100 in the measuring apparatus 10A.

The measuring apparatus 10A includes the processing device 100 and a data acquisition device 300.

In the example illustrated in FIG. 12, the processing device 100 includes a processor 101, a main memory 102, an auxiliary memory 103, a drive device 104, a network I/F device 106, and an input device 107.

The processor 101 is a computation device that executes a program stored in the main memory 102 or the auxiliary memory 103. The processor 101 receives data from the input device 107 or a storage device and outputs data obtained by performing computation and processing to a storage device and the like.

The main memory 102 includes a read only memory (ROM), a random access memory (RAM), and the like. The main memory 102 is a storage device that stores or temporarily holds a program such as an operating system (OS) (which is basic software) and application software, which is executed by the processor 101, and data.

The auxiliary memory 103 includes a hard disk drive (HDD) and the like. The auxiliary memory 103 is a storage device that stores data associated with the application software and the like.

The drive device 104 reads a program from a recording medium 105, for example, a flexible disk and installs the read program on the storage device.

The recording medium 105 stores a predetermined program. The program stored in the recording medium 105 is installed on the processing device 100 through the drive device 104. The predetermined program which has been installed may be performed by the processing device 100.

The network I/F device 106 is an interface between the processing device 100 and peripheral devices which have a communication function. The processing device 100 and peripheral devices are connected to each other through a network constructed by a data transmission path such as a wired and/or wireless communication line.

The input device 107 includes a keyboard, a mouse, a touchpad, and the like, and the keyboard includes a cursor key, number inputting keys, various function keys, and the like.

In the example illustrated in FIG. 12, various types of processing which will be described below may be realized by the processing device 100 executing the program. In addition, the program may be recorded in the recording medium 105, the processing device 100 may read data in the recording medium 105 in which the program is recorded, and thus the various types of processing which will be described below may be realized. As the recording medium 105, various types of recording mediums may be used. Examples of the recording medium 105 includes a recording medium in which information is recorded optically, electrically, or magnetically, such as a compact disc (CD)-ROM, a flexible disk, and a magneto-optical disk, a semiconductor memory in which information is electrically recorded, such as a ROM and a flash memory. A carrier wave is not included in the recording medium 105.

As illustrated in FIG. 11, the processing device 100 includes a stable period detection unit 11 (example of first specification unit) and a semi-stable period-detecting parameter determination unit 12 (example of second threshold parameter determination unit). The semi-stable period-detecting parameter determination unit 12 determines a parameter (parameter for the second threshold) appropriate for detecting a semi-stable period (example of second period candidate), as will be described later. The processing device 100 includes a semi-stable period enumeration unit 13, semi-stable period selection unit 14 (example of second specification unit), a transitional period detection unit 15 (example of transitional period specification unit), and a feature value calculation unit 16. Each of the units (stable period detection unit 11 to feature value calculation unit 16) may be realized by the processor 101 illustrated in FIG. 12, for example. The processing device 100 includes a stable period detection algorithm database 20, a second threshold candidate database 21, a semi-stable period selection index database 22, and a feature value database 23, as illustrated in FIG. 11. Each of the databases (stable period detection algorithm database 20 to feature value database 23) may be realized by the auxiliary memory 103 illustrated in FIG. 12, for example.

Similarly, the data acquisition device 300 has a hardware configuration as illustrated in FIG. 12. The data acquisition device 300 includes a sensor data acquisition unit 32 and a sensor data storage unit 34. A portion or the entirety of the function of the data acquisition device 300 may be realized by the processing device 100.

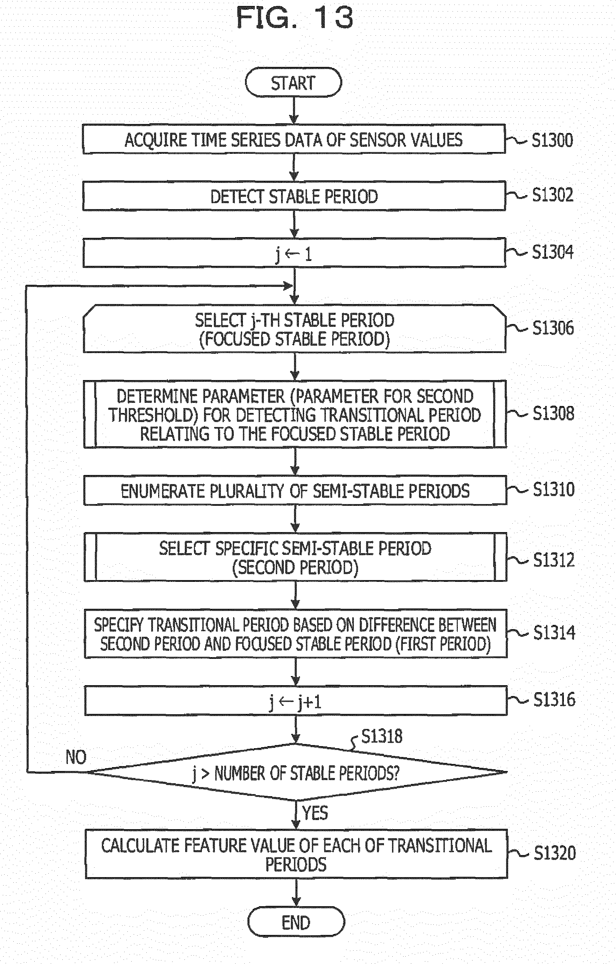

FIG. 13 is a flowchart illustrating an example of processing performed by the measuring apparatus 10A according to the example illustrated in FIG. 11.

In Step S1300, the sensor data acquisition unit 32 acquires time series data of sensor values from the sensor 2. The sensor data acquisition unit 32 stores the acquired time series data in the sensor data storage unit 34.

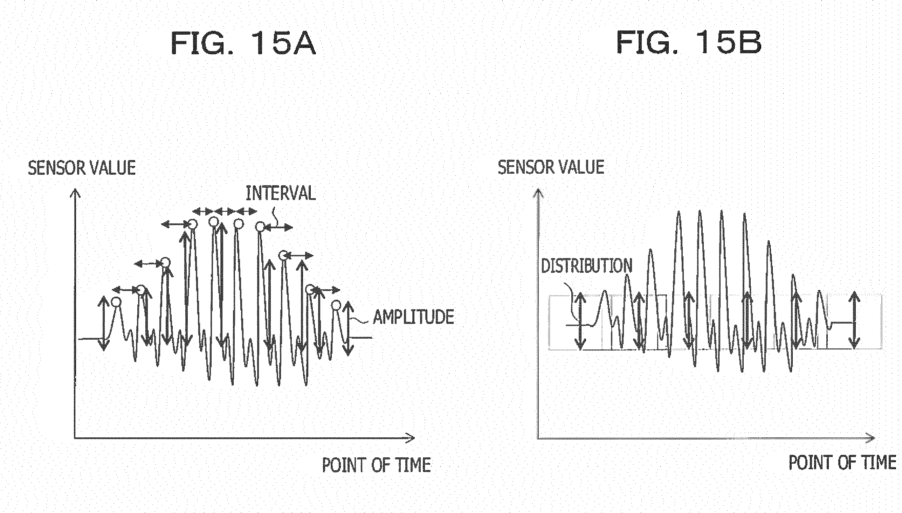

In Step S1302, the stable period detection unit 11 acquires the time series data in the sensor data storage unit 34, and detects a stable period based on the acquired time series data. At this time, the stable period detection unit 11 detects the stable period with reference to the stable period detection algorithm database 20. FIGs. 15A and 15B are diagrams illustrating an example of the stable period detection algorithm. FIG. 15A illustrates an example of an algorithm for detecting the stable period by applying the first threshold relating to two types of parameters of the interval between peaks and the amplitude of a peak. The algorithm is appropriate for detecting the stable state of the gait. For example, a period in which the interval between peaks based on sensor values is in a predetermined range (example of a portion of the first threshold) and the amplitude of a peak based on the sensor values is equal to or greater than a predetermined value (example of another portion of the first threshold) may be specified as the stable period of the gait. FIG. 15B illustrates an example of an algorithm for detecting the stable period by applying the first threshold relating to one type of parameter of distribution. The algorithm is appropriate for detecting the stationary posture. For example, a period in which the distribution based on sensor values is in a predetermined range (example of the first threshold) may be specified as the stable period relating to the stationary posture. In this manner, an algorithm may be prepared for each movement or each posture which is a detection target, and may have been stored in advance in the stable period detection algorithm database 20. At this time, two or more algorithms (that is, two types or more of first thresholds) may be prepared for a specific movement or a specific posture which is a detection target and may have been stored in advance in the stable period detection algorithm database 20.

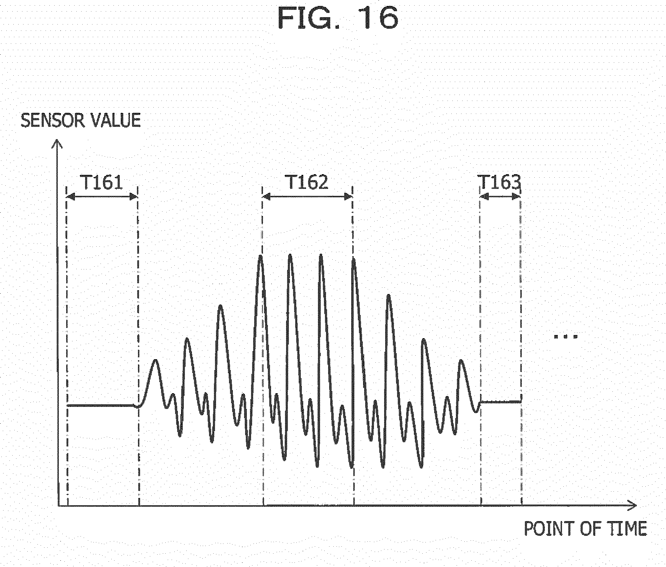

In Step S1302, when a plurality of stable periods (stable periods which are timely separated from each other) is detected, the stable period detection unit 11 sets (stores) the number of the plurality of stable periods as "the number of stable periods". For example, in the example illustrated in FIG. 16, a period T161 is detected as the stable period relating to the stationary posture, a period T162 is detected as the stable period relating to the gait. A period T163 is detected as the stable period relating to the stationary posture.

In Step S1304, the semi-stable period-detecting parameter determination unit 12 sets a value j to be an initial value of "1".

In Step S1306, the semi-stable period-detecting parameter determination unit 12 selects the j-th stable period as a focused stable period.

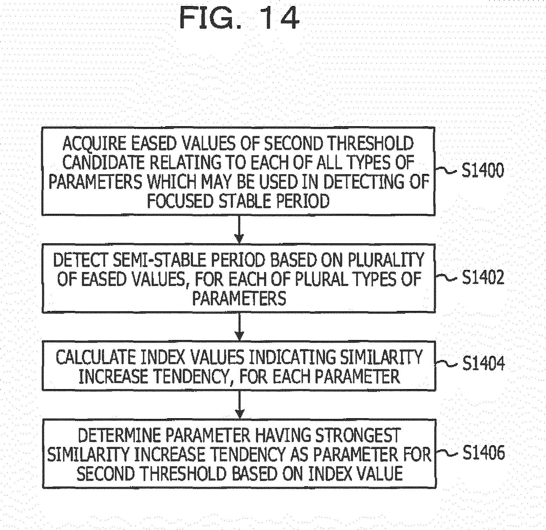

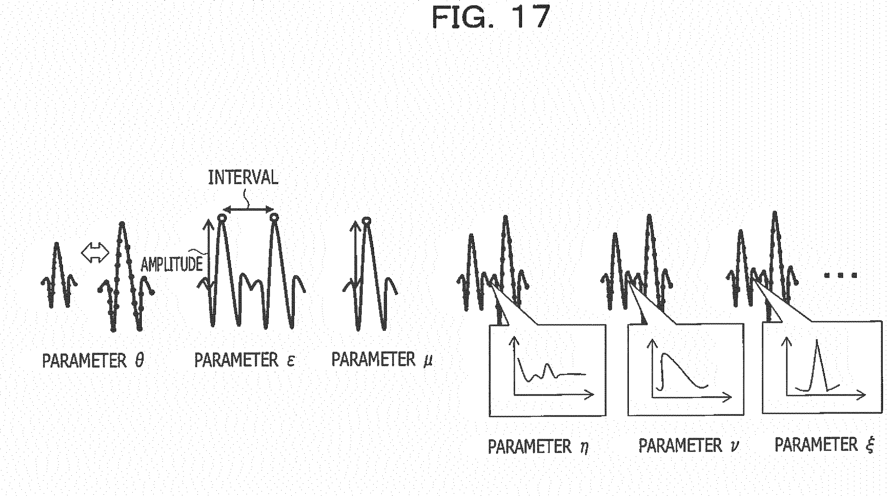

In Step S1308, the semi-stable period-detecting parameter determination unit 12 determines a parameter appropriate for detecting the transitional period relating to the focused stable period, as the parameter for the second threshold, with reference to the second threshold candidate database 21. For example, the semi-stable period-detecting parameter determination unit 12 determines the parameter appropriate for detecting the semi-stable period relating to the focused stable period among the plural types of parameters of $\theta$, $\varepsilon$, and the like as illustrated in FIG. 17, as the parameter for the second threshold. This process will be described later with reference to FIG. 14.

In Step S1310, the semi-stable period enumeration unit 13 enumerates a plurality of semi-stable periods based on a plurality of eased values of a second threshold candidate relating to the parameter for the second threshold which is determined by the semi-stable period-detecting parameter determination unit 12. The second threshold candidate is a candidate which may be used as the second threshold. The "eased value" represents a relative relationship, that is, mitigation compared to an original threshold for detecting the stable state of a movement or a posture relating to the focused stable period (threshold for detecting the stable period relating to the parameter which relates to the eased value). The stable state which may be detected based on the eased value may be different from the stable state which is originally to be detected because of the eased value. Thus, here, the stable state detected based on the eased value is referred to as "a semi-stable state". An example and the like of the eased value will be described later.

Specifically, the semi-stable period enumeration unit 13 enumerates the stable period of a movement or a posture relating to the focused stable period which may be detected based on the eased value, as the semi-stable period relating to the eased value for each of the plurality of eased values of the second threshold candidate relating to the parameter for the second threshold. Thus, a plurality of semi-stable periods which include at least a portion of the focused stable period is enumerated. For example, when the focused stable period is a stable period relating to the gait, the stable period of the gait which may be detected based on the eased value is enumerated as the semi-stable period relating to the eased value, for each of the plurality of eased values of the second threshold candidate relating to the parameter for the second threshold.

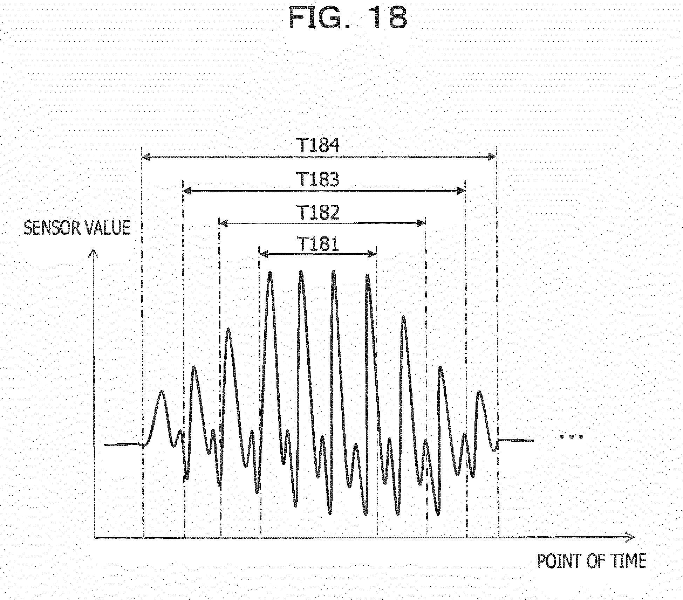

FIG. 18 illustrates three semi-stable periods T182, T183, and T184 which are enumerated for the focused stable period T181 and are correlated with the time series data. The semi-stable periods T182, T183, and T184 are detected based on three eased values which have different degrees of mitigation. The number of enumerated semi-stable periods is any value, may be equal to or greater than 4, or may be 2 or 1.

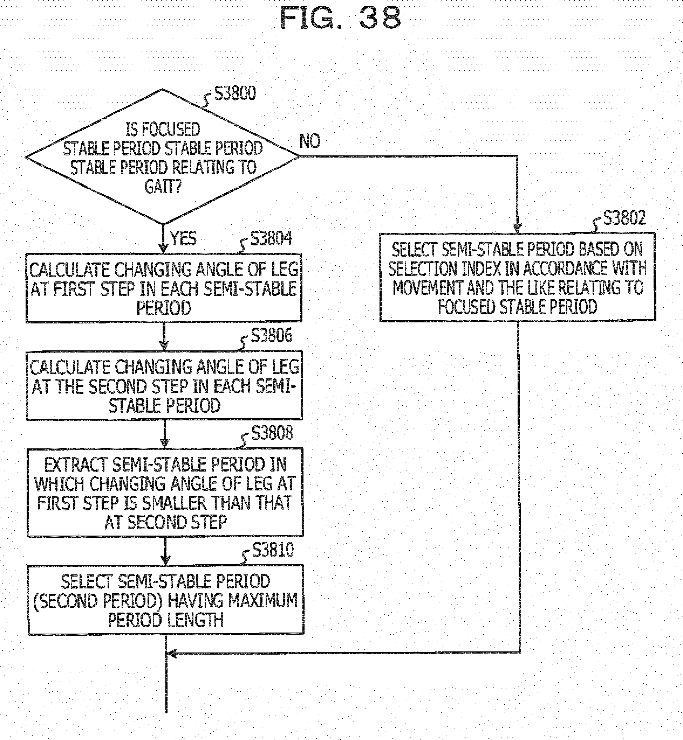

In Step S1312, the semi-stable period selection unit 14 selects one semi-stable period from a plurality of semi-stable periods which have been enumerated in Step S1310, with reference to the semi-stable period selection index database 22. The semi-stable period selection unit 14 outputs the semi-stable period which has been selected in this manner, as the second period. An example of this process will be described later with reference to FIG. 38.

In the semi-stable period selection index database 22, a rule (selection index) for specifying the second period from the plurality of enumerated semi-stable periods is stored. Selection indices may be different from each other depending on a movement or a posture relating to the focused stable period.

For example, a semi-stable period having a period length which is the maximum value, the minimum value, an average value, or the medium value among the plurality of enumerated semi-stable periods may be specified as the second period. In addition, a semi-stable period having a period length which is the maximum value, the minimum value, an average value, or the medium value among a plurality of enumerated semi-stable periods which have the length which is equal to or greater or less than threshold times the length of the focused stable period may be specified as the second period. A semi-stable period having a period length which is the maximum value, the minimum value, an average value, or the medium value among a plurality of enumerated semi-stable periods which are equal to or greater or less than a predetermined threshold may be specified as the second period. In these cases, the eased value of the second threshold candidate used in obtaining of the semi-stable period which has been selected as the second period is employed as the second threshold. Thus, in other words, in Step S1312, the semi-stable period selection unit 14 determines the second threshold with reference to the semi-stable period selection index database 22.

In addition, a period obtained by overlapping in all or some of the enumerated semi-stable periods may be specified as the second period. In this case, the eased value of the second threshold candidate used in obtaining a plurality of semi-stable periods forming the overlapped period may be employed as the second threshold.

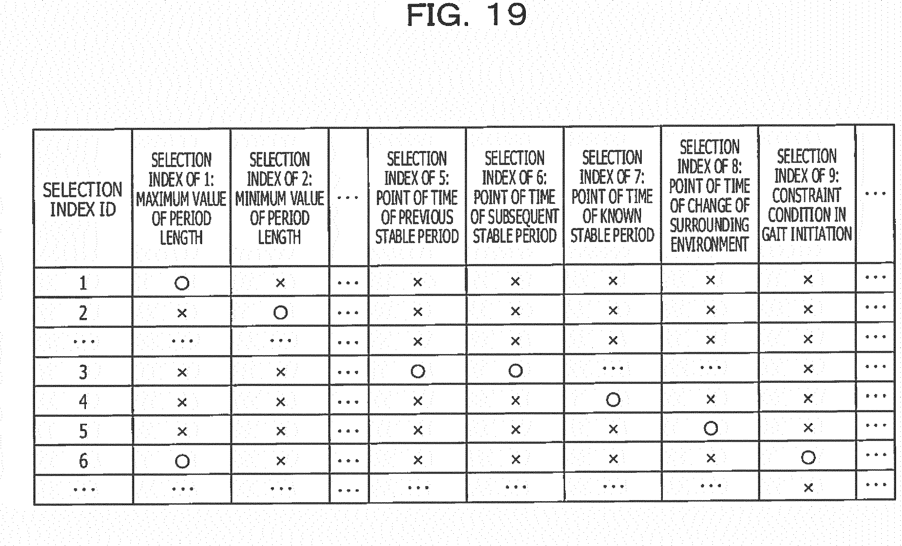

FIG. 19 is a diagram illustrating an example of data in the semi-stable period selection index database 22. FIG. 19 illustrates a table which represents a selection index used among selection indices 1, 2,..., 5, 6, 7, 8, 9,..., in correlation with the selection index ID. In FIG. 19, "o" indicates that the corresponding selection index is used. For example, in a case of the selection index ID = 1, using of "the maximum value of the period length" for the selection index of 1 is represented. In this case, a semi-stable period having a period length of the maximum value among the plurality of enumerated semi-stable periods is specified as the second period. The selection index ID may be designated by a user, or may be automatically designated in accordance with a movement or a posture relating to the focused stable period. In the example illustrated in FIG. 19, data in the semi-stable period selection index database 22 is used for selecting a semi-stable period relating to the gait. Plural pieces of data in the semi-stable period selection index database 22 may be prepared in accordance with a movement or a posture which is a detection target.

In Step S1314, the transitional period detection unit 15 specifies a transitional period relating to the focused stable period based on a difference between the second period obtained in Step S1312 and the focused stable period (first period) selected in Step S1306. For example, the transitional period detection unit 15 specifies a period obtained by excluding an overlapped portion of the focused stable period (first period) selected in Step S1306 from the second period obtained in Step S1312, as a transitional period relating to the focused stable period.

In Step S1316, the semi-stable period-detecting parameter determination unit 12 performs increment of "1" on the value j.

In Step S1318, the semi-stable period-detecting parameter determination unit 12 determines whether or not the value j is greater than the number of stable periods. That is, the semi-stable period-detecting parameter determination unit 12 determines whether or not a transitional period is specified for the all of the detected stable periods. When the value is greater than the number of stable periods, the process proceeds to Step S1320. When the value j is not greater than the number of stable periods, the process returns to Step S1306 and the processes of Steps S1306 to S1314 are performed on the j-th stable period (new focused stable period).

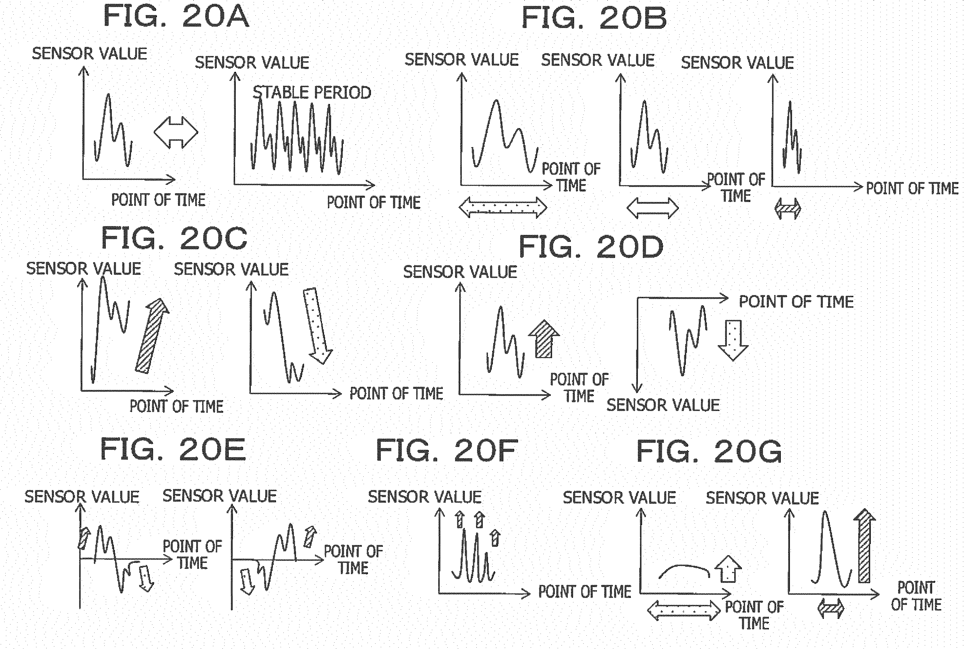

In Step S1320, the feature value calculation unit 16 calculates a feature value of each of transitional periods with reference to the feature value database 23. The calculated feature value may be determined in accordance with a movement and a posture which are detection targets. For example, the feature value may be a feature value (feature obtained from a shape) as illustrated in FIGs. 20A to 20G. Such a feature value is pre-defined and is stored in the feature value database 23. For example, FIG. 20A illustrates similarity with a pattern in the stable period and FIG. 20B illustrates a rising time toward the stable period (or from the stable period) as an example of the feature value. FIG. 20C illustrates a changing tendency (rising to the right or falling to the right) in a time axis, as an example of the feature value. FIG. 20D illustrates a polarity (positivity or negativity), FIG. 20E illustrates biphase as an example of the feature value, FIG. 20F illustrates multimodality as an example of the feature value. FIG. 20G illustrates flatness or sharpness as an example of the feature value. For example, a degree of similarity with the pattern in the stable period may be represented, for example, by comparison of the first threshold used when the stable period is detected and the second threshold used when the semi-stable period relating to the second period is detected. The length of the transitional period may be used for the rising time toward the stable period (or from the stable period).

The process of Step S1320 may be put between Step S1314 and Step S1316. In this case, the feature value is calculated for each transitional period relating to the value j.

FIG. 14 is a flowchart illustrating an example of the process of Step S1308 in FIG. 13.

In Step S1400, the semi-stable period-detecting parameter determination unit 12 acquires eased values of the second threshold candidate relating to each of all types of parameters which may be used in detecting of the focused stable period, with reference to the second threshold candidate database 21. For example, when the focused stable period in Step S1306 is a stable period relating to the gait, the semi-stable period-detecting parameter determination unit 12 acquires the eased values of the second threshold candidate relating to each of all types of parameters which may be used in detecting of the stable period which relates to the gait.

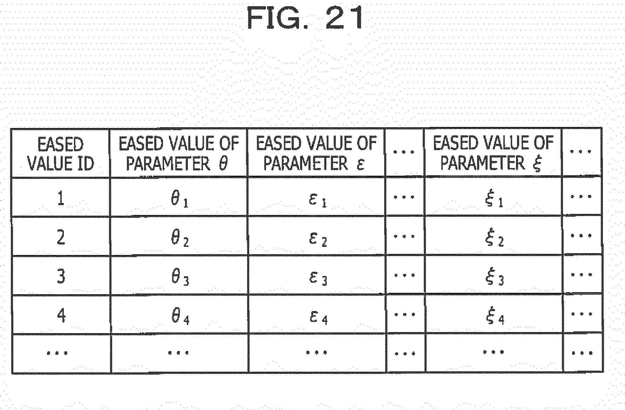

FIG. 21 illustrates an example of data (eased values of the second threshold candidate) in the second threshold candidate database 21. FIG. 21 illustrates data (eased values of the second threshold candidate) relating to the focused stable period in the second threshold candidate database 21. The data is prepared for each movement or each posture which is a detection target, and is stored in the second threshold candidate database 21. In the example illustrated in FIG. 21, a plurality of eased values of second threshold candidates relating to the plural types of parameters θ, ε,...,ξ is represented. The eased value ID represents a degree of mitigation. For example, the degree of mitigation becomes great as a value of the eased value ID becomes greater.

In Step S1402, the semi-stable period-detecting parameter determination unit 12 detects a semi-stable period based on the plurality of eased values, for each of the plural types of parameters. The semi-stable period-detecting parameter determination unit 12 calculates a length (period length) of the semi-stable period for each of the eased values. In this manner, the period length of the semi-stable period for each of the eased values is calculated for each of the plural types of parameters.

In Step S1404, the semi-stable period-detecting parameter determination unit 12 calculates index values (evaluation values) indicating strength of the similarity increase tendency, for each of the plural types of parameters. The index value indicating the strength of the similarity increase tendency may be calculated as an index value (evaluation value) indicating strength of a monotonous increase tendency as follows, for example.

$$E(\alpha) = \frac{1}{M_\alpha} \sum_{m=1}^{M_\alpha} \sigma(L_{\alpha_m})$$

[Math 1]

Here, α indicates the type of parameter and is as follows.

$$\alpha \in \{\theta, \varepsilon, \mu, ...\}$$

[Math 2]

$L\alpha_m$ indicates the period length of a semi-stable period based on the eased value of mitigation ID = m, which relates to the parameter α. If Mα indicates the maximum value (the number of eased values) of the mitigation ID relating to the parameter α, $\sigma(L\alpha_m)$ is as follows.

$$\sigma\left(L_{\alpha_m}\right)=\begin{cases} 1 & \left(if\ L_{\alpha_m} > L_{\alpha_{m-1}}\right) \\ -1 & \left(if\ L_{\alpha_m} < L_{\alpha_{m-1}}\right) \\ 0 & (otherwise) \end{cases}$$

[Math 3]

In the example illustrated in FIG. 21, a value of the eased value ID = 1 is the same as the threshold for detecting the stable period, and is not a "eased value" in a strict meaning. The value of the eased value ID = 1 may cause the degree of mitigation to be handled by using the eased value of "0" (see m = 1 of $\Sigma$ in the expression of Math. 1), and may be omitted from data in the second threshold candidate database 21 (in this case, m = 1 of $\Sigma$ in the expression of Math. 1 is changed to m = 2).

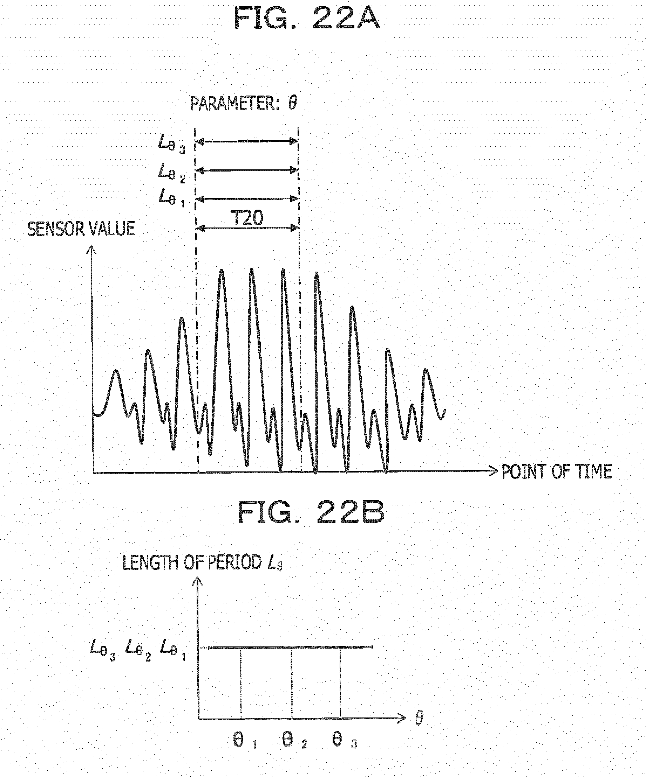

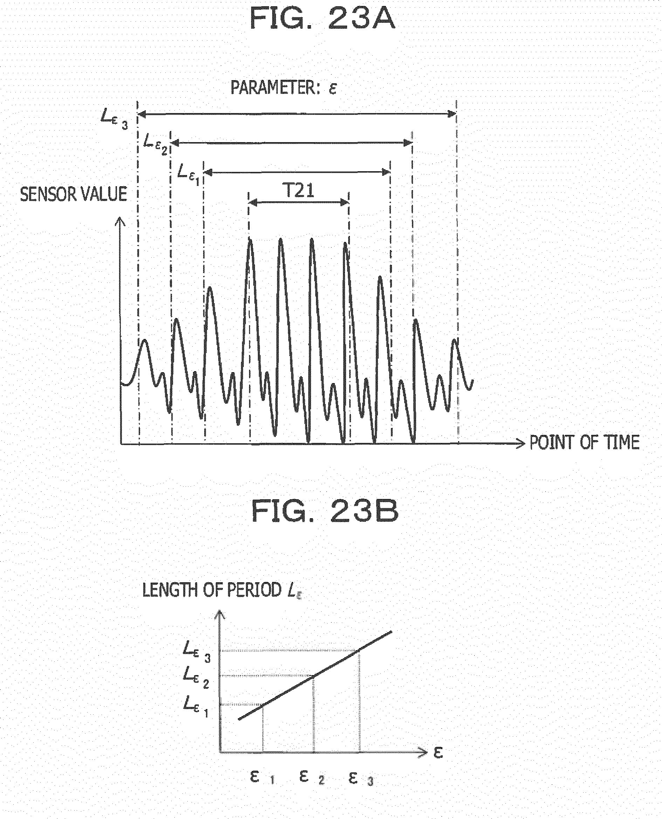

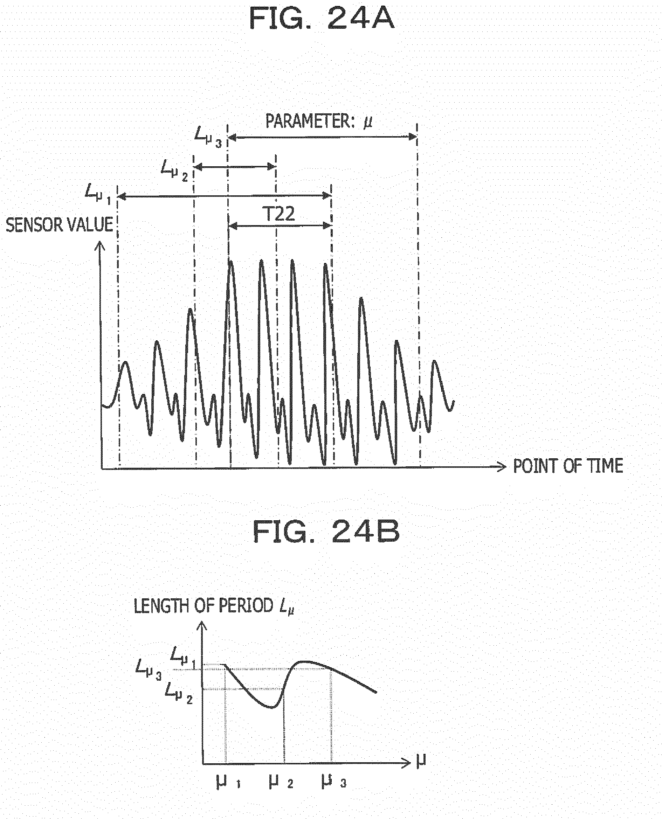

FIGs. 22A to 24B are diagrams illustrating examples of the period length of a semi-stable period for each of the eased values. FIG. 22A schematically illustrates period lengths $L_{\theta 1}$, $L_{\theta 2}$, and $L_{\theta 3}$ of semi-stable periods which respectively relates to three eased values $\theta_1$, $\theta_2$, and $\theta_3$ relating to the parameter $\theta$. FIG. 22B illustrates a relationship between the three eased values $\theta_1$, $\theta_2$, and $\theta_3$ relating to the parameter $\theta$ and the period lengths $L_{\theta 1}$, $L_{\theta 2}$, and $L_{\theta 3}$ of the semi-stable periods which respectively relate to the three eased values $\theta_1$, $\theta_2$, and $\theta_3$. FIG. 23A schematically illustrates period lengths $L_{\varepsilon 1}$, $L_{\varepsilon 2}$, and $L_{\varepsilon 3}$ of semi-stable periods which respectively relate to three eased values $\varepsilon_1$, $\varepsilon_2$, and $\varepsilon_3$ relating to the parameter c. FIG. 23B illustrates a relationship between the three eased values $\varepsilon_1$, $\varepsilon_2$, and $\varepsilon_3$ relating to the parameter $\varepsilon$ and the period lengths $L_{\varepsilon 1}$, $L_{\varepsilon 2}$, and $L_{\varepsilon 3}$ of the semi-stable periods which respectively relate to the three eased values $\varepsilon_1$, $\varepsilon_2$, and $\varepsilon_3$. FIG. 24A schematically illustrates period lengths $L_{\mu 1}$, $L_{\mu 2}$, and $L_{\mu 3}$ of semi-stable periods which respectively relate to three eased values $\mu_1$, $\mu_2$, and $\mu_3$ relating to the parameter $\mu$. FIG. 24B illustrate a relationship between the three eased values $\mu_1$, $\mu_2$, and $\mu_3$ relating to the parameter $\mu$ and the period lengths $L_{\mu 1}$, $L_{\mu 2}$, and $L_{\mu 3}$ of the semi-stable periods which respectively relate to the three eased values $\mu_1$, $\mu_2$, and $\mu_3$.

Regarding the second threshold candidate relating to the parameter $\theta$, the period length is not increased in an order of the period lengths $L_{\theta 1}$, $L_{\theta 2}$, and $L_{03}$ of the semi-stable periods even when mitigation may be performed in an order of the eased values $\theta_1$, $\theta_2$, and $\theta_3$, as illustrated in FIGs. 22A and 22B. That is, the period lengths $L_{\theta 1}$, $L_{\theta 2}$, and $L_{03}$ of the semi-stable periods are substantially the same as each other. Regarding the second threshold candidate relating to the parameter $\varepsilon$, the period length is increased in an order of the period lengths $L_{\varepsilon 1}$, $L_{\varepsilon 2}$, and $L_{\varepsilon 3}$ of the semi-stable periods when mitigation may be performed in an order of the eased values $\varepsilon_1$, $\varepsilon_2$, and $\varepsilon_3$, as illustrated in FIGs. 23A and 23B. Regarding the second threshold candidate relating to the parameter $\mu$, the period length is not increased (reduced and then increased) in an order of the period lengths $L_{\mu 1}$, $L_{\mu 2}$, and $L_{\mu 3}$ of the semi-stable periods when mitigation may be performed in an order of the eased values $\mu_1$, $\mu_2$, and $\mu_3$, as illustrated in FIGs. 24A and 24B.

Here, the index value $E(\alpha)$ is increased as the period length of the semi-stable period is increased with an increase of the degree of mitigation of the eased value for the second threshold candidate relating to the parameter $\alpha$. That is, the index value $E(\alpha)$ indicates the strength of the monotonous increase tendency of the period length of the semi-stable period with an increase of the degree of mitigation of the second threshold candidate. An increase of the period length of the semi-stable period with an increase of the degree of mitigation of the eased value of the second threshold candidate means that the above-described similarity increase tendency is present. Thus, specifying of a certain second threshold candidate having the similarity increase tendency becomes easy by using the index value $E(\alpha)$.

In Step S1406, the semi-stable period-detecting parameter determination unit 12 determines a parameter for calculating the index value indicating that the similarity increase tendency is strongest, as the parameter for the second threshold. In the examples illustrated in FIGs. 22A to 24B, the index value $E(\varepsilon)$ relating to the parameter $\varepsilon$ becomes "1" and becomes the maximum. Accordingly, in this case, the semi-stable period-detecting parameter determination unit 12 determines the parameter $\varepsilon$ as the parameter for the second threshold.

Generally, there is a high probability that the parameter relating to the first threshold which is used when the focused stable period is detected is a parameter having the strongest similarity increase tendency. However, the parameter relating to the first threshold which is used when the focused stable period is detected may be a parameter which does not have the strongest similarity increase tendency, due to noises and the like on the time series data. In this case, if the parameter relating to the first threshold which is used when the focused stable period is detected is determined as the parameter for the second threshold, specifying of the transitional period with high accuracy may be difficult.

From this point, according to the processes illustrated in FIGs. 13 and 14, the parameter having the strongest similarity increase tendency is searched for among the plural types of parameters. Thus, the parameter having the strongest similarity increase tendency may be used as the parameter for the second threshold. Accordingly, even when the parameter relating to the first threshold which is used when the focused stable period is detected is not a parameter having the strongest similarity increase tendency, it is possible to specify the transitional period with high accuracy.

Next, a specific example of the processes illustrated in FIGs. 13 and 14 will be described.

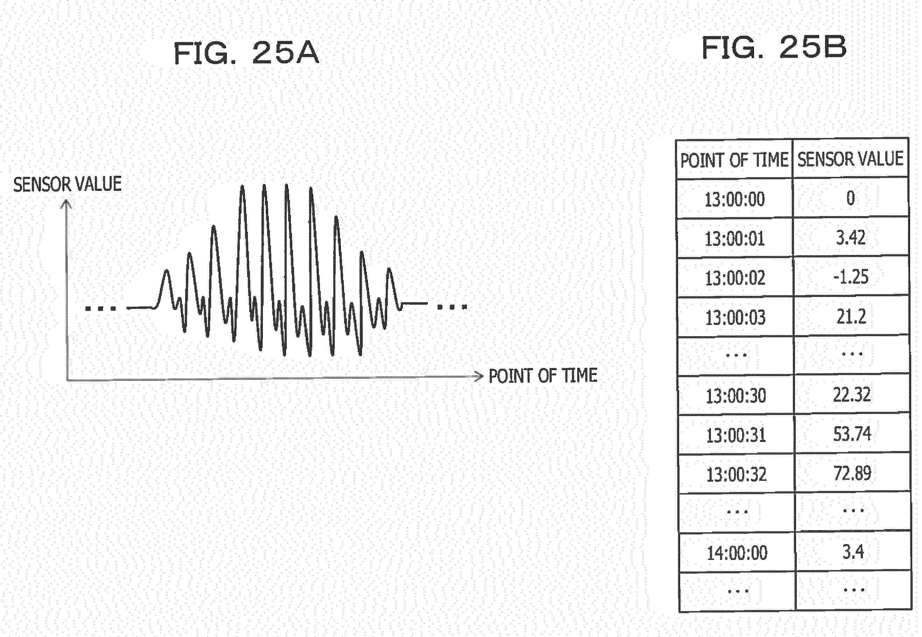

FIGs. 25A and 25B are diagrams illustrating an example of the time series data of sensor values, which is stored in the sensor data storage unit 34. FIG. 25A illustrates the time series data of the sensor values in a form of a waveform. FIG. 25B illustrates the time series data of the sensor values. In this manner, the sensor value may be stored in associated with time information.

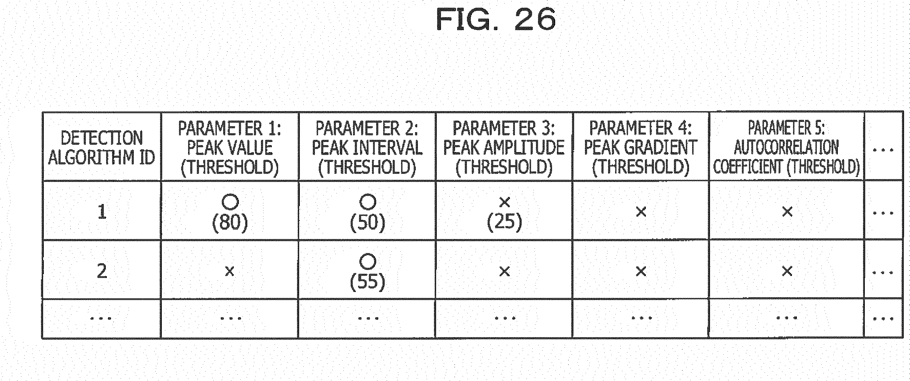

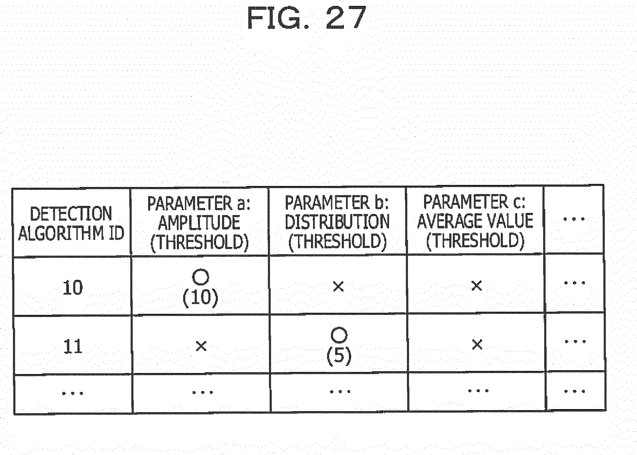

FIGs. 26 and 27 are diagrams illustrating an example of the stable period detection algorithm which is stored in the stable period detection algorithm database 20. For example, the stable period detection algorithm illustrated in FIG. 26 is prepared as an algorithm for detecting the stable state of the gait. The stable period detection algorithm illustrated in FIG. 27 is prepared as an algorithm for detecting the stationary posture. In the example illustrated in FIG. 26, a plurality of detection algorithms for detecting the stable state of the gait is prepared. Each of the detection algorithms is correlated with a detection algorithm ID and FIG. 26 illustrates a table which represents the type of parameter which is used among various parameters 1 to 5,.... In FIG. 26, "o" indicates that the corresponding type of parameter is used and a numeral in brackets indicates an example of a value of a threshold (first threshold) relating to the parameter. In FIG. 27, marks are similar to those in FIG. 26.

For example, the stable period detection unit 11 detects the stable state of the gait based on the detection algorithms illustrated in FIG. 26. At this time, the stable period detection unit 11 may detect the stable state of the gait by using only one specific detection algorithm among the detection algorithms illustrated in FIG. 26. In this case, the one specific detection algorithm may be designated by a user or be set as default. The stable period detection unit 11 may detect the stable state of the gait by using a plurality of specific detection algorithm or all of detection algorithms among the detection algorithm illustrated in FIG. 26. In this case, the stable period detection unit 11 may employ a result of the detection algorithm having highest detection accuracy.

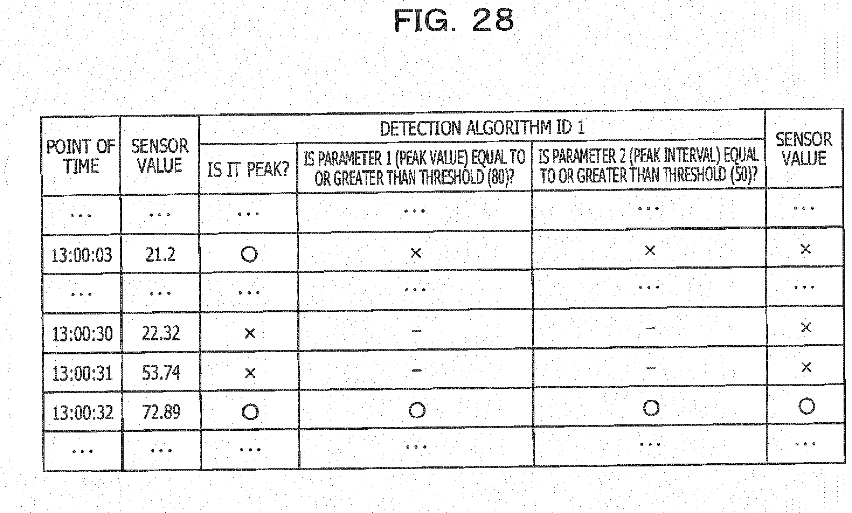

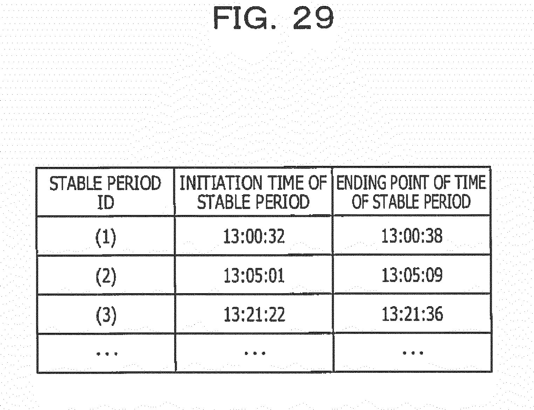

FIGs. 28 and 29 are diagrams illustrating a detection method of the stable state of the gait by the stable period detection unit 11. Here, the detection method by using the detection algorithm ID = 1 illustrated in FIG. 26 will be described as an example.

The stable period detection unit 11 determines whether or not a sensor value at each point of time is a peak, and outputs a determination result. In FIG. 28, "o" in a line of "is it a peak?" indicates that it is determined that the sensor value is a peak. When a peak is detected, the stable period detection unit 11 determines whether or not a value of the detected peak is equal to or greater than a predetermined value of "80" (example of a portion of the first threshold), and determines whether or not an interval (time interval to a peak before one peak) between peaks is equal to or greater than a predetermined value of "50" (example of another portion of the first threshold). In FIG. 28, "o" in a line of "parameter of 1 ···?" indicates that it is determined that the value of the detected peak is equal to or greater than the predetermined value of "80". "o" in a line of "parameter of 2 ···?" indicates that it is determined that the interval between peaks is equal to or greater than the predetermined value of "50". The stable period detection unit 11 determines whether or not a sensor value at each point of time is in the stable period, based on the determination results, "o" in a line of "is it in the stable period?" indicates, for example, that all of the three conditions are satisfied. For example, the stable period detection unit 11 determines that the point of time of "13:00:32" is an initiation time of the stable period. In this case, the stable period detection unit 11 determines whether a sensor value at each point of time until the next peak is detected is in the stable period. When the remaining two conditions are satisfied at the next peak, the stable period detection unit 11 determines that the stable period continues. If a peak at which the remaining two conditions are not satisfied is detected after the initiation time of the stable period, the stable period detection unit 11 determines that a point of time of a peak before one from the detected peak is an ending point of time of the stable period. The detection method is applied to a sensor value at each point of time, and thus a detection result of the stable period as illustrated in FIG. 29 is obtained. In the example illustrated in FIG. 29, the stable period ID is associated with the initiation time and the ending point of time for each of stable periods when a peak is detected.

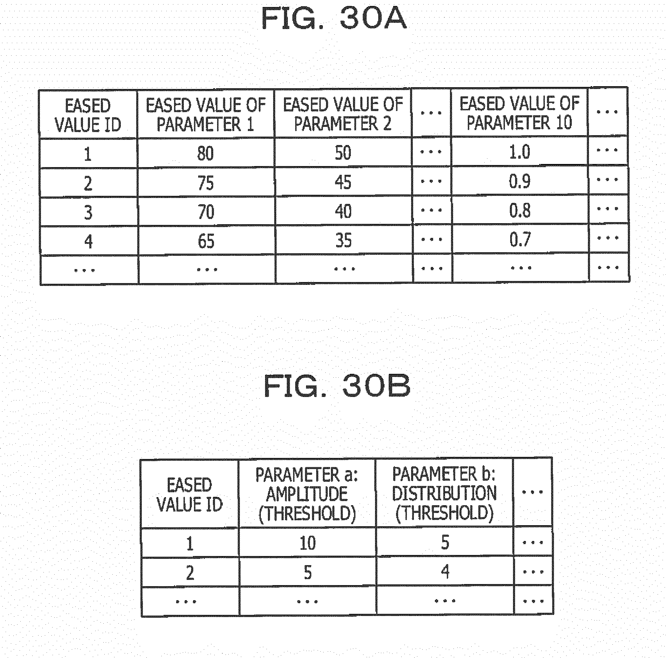

FIGs. 30A and 30B are diagrams illustrating examples of the eased values relating to various parameters used in the stable period detection algorithm. FIG. 30A illustrates an example of eased values relating to the various parameters used in the detection algorithm of the stable state of the gait. FIG. 30B illustrates an example of eased values relating to the various parameters used in the detection algorithm of the stationary posture. These types of data are stored in the second threshold candidate database 21 as described above. The eased value ID represents the degree of mitigation as described with reference to FIG. 21. For example, the degree of mitigation is increased as the value of the eased value ID becomes greater. In the example illustrated in FIG. 30, a eased value of the eased value ID = 1 is the same as the threshold for detecting the stable period, as found out by comparing FIGs. 26 and 27 to each other, but is not the "eased value" in a strict meaning. However, as described above, the eased value of the eased value ID = 1 may be used for obtaining an index value (evaluation value) E of the monotonous increase tendency.

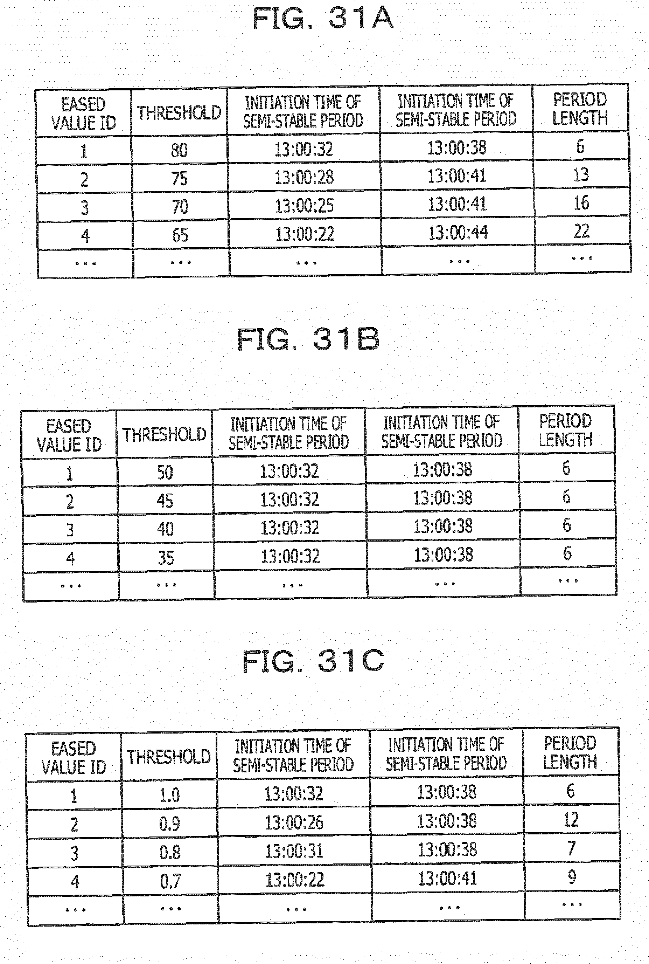

FIGs. 31A to 31C are diagrams illustrating detection results of the semi-stable period and the period length thereof by using the eased values relating to the various parameters. FIG. 31A illustrates the detection result relating to Parameter 1. FIG. 31B illustrates the detection result relating to Parameter 2. FIG. 31C illustrates the detection result relating to Parameter 10. Such a detection result is obtained for each of the parameters, as described above. When the stable period detected by using the detection method using the detection algorithm ID = 1 illustrated in FIG. 26 is the focused stable period, detection results (see FIGs. 31A and 31B) of the semi-stable period and the period length thereof may be obtained for each of Parameter 1 and Parameter 2. Instead of the obtained detection results or in addition to the obtained detection results, the stable period detected by using the same method (using the eased value instead of the first threshold) as the detection method using detection algorithm ID = 1 may be handled as the semi-stable period and thus the detection result of the semi-stable period and the period length thereof may be obtained.

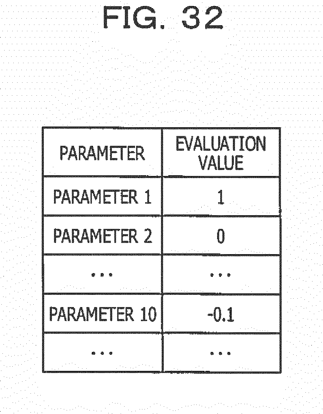

FIG. 32 is a diagram illustrating an example of a calculation result of the index value (evaluation value) E of the monotonous increase tendency, which is obtained based on the detection results illustrated in FIGs. 31A to 31C. A calculation method of the index value E of the monotonous increase tendency is as described above. In the example illustrated in FIG. 32, it is indicated that the index value E relating to Parameter 1 has the maximum value of "1" and has a strong monotonous increase tendency. In this case, Parameter 1 is determined as the parameter for the second threshold.

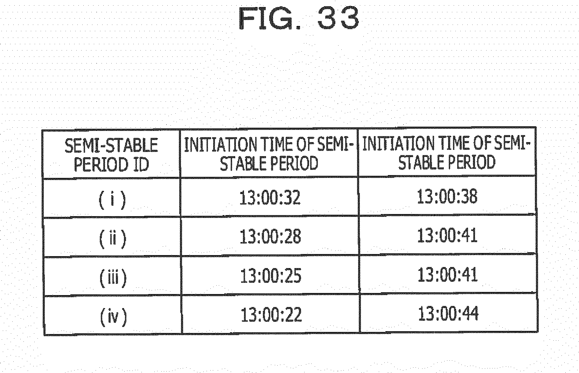

FIG. 33 is a diagram illustrating an example of an enumeration result of semi-stable periods, obtained based on Parameter 1 which is determined as the parameter for the second threshold, by the semi-stable period enumeration unit 13. The enumeration result of the semi-stable periods illustrated in FIG. 33 relates to one certain focused stable period. When a plurality of stable periods is detected, the parameter for the second threshold may be detected for each of the stable periods and the enumeration result of the semi-stable periods as illustrated in FIG. 33 may be output. In FIG. 33, a semi-stable period ID is assigned to each of the detected semi-stable periods. The semi-stable period ID may correspond to the eased value ID relating to the eased value which is used for detecting the semi-stable period relating to the corresponding semi-stable period ID. The semi-stable period enumeration unit 13 may enumerate semi-stable periods by using the calculation method as illustrated in FIG. 31 (that is, processing result of the semi-stable period-detecting parameter determination unit 12). This is because the same processing has been already performed by the semi-stable period-detecting parameter determination unit 12.

Next, another operation example of the semi-stable period selection unit 14 will be described.

The semi-stable period selection unit 14 selects one semi-stable period from the plurality of semi-stable periods which are enumerated by the semi-stable period enumeration unit 13, with reference to the semi-stable period selection index database 22, as described above. At this time, when information of the context may be used, the semi-stable period selection unit 14 may select one specific semi-stable period by using the information of the context.

As a narrowing method using the information of the context, for example, any or combination of the following methods may be used.

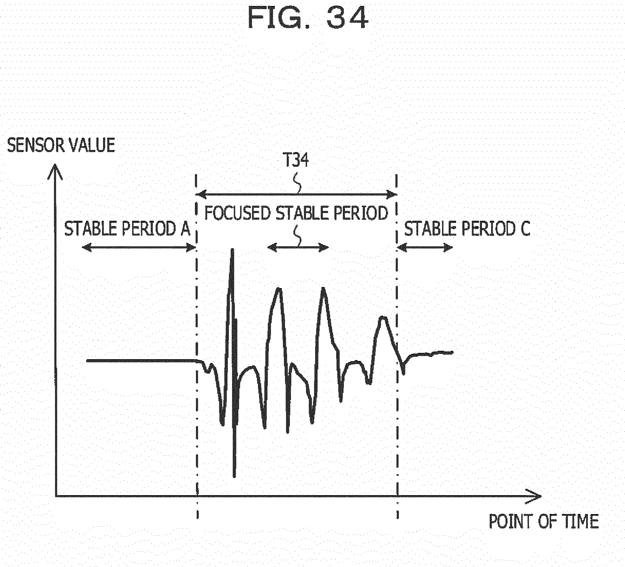

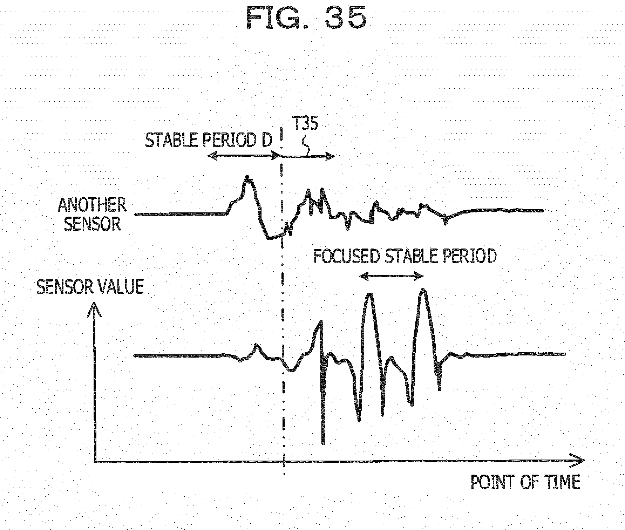

For example, when there is the stable period before and after the focused stable period, the semi-stable period selection unit 14 may exclude the semi-stable period overlapping the stable period from a selection target. This is because the transitional period has no portion which overlaps other stable periods. The stable period before and after the focused stable period may be detected by a sensor which is different from the sensor 2 used for detecting the focused stable period. For example, in the example illustrated in FIG. 34, stable periods A and C are present before and after the focused stable period. In this case, the semi-stable period selection unit 14 may exclude a semi-stable period which protrude from a period T34 from an ending point of time of the stable period A to an initiation time of the stable period C, from the selection target (see selection index IDS in FIG. 19). In the example illustrated in FIG. 35, a stable period D is detected by a sensor which is different from the sensor 2 used for detecting the focused stable period. In this case, the semi-stable period selection unit 14 may exclude a semi-stable period protruding ahead of an ending point of time of the stable period D, from the selection target. That is, the semi-stable period selection unit 14 may select a semi-stable period belonging into a period T35 which is started from the ending point of time of the stable period D.

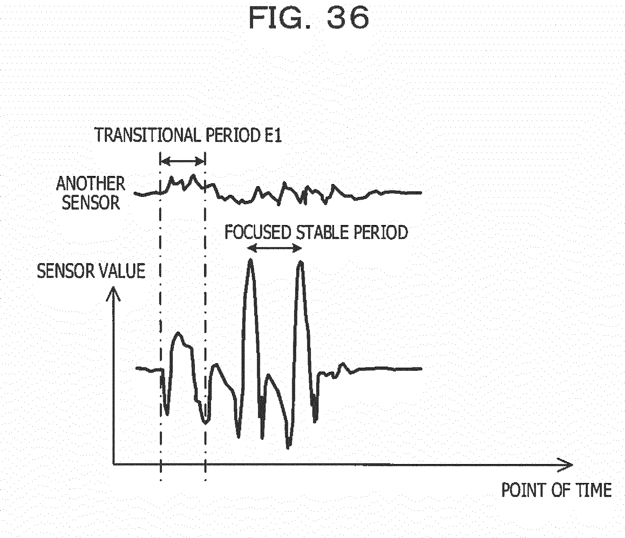

In addition, when the transitional period has been already detected by a sensor which is different from the sensor 2 used for detecting the focused stable period, the semi-stable period selection unit 14 may select a semi-stable period including the detected transitional period prior to other semi-stable periods. This is because the calculation method of the transitional period which has been already detected may be used. For example, in the example illustrated in FIG. 36, a transitional period E1 is detected by a sensor which is different from the sensor 2 used for detecting the focused stable period. In this case, the semi-stable period selection unit 14 may select a semi-stable period including the transitional period E1 prior to other semi-stable periods (see selection index ID6 in FIG. 19). The selection method may be employed under a condition that the transitional period E1 is detected as a transitional period relating to the same movement or posture as a movement or posture which relates to the focused stable period.

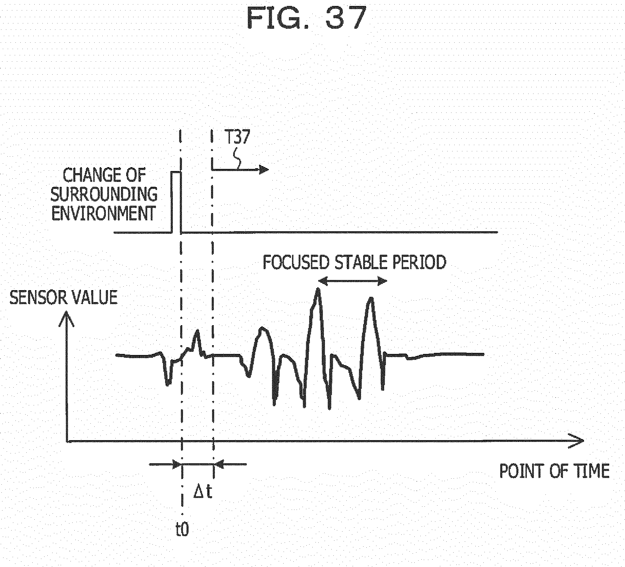

When the surrounding environment is changed, the semi-stable period selection unit 14 may exclude a semi-stable period overlapping a minute time of the point of time, from the selection target (see selection index ID7 in FIG. 19). This is because noises and the like occurring due to a change of the surrounding environment have an influence on the sensor value just after the surrounding environment is changed. This is because there is high probability of not being the transitional period just after the surrounding environment is changed. This is because, for example, when a change of the surrounding environment, for example, moving of a chair, occurs relating to the transitional period of the gait, there is low probability of initiating the gait just after the change. For example, in the example illustrated in FIG. 37, the change of the surrounding environment is detected at the point of time t0. In this case, the semi-stable period selection unit 14 may exclude a semi-stable period overlapping a minute time $\Delta t$ of the point of time t0, from the selection target. That is, the semi-stable period selection unit 14 may select a semi-stable period belonging into a period T37 which is started after the minute time $\Delta t$ of a point of time at which the surrounding environment is changed.

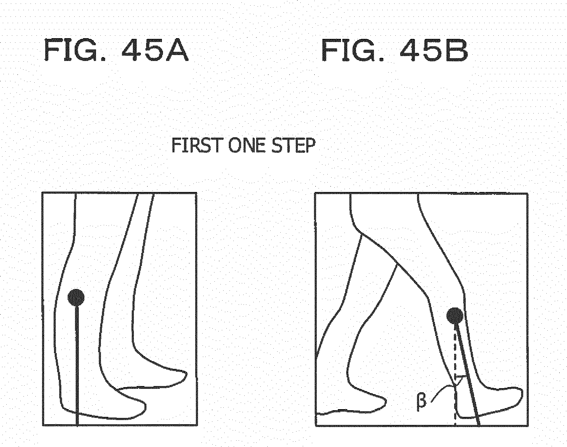

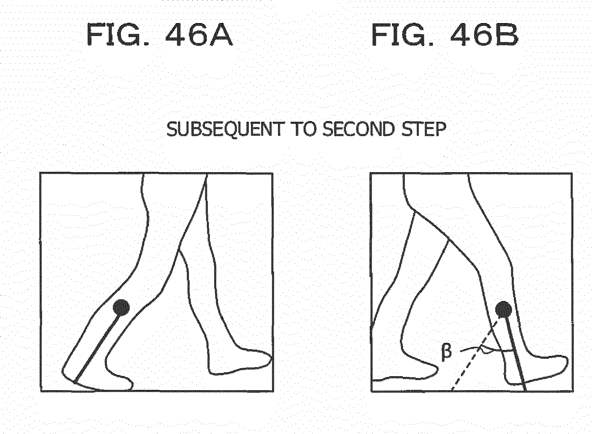

When the focused stable period is the stable period of the gait, the semi-stable period selection unit 14 may select a semi-stable period which satisfies a constraint condition in the gait initiation. When the focused stable period is the stable period of the gait, the semi-stable period selection unit 14 may select a semi-stable period which satisfies the constraint condition in the gait initiation and has a period length of the maximum value (see selection index ID8 in FIG. 19). The constraint condition in the gait initiation may include a condition relating to a relationship between a changing angle of the leg at a first one step and a changing angle of the leg at a time of being subsequent to the next one step (second step). Specifically, the semi-stable period selection unit 14 may determine that the constraint condition of the gait initiation is satisfied, when the changing angle of the leg at the time of the first one step is smaller than that at the time of being subsequent to the second step. This is because, generally, a distance moved by the leg on a stepping side or an angle obtained by the remaining leg being inclined forward with moving of the gait at a time of the first one step from a state where both of the legs are set is smaller than the distance or the angle relating to being subsequent to the second step, in a case of the gait (see FIGs. 45 and 46). Next, the selection method based on the constraint condition in the gait initiation will be described in more details with reference to FIGs. 38 to 48.

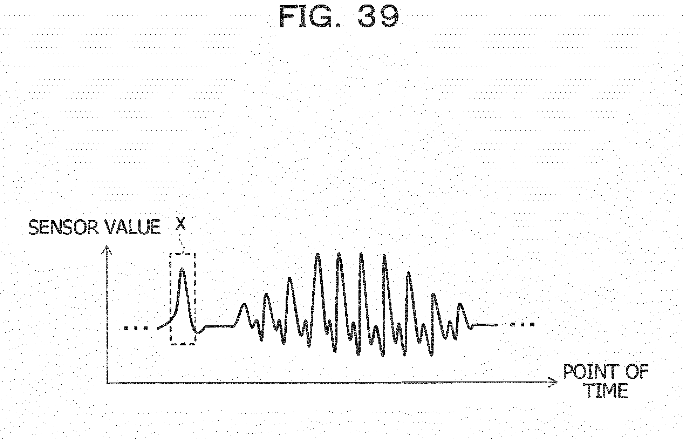

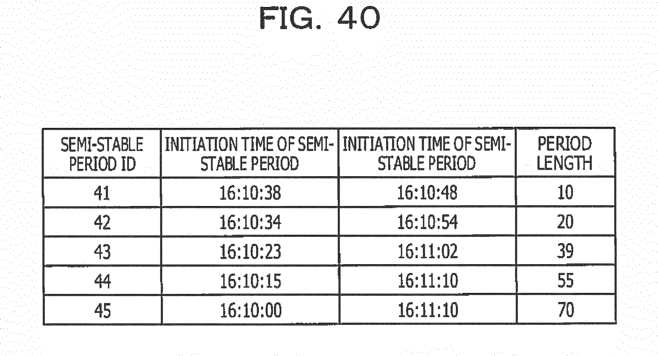

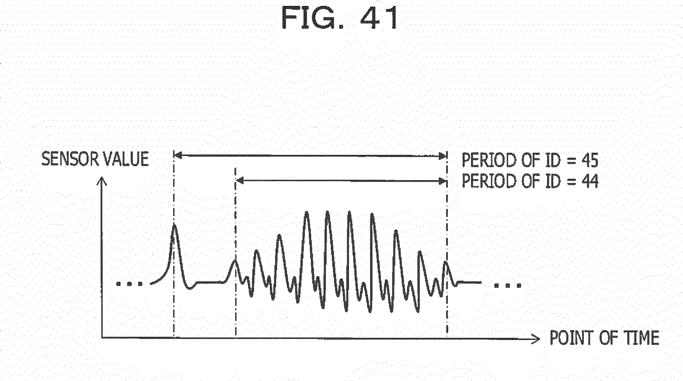

FIG. 38 is a flowchart illustrating an example of the process of Step S1312 in FIG. 13. Here, the processes illustrated in FIG. 38 will be described with reference to FIGs. 39 to 48. FIG. 39 illustrates an example of the time series data including the focused stable period which relates to the gait. FIGs. 40 to 48 illustrate processing results and the like based on the time series data illustrated in FIG. 39. In the example illustrated in FIG. 39, a movement occurs when an object is picked before the gait initiation (see FIG. 10) and a peak of the sensor value is generated by the movement, as illustrated at an X part in FIG. 39. FIG. 40 is a diagram illustrating an example of the plurality of semi-stable periods which are enumerated based on the time series data illustrated in FIG. 39, in Step S1310. FIG. 41 is a diagram illustrating a relationship between the semi-stable period ID = 44 and the semi-stable period ID = 45, and the time series data.

In Step S3800, the semi-stable period selection unit 14 determines whether or not the focused stable period is a stable period relating to the gait. When the focused stable period is a stable period relating to the gait, the process proceeds to Step S3804. In other cases, the process proceeds to Step S3802.

In Step S3802, the semi-stable period selection unit 14 selects one semi-stable period from the plurality of semi-stable periods which are enumerated in Step S1310, based on the selection index in accordance with a movement or a posture relating to the focused stable period. The semi-stable period selection unit 14 outputs the semi-stable period which is selected in this manner, as the second period.

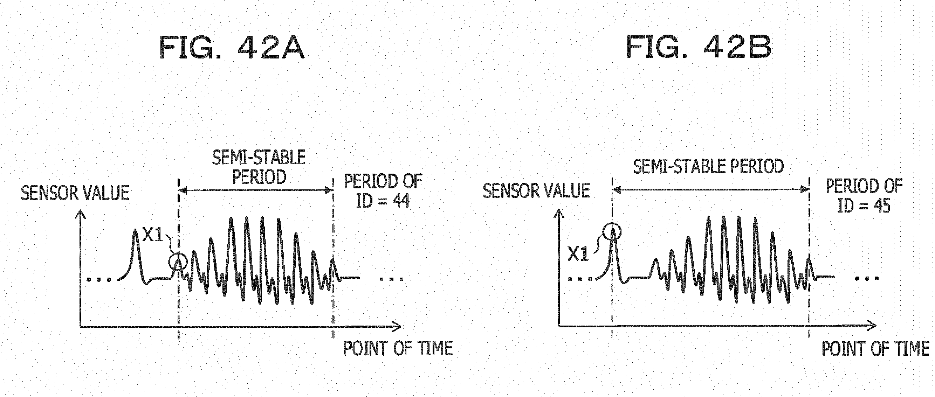

In Step S3804, the semi-stable period selection unit 14 calculates the changing angle of the leg at the first one step in each of the plurality of semi-stable periods which are enumerated in Step S1310. The first one step is one step at first during the semi-stable period. The changing angle is a changing quantity of an angle (changing angle $\beta$) before and after stepping forward, and the angle is an angle of the leg on the stepping side to the ground, as illustrated in FIGs. 45A and 46B. FIGs. 42A and 42B are diagrams illustrating a specifying method of the first one step in the semi-stable period. FIG. 42A relates to a semi-stable period of the semi-stable period ID = 44. FIG. 42B relates to a semi-stable period of the semi-stable period ID = 45. As illustrated by X1 in FIGs. 42A and 42B, the point of time of the first one step is specified based on a position of the first peak of sensor values in the semi-stable period. Thus, in a case of the semi-stable period of the semi-stable period ID = 45, not the point of time of the first one step but a point of time of a peak position by the movement when an object is picked before the gait initiation is actually specified (erroneously) as "the point of time of the first one step".

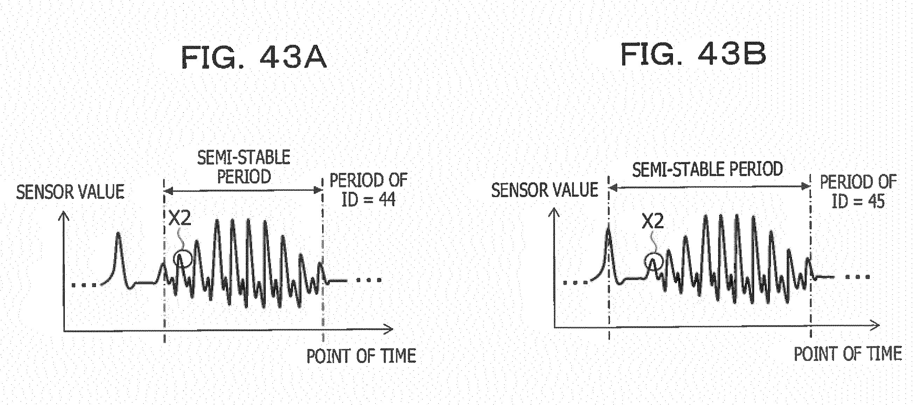

In Step S3806, the semi-stable period selection unit 14 calculates the changing angle of the leg at the next one step in each of the plurality of semi-stable periods which are enumerated in Step S1310. The next one step corresponds to the second step in the semi-stable period. FIGs. 43A and 43B are diagrams illustrating the specifying method of the next one step in the semi-stable period. FIG. 43A relates to a semi-stable period of the semi-stable period ID = 44. FIG. 43B relates to a semi-stable period of the semi-stable period ID = 45. As illustrated by X2 in FIGs. 43A and 43B, the point of time of the second step is specified based on a position of the second peak of sensor values in the semi-stable period. Thus, in a case of the semi-stable period of the semi-stable period ID = 45, not the point of time of the second step but the point of time of the first one step is actually specified (erroneously) as "the point of time of the second step".

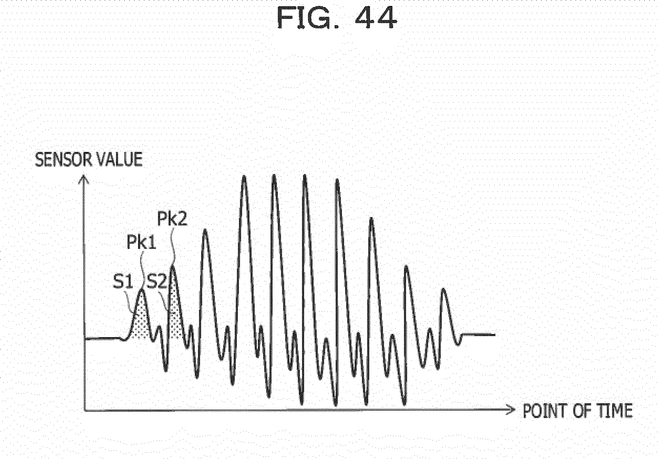

FIG. 44 is a diagram illustrating an example of a calculation method of the changing angle of the leg. FIGs. 45A and 45B are diagrams illustrating the changing angle of the leg at the first step. FIGs. 46A and 46B are diagrams illustrating the changing angle of the leg at the second step. In FIG. 44, a position of a peak specified as the first step is indicated by "Pk1", and a position of a peak specified as the second step is indicated by "Pk2". The semi-stable period selection unit 14 calculates the changing angle β of the leg based on the following expression, for example.

$$\beta = \sum_{t=t_n}^{t_m} X[t]$$

[Math 4]

Here, X[t] indicates a sensor value at a point t of time. tn and tm respectively indicate a starting point of time of a peak and an ending point of time thereof. For example, in a case of the changing angle of the leg at the first step, the points tn and tm of time may be respectively a point of time at which a very small value before and after the peak specified as the first step is obtained and a point of time at which intersecting with a base line is performed. Similarly, in a case of the changing angle of the leg at the second step, the points tn and tm of time may be respectively a point of time at which a very small value before and after the peak specified as the second step is obtained and a point of time at which intersecting with a base line is performed. When the expression of Math 4 is used, the changing angle β of the leg at the first step corresponds to an area (integrated value) S1 relating to the position Pk1 of the peak illustrated in FIG. 44. The changing angle β of the leg at the second step corresponds to an area S2 relating to the position Pk2 of the peak illustrated in FIG. 44.

In Step S3808, the semi-stable period selection unit 14 extracts a semi-stable period among the plurality of semi-stable periods which are enumerated in Step S1310, in which the changing angle of the leg at the first step is smaller than that at the second step. When there is no semi-stable period which satisfies the constraint condition, the semi-stable period selection unit 14 may select a semi-stable period based on other selection indices.

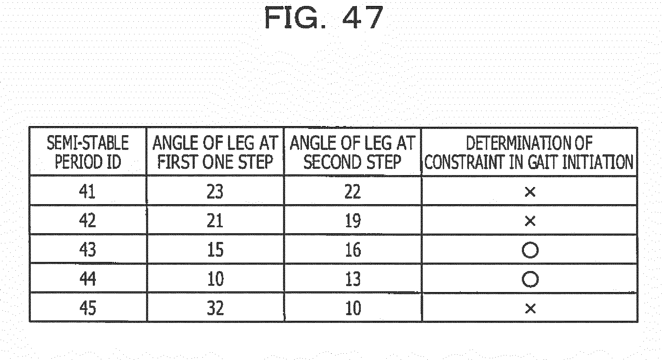

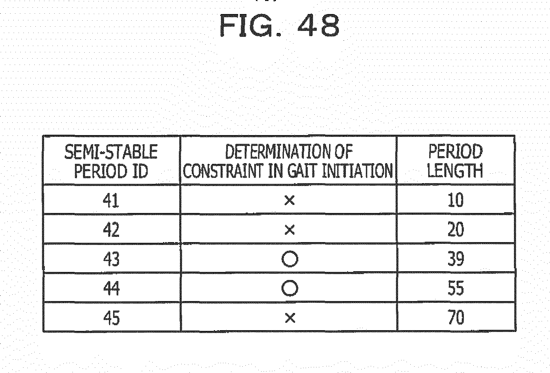

In Step S3810, the semi-stable period selection unit 14 selects a semi-stable period having the maximum period length, among the plurality of semi-stable periods which are enumerated in Step S3808. When the semi-stable period extracted in Step S3808 is one, the semi-stable period selection unit 14 selects the semi-stable period itself. The semi-stable period selection unit 14 outputs the semi-stable period which is selected in this manner, as the second period. FIG. 47 is a diagram illustrating an example of the calculation results in Steps S3804 and S3806, and an example of the determination result of the constraint condition in the gait initiation. For example, in the example illustrated in FIG. 47, the constraint condition in the gait initiation is satisfied in the semi-stable period of the semi-stable period ID = 43 and the semi-stable period of the semi-stable period ID = 44. In this case, the semi-stable period selection unit 14 selects the semi-stable period of the semi-stable period ID = 44 which has a long period length, as illustrated in FIG. 48.

In a method of simply selecting the semi-stable period having the maximum period length, the semi-stable period of the semi-stable period ID = 45 is selected. However, the first one step in the semi-stable period of the semi-stable period ID = 45 is actually not the first one step, but a movement when an object is picked before the gait initiation, as described above. In this manner, in the method of simply selecting the semi-stable period having the maximum period length, detecting of the semi-stable period (for example, semi-stable period including only the transitional period) with high accuracy may be difficult.

In this manner, according to the processes illustrated in FIG. 38, constraint condition in the gait initiation is imposed in comparison to the method of simply selecting the semi-stable period having the maximum period length. Thus, it is possible to detect a semi-stable period with high accuracy. That is, there is high probability of selecting a semi-stable period which does not include a period for a movement having no relationship with gait before the gait initiation. As a result, it is possible to improve accuracy for the transitional period specified based on the semi-stable period selected by the semi-stable period selection unit 14.

In the processes illustrated in FIG. 38, the changing angle is used as the constraint condition in the gait initiation. However, other parameter having one-to-one correspondence to the changing angle may be equivalently used. For example, instead of the changing angle, the changing quantity (that is, moving distance of the stepping foot) at a position of the stepping foot, which is a parameter substantially equivalent to the changing angle may be used.

The above-described constraint condition in the gait initiation may be replaced with a constraint condition in stopping of the gait. In this case, the semi-stable period selection unit 14 may determine that the changing angle of the leg at a time of the last one step satisfies the constraint condition in stopping of the gait, when the changing angle of the leg at a time of the last one step is smaller than that at a step before one step of the last one step.

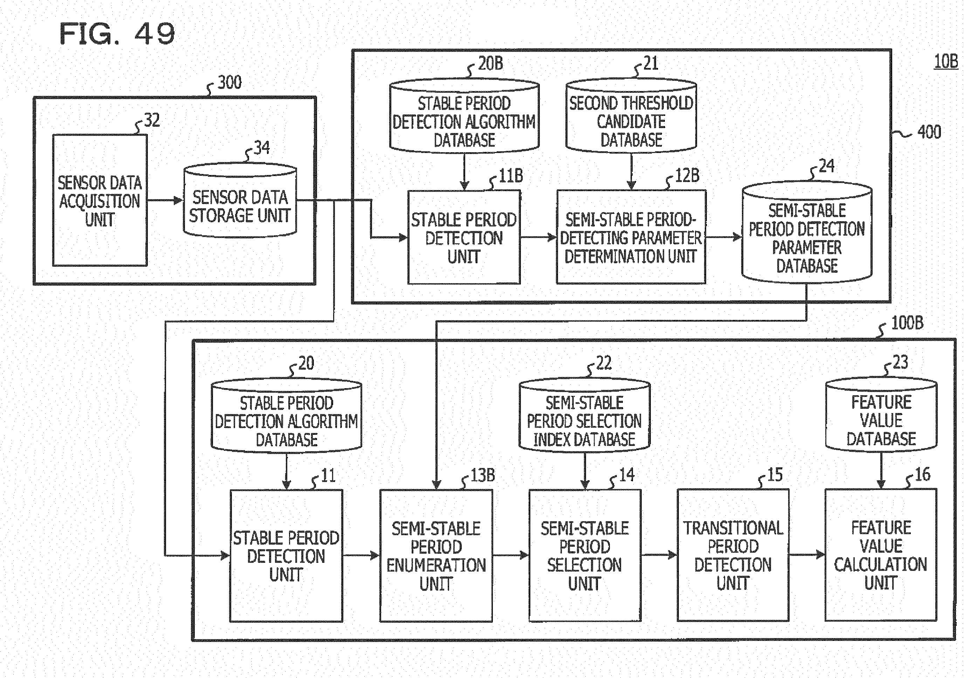

Next, a configuration example and an operation example of a measuring apparatus 10B as another example of the measuring apparatus 10 will be described with reference to FIGs. 49 to 52.

FIG. 49 is a diagram illustrating another example of the configuration of the measuring apparatus 10B. Components in FIG. 49 which are the same as those of the measuring apparatus 10A illustrated in FIG. 11 are denoted by the same reference signs and descriptions thereof will be omitted.

The measuring apparatus 10B includes a processing device 100B, a data acquisition device 300, and a parameter learning device 400.

The hardware configuration of the processing device 100B may be as illustrated in FIG. 12. The hardware configuration of the data acquisition device 300 may be as illustrated in FIG. 12.

The processing device 100B is different from the above-described processing device 100 of the measuring apparatus 10A in that the processing device 100B does not include the second threshold candidate database 21 and the semi-stable period-detecting parameter determination unit 12, as illustrated in FIG. 49, and the semi-stable period enumeration unit 13 is substituted with a semi-stable period enumeration unit 13B.

The parameter learning device 400 includes a stable period detection unit 11B and a semi-stable period-detecting parameter determination unit 12B. The units may be realized by the processor 101 illustrated in FIG. 12, for example. The parameter learning device 400 includes a stable period detection algorithm database 20B, a second threshold candidate database 21, and a semi-stable period detection parameter database 24. Each of the databases may be realized by the auxiliary memory 103 illustrated in FIG. 12, for example. The stable period detection algorithm database 20B may be the same as the stable period detection algorithm database 20. The stable period detection unit 11B may be the same as the stable period detection unit 11.

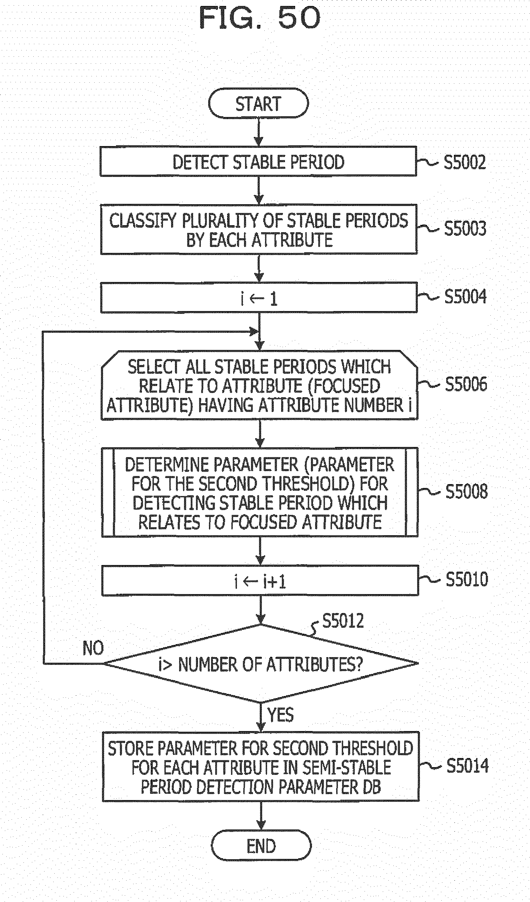

FIG. 50 is a flowchart illustrating an example of processes executed by the parameter learning device 400 in the measuring apparatus 10B according to the example illustrated in FIG. 49. The processes illustrated in FIG. 50 may be executed without synchronization with the processes by the processing device 100B. The processes illustrated in FIG. 50 may be executed based on sensor data (time series data in the sensor data storage unit 34) which is different from the processes by the processing device 100B. The processes illustrated in FIG. 50 may be executed each time the time series data is accumulated in the sensor data storage unit 34. Data in the semi-stable period detection parameter database 24 corresponding to the processes illustrated in FIG. 50 may be updated.

In Step S5002, the stable period detection unit 11B acquires the time series data in the sensor data storage unit 34 and detects a stable period based on the acquired time series data.

In Step S5003, the semi-stable period-detecting parameter determination unit 12B classifies the plurality of stable periods (stable periods which are timely separated from each other) by each attribute. The attribute represents a movement or a posture relating to the stable period. For example, when the plurality of stable periods relating to the stable period of the gait is detected, the plurality of stable periods relating to the stable period of the gait is classified as a group of the same attribute. The attribute numbers which are different from each other for each attribute are assigned to the plurality of stable periods. Here, the attribute number is assigned in ascending order from 1, as an example.

In Step S5004, the semi-stable period-detecting parameter determination unit 12B sets a value i to an initial value of "1".

In Step S5006, the semi-stable period-detecting parameter determination unit 12B selects (extracts) all stable periods which relate to an attribute having an attribute number of the value i. In the following descriptions, the attribute relating to the extracted stable period is also referred to a "focused attribute".

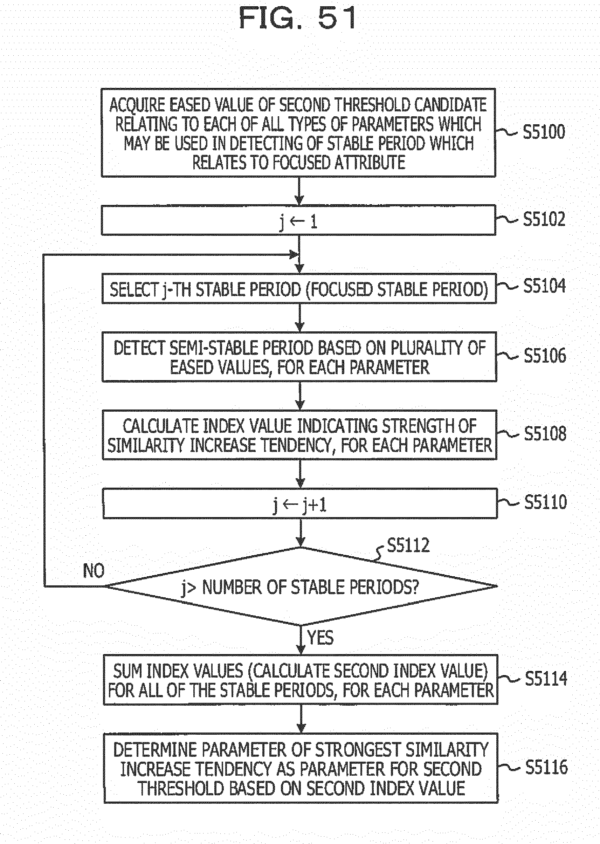

In Step S5008, the semi-stable period-detecting parameter determination unit 12B determines a parameter appropriate for detecting a semi-stable period which relates to the focused attribute, as the parameter for the second threshold, which relates to the focused attribute with reference to the second threshold candidate database 21. The process will be described later with reference to FIG. 51.

In Step S5010, the semi-stable period-detecting parameter determination unit 12B performs increment of "1" on the value i.

In Step S5012, the semi-stable period-detecting parameter determination unit 12B determines whether or not the value i is greater than the number of attributes (the number of attributes classified in Step S5003). That is, the semi-stable period-detecting parameter determination unit 12B determines whether or not the parameter for the second threshold, which relates to each of all of the attributes relating to the detected stable period is determined. When the value i is greater than the number of attributes, the process proceeds to Step S5014. When the value i is not greater than the number of attributes, the process returns to Step S5006 and the processes of Steps S5006 to S5008 are executed for an attribute (new attribute) having an attribute number of the value i.

In Step S5014, the semi-stable period-detecting parameter determination unit 12B stores the parameter for the second threshold which is determined for each attribute in the semi-stable period detection parameter database 24 in a state of being associated with each attribute. At this time, the semi-stable period-detecting parameter determination unit 12B may store the eased value of the second threshold candidate relating to the parameter for the second threshold which is determined for each attribute, based on data (eased value of the second threshold candidate) in the second threshold candidate database 21.

FIG. 51 is a flowchart illustrating an example of the process of Step S5008 in FIG. 50.

In Step S5100, the semi-stable period-detecting parameter determination unit 12B acquires the eased value of the second threshold candidate relating to each of all types of parameters which may be used in detecting of a stable period relating to the focused attribute, with reference to the second threshold candidate database 21. For example, when the focused attribute is the gait, the semi-stable period-detecting parameter determination unit 12B acquires the eased value of the second threshold candidate relating to each of all types of parameters which may be used in detecting of a stable period relating to the gait. The eased value of the second threshold candidate relating to each of all types of parameters may be acquired from the second threshold candidate database 21 (see FIG. 21).

In Step S5102, the semi-stable period-detecting parameter determination unit 12B sets the value j to the initial value of "1".

In Step S5104, the semi-stable period-detecting parameter determination unit 12B selects the j-th stable period as the focused stable period.

In Step S5106, the semi-stable period-detecting parameter determination unit 12B detects a semi-stable period based on the plurality of eased values, for each of the plural types of parameters. The semi-stable period-detecting parameter determination unit 12B calculates the length (period length) of the semi-stable period of each of the eased values. In this manner, the period length of the semi-stable period of each of the eased values is calculated for each of the plural types of parameters.

In Step S5108, the semi-stable period-detecting parameter determination unit 12B calculates the index value (evaluation value) indicating the strength of the similarity increase tendency, for each of the plural types of parameters. The index value Indicating the strength of the similarity increase tendency may be calculated as the index value (evaluation value) indicating the strength of the monotonous increase tendency as represented by Math 3, for example. In the following descriptions, the index value is referred to as a "first index value".

In Step S5110, the semi-stable period-detecting parameter determination unit 12B performs increment of "1" on the value j.

In Step S5112, the semi-stable period-detecting parameter determination unit 12B determines whether or not the value j is greater than the number of stable periods relating to the focused attribute. That is, the semi-stable period-detecting parameter determination unit 12B determines whether or not the first index value for all of the stable period relating to the focused attribute is calculated. When the value j is greater than the number of stable periods relating to the focused attribute, the process proceeds to Step S5114. When the value j is not greater than the number of stable periods relating to the focused attribute, the process returns to Step S5104. Then, the processes of Steps S5106 to S5108 are executed for the j-th stable period (new focused stable period).

In Step S5114, the semi-stable period-detecting parameter determination unit 12B calculates a second index value E'($\alpha$) by summing first index values which are calculated for all of the stable periods, for each of the plural types of parameters. The second index value E'($\alpha$) may be as follows.

$$E'(\alpha) = \sum_{j=1}^{J_i} \left\{ \frac{1}{M_\alpha} \sum_{m=1}^{M_\alpha} \sigma\left(L_{\alpha_m}^{(j)}\right) \right\}$$

[Math 5]

Here, $\alpha$ indicates the type of the parameter, and is as in Math 2. $J_i$ indicates the number of stable periods relating to the attribute having an attribute number of the value i. $L_{\alpha m}(j)$ indicates the period length of a semi-stable period relating to a stable period which relates to the value j, and indicates the period length of the semi-stable period based on the eased value of the mitigation ID = m, which relates to the parameter $\alpha$. If $M_\alpha$ is set to the maximum value of mitigation IDs relating to the parameter $\alpha$, $\sigma(L_{\alpha m}(j))$ is as follows.

$$\sigma\left(L_{\alpha_m}^{(J)}\right)=\begin{cases} 1 & \left(if\ \ L_{\alpha_m}^{(J)} > L_{\alpha_{m-1}}^{(J)}\right) \\ -1 & \left(if\ \ L_{\alpha_m}^{(J)} < L_{\alpha_{m-1}}^{(J)}\right) \\ 0 & (otherwise) \end{cases}$$

[Math 6]

In Step S5116, the semi-stable period-detecting parameter determination unit 12B determines a parameter for calculating the second index value E' indicating that the similarity increase tendency is strongest, as the parameter for the second threshold relating to the focused attribute. The second index value E' indicates that the similarity increase tendency becomes strongest as the second index value E' is increased.

According to the processes illustrated in FIGs. 50 and 51, a parameter (parameter for the second threshold) appropriate for detecting a semi-stable period from the stable period relating to the attribute may be determined based on a summation (second index value) of first evaluation values relating to all of the stable periods which relate to the attribute, for each attribute. Thus, it is possible to determine the parameter for the second threshold based on the summation (second index value) of the first evaluation values relating to the plurality of stable periods, with high reliability.

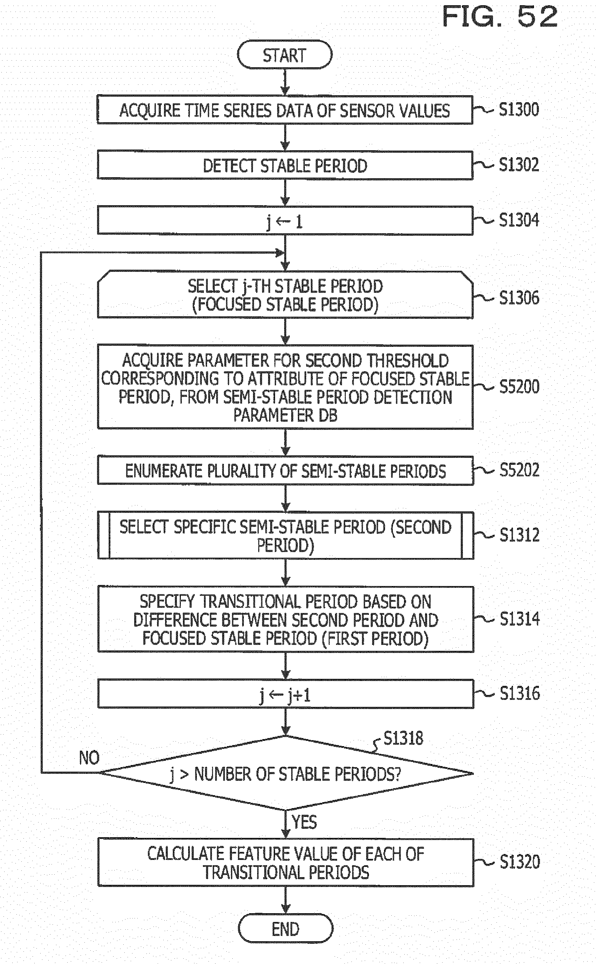

FIG. 52 is a flowchart illustrating an example of the processes executed by the data acquisition device 300 and the processing device 100B.

The processes illustrated in FIG. 52 are different from the processes illustrated in FIG. 13 in that the processes of Steps S5200 and S5202 are executed instead of the processes of Steps S1308 and S1310 of the processes illustrated in FIG. 13.

In Step S5200, the semi-stable period enumeration unit 13B acquires the parameter for the second threshold corresponding to the attribute of the focused stable period, from the semi-stable period detection parameter database 24. When the eased value of the second threshold candidate relating to the parameter for the second threshold is stored in the semi-stable period detection parameter database 24, instead of or in addition to the parameter for the second threshold, the semi-stable period enumeration unit 13B acquires the eased value. When the eased value of the second threshold candidate relating to the parameter for the second threshold is not stored in the semi-stable period detection parameter database 24, the semi-stable period enumeration unit 13B may generate the eased value of the second threshold candidate based on the parameter for the second threshold.

In Step 55202, the semi-stable period enumeration unit 13B enumerates the plurality of semi-stable periods based on the eased value of the second threshold candidate relating to the parameter for the second threshold, which is acquired in Step S5200. The method is as described above.

Most of the above descriptions relates to the transitional period of the gait. However, the above descriptions may be similarly applied for a movement or a posture other than the gait. An application example for the movement or the posture other than the gait will be described below.

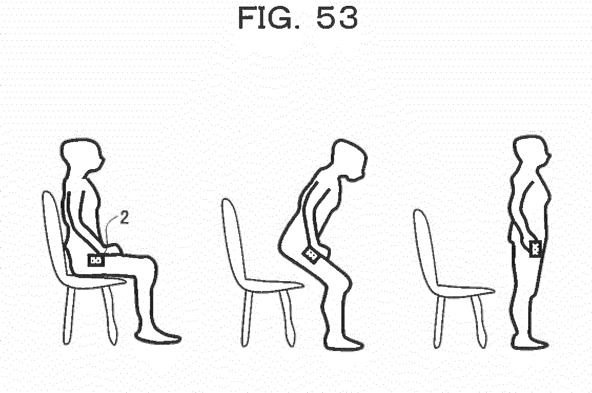

An application example for a standing position and a sitting position (example of the posture) will be described with reference to FIGs. 53 to 63.

FIG. 53 is a diagram illustrating a transitional state for the standing position. FIG. 53 schematically illustrates a movement of a person from a state (sitting position) of sitting on a chair to the standing position. The sensor 2 is illustrated in FIG. 53. Here, as the sensor 2, an acceleration sensor is used. For example, the sensor 2 may be the acceleration sensor mounted in a portable terminal which is in a pocket of the person.

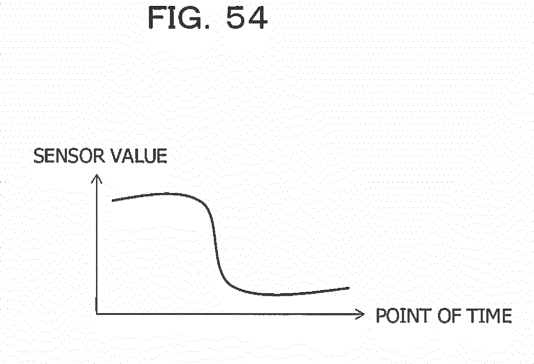

FIG. 54 is a diagram illustrating an example of the time series data of the sensor 2 obtained at a time of the movement illustrated in FIG. 53. In the example illustrated in FIG. 54, regarding a detection direction of acceleration by the sensor 2, a downward direction in the posture of the sitting position in FIG. 53 is set to be positive.

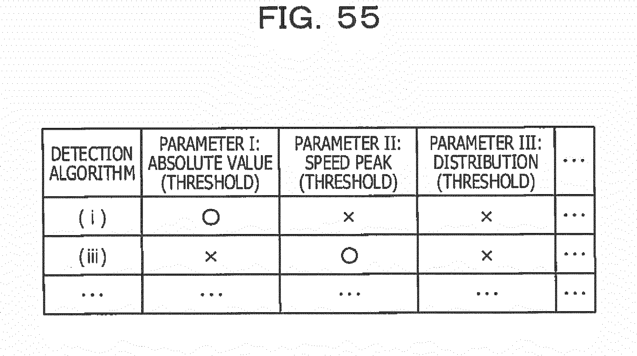

FIG. 55 is a diagram illustrating an example of a detection algorithm of a stable period at the standing position. The detection algorithm of the stable period at the standing position may be stored in the stable period detection algorithm database 20. In the example illustrated in FIG. 55, a first threshold for detecting the stable period at the standing position includes a threshold (for example, range of sensor values) for the size (absolute value) of the sensor values, a threshold relating to a peak of a speed (for example, acquired by integrating the sensor values), a threshold relating to distribution of the sensor values, and the like. A specific value of the first threshold may be adapted based on the real data relating to the standing position, similarly to other movement or posture such as the gait.

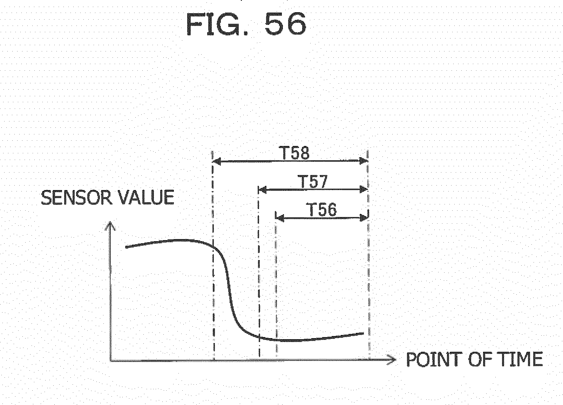

FIG. 56 is a diagram illustrating an example of a plurality of enumerated semi-stable periods. The semi-stable period may be detected based on the plurality of eased values of the second threshold candidate relating to a parameter for detecting the stable period at the standing position, similarly to the above descriptions. In the example illustrated in FIG. 56, two semi-stable periods T57 and T58 detected based on the plurality of eased values of the second threshold candidate relating to the size (absolute value) of the sensor values are illustrated. The semi-stable periods T57 and T58 relating to the standing position are typically detected in a form of including a period (candidate of the transitional period) which is not included in the stable period T56, ahead of the stable period T56, as illustrated in FIG. 56.

Similarly to the case of the gait, the measuring apparatus 10 specifies the second period based on the second threshold relating to a parameter which has a similarity increase tendency. For example, the measuring apparatus 10 specifies the second period among the plurality of enumerated semi-stable periods, based on the plurality of eased values of the second threshold candidate which relates to the parameter having the similarity increase tendency. In this case, the parameter for the second threshold may be a parameter which has a similarity increase tendency only on a side ahead of the stable period. Similarly to the case of the gait, the measuring apparatus 10 specifies a transitional period based on a difference between the second period and the first period (stable period). In a case of the standing position, the sitting position, or the like, the measuring apparatus 10 may specify the transitional period only ahead of the first period (stable period). That is, when the second period includes a period which is not included in the first period (stable period), after the first period (stable period), the period may be excluded from the transitional period.

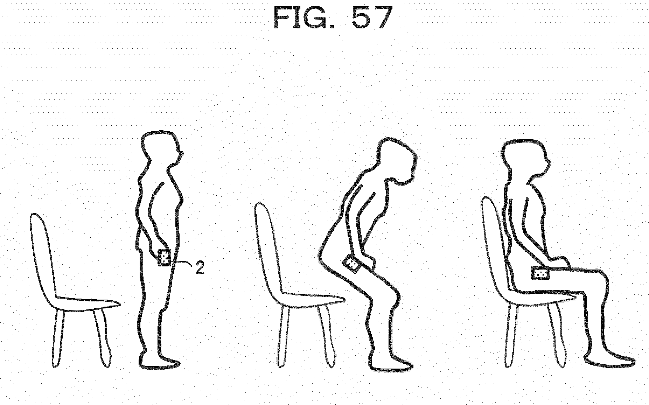

FIG. 57 is a diagram illustrating the transitional state for the sitting position. FIG. 57 schematically illustrates a movement of a person from a standing state (standing position) to the sitting position. The sensor 2 is illustrated in FIG. 57. Here, the sensor 2 is set to be an acceleration sensor, similarly.

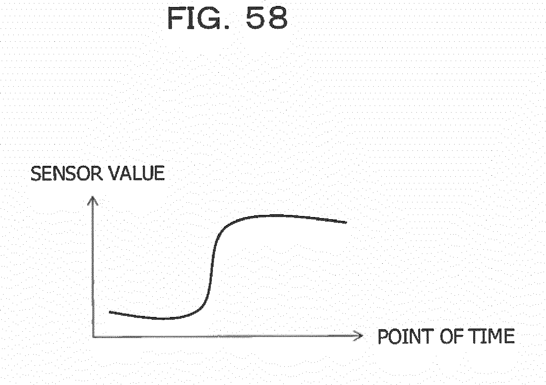

FIG. 58 is a diagram illustrating an example of the time series data of the sensor 2 obtained at a time of the movement illustrated in FIG. 57. In the example illustrated in FIG. 58, regarding a detection direction of acceleration by the sensor 2, a downward direction in the posture of the sitting position in FIG. 57 is set to be positive.

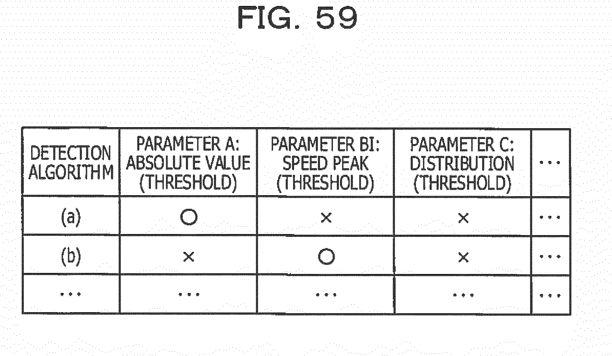

FIG. 59 is a diagram illustrating an example of a detection algorithm of a stable period at the sitting position. The detection algorithm of the stable period at the sitting position may be stored in the stable period detection algorithm database 20. In the example illustrated in FIG. 59, a first threshold for detecting the stable period at the sitting position includes a threshold (for example, range of sensor values) for the size (absolute value) of the sensor values, a threshold relating to a peak of a speed, a threshold relating to distribution of the sensor values, and the like. A specific value of the first threshold may be adapted based on the real data relating to the sitting position, similarly to other movement or posture such as the gait.

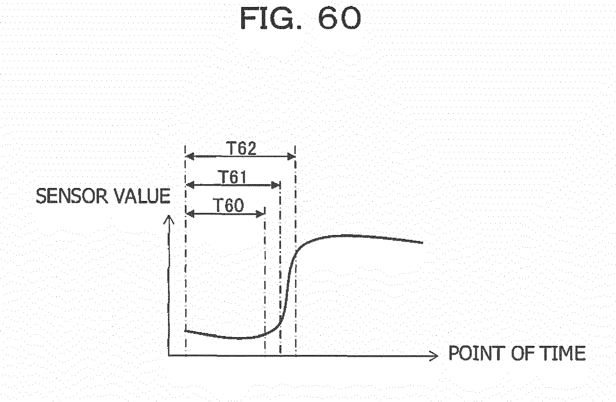

FIG. 60 is a diagram illustrating an example of a plurality of enumerated semi-stable periods. The semi-stable period may be detected based on the plurality of eased values of the second threshold candidate relating to a parameter for detecting the stable period at the sitting position, similarly to the above descriptions. In the example illustrated in FIG. 60, two semi-stable periods T61 and T62 detected based on the plurality of eased values of the second threshold candidate relating to the size (absolute value) of the sensor values are illustrated. The semi-stable periods T61 and T62 relating to the sitting position are typically detected in a form of including a period (candidate of the transitional period) which is not included in the stable period T60, after the stable period T60, as illustrated in FIG. 60.

Similarly to the case of the gait, the measuring apparatus 10 specifies the second period based on the second threshold relating to a parameter which has a similarity increase tendency. For example, the measuring apparatus 10 specifies the second period among the plurality of enumerated semi-stable periods, based on the plurality of eased values of the second threshold candidate which relates to the parameter having the similarity increase tendency. In this case, the parameter for the second threshold may be a parameter which has a similarity increase tendency only on a side after the stable period.

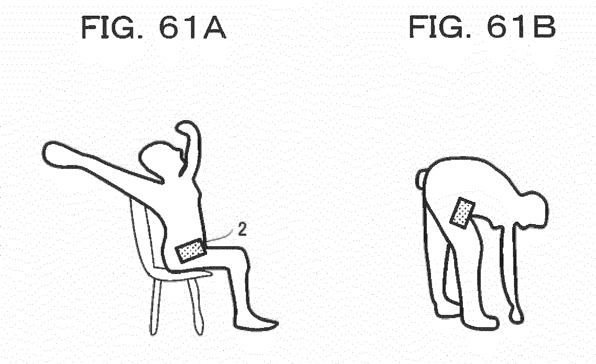

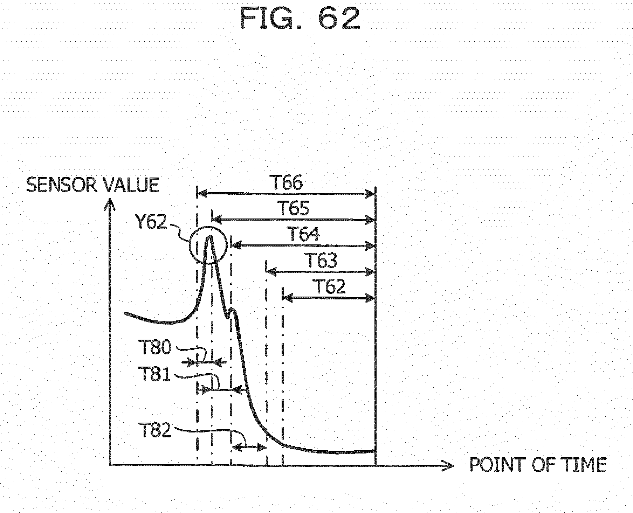

FIG. 62 is a diagram illustrating a constraint condition relating to the standing position. In the example illustrated in FIG. 62, as illustrated by Y62 in FIG. 62, sensor values has a peak caused by stretching at the sitting position as illustrated in FIG. 61A. In FIG. 62, five semi-stable periods T62 to T66 detected based on the plurality of eased values of the second threshold candidate are enumerated.

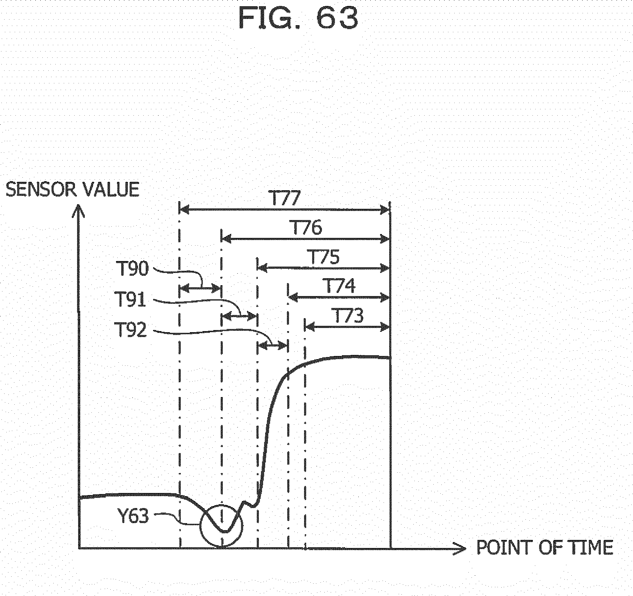

FIG. 63 is a diagram illustrating a constraint condition relating to the sitting position. In the example illustrated in FIG. 63, as illustrated by Y63 in FIG. 63, sensor values has a peak caused by a movement of picking an object at the sitting position as illustrated in FIG. 61B. In FIG. 63, five semi-stable periods T73 to T77 detected based on the plurality of eased values of the second threshold candidate are enumerated.

Similarly to the case of the gait, various movements are included before and after the stable period of the standing position or the sitting position, in addition to the movement of the transitional state which is originally desired to be extracted. For example, transition to the standing position may occur because stretching is performed at the sitting position as illustrated in FIG. 61A. Transition to the sitting position may occur because the movement of picking an object at the sitting position as illustrated in FIG. 61B is performed. Thus, similarly to the case of the gait, the measuring apparatus 10 may specify a semi-stable period which satisfies a predetermined constraint condition, as the second period.

For example, the constraint condition represented by the following expression may be used.

$$D(k) < Th$$

$$D(k) = \left| R\left(X\left[t_s^{(k)} : t_s^{(k-1)}\right]\right) - R\left(X\left[t_s^{(k+1)} : t_s^{(k)}\right]\right)\right|$$

[Math 7]

Here, R(X[to:tp]) indicates a regression coefficient of sensor data for a period from a point to of time until a point tp of time. ts(k) indicates an initiation time of a semi-stable period relating to the eased value ID = k. Thus, for example, R(X[ts(k):ts(k-1)]) indicates a regression coefficient of sensor data for a period from the initiation time ts(k) of the semi-stable period relating to the eased value ID = k until the initiation time ts(k-1) of a semi-stable period relating to the eased value ID = k-1.

In the expression of Math 7, X[ts(k):ts(k-1)] represents characteristics of a period extended ahead of the semi-stable period when a value of the second threshold candidate is eased (that is, when mitigation is performed from the eased value ID = k-1 to the eased value ID = k). The value of the second threshold candidate is eased, and a portion of the semi-stable period, which is extended on a side ahead of the semi-stable period by the mitigation is also referred to as "an extension period" below. For example, in the example illustrated in FIG. 62, extension periods are respectively indicated by T80 to T82. In the example illustrated in FIG. 63, extension periods are respectively indicated by T90 to T92.

When the extension period is a transitional period, the characteristics (data tendency) in the extension period are proximate to characteristics in extension periods before and after this extension period (or stable period relating to the transitional period). The constraint condition represented by the expression of Math 7 uses similarity of the characteristics. That is, similarity in that a variation dependency of sensor values caught in the semi-stable period is within a certain value is used in the transitional period. This is because a posture is continuously changed to a certain extent and a tendency of a change for which the posture is changed is not large. If the tendency of a change is large, that is because the tendency of a change means a posture having no relationship, not the transitional state of a certain posture. According to the constraint condition represented by the expression of Math 7, when the regression coefficient relating to a certain extension period has an absolute value of a difference of a predetermined value Th or more from the regression coefficient relating to the previous extension period, it is determined that the semi-stable period having the extension period does not satisfy the constraint condition.

In the example illustrated in FIG. 62, characteristics (data tendency) in the extension period T82 are decrease characteristics. Characteristics in the extension period T81 before the extension period T82 are decrease characteristics, and characteristics in the extension period T80 before the extension period T81 are increase characteristics. In this case, it is determined that a semi-stable period T65 having the extension period T81 and a semi-stable period T66 having the extension period T80 do not satisfy the constraint condition. It is determined that a semi-stable period T64 having the extension period T82 satisfies the constraint condition. Thus, in this case, the semi-stable period T64 having the maximum period length may be selected as the second period, among the semi-stable periods T62, T63, and T64 which satisfy the constraint condition.

In the example illustrated in FIG. 63, characteristics (data tendency) in the extension period T92 are increase characteristics. Characteristics in the extension period T91 before the extension period T92 are increase characteristics, and characteristics in the extension period T90 before the extension period T91 are decrease characteristics. In this case, it is determined that a semi-stable period T76 having the extension period T91 and a semi-stable period T77 having the extension period T90 do not satisfy the constraint condition. It is determined that a semi-stable period T75 having the extension period T92 satisfies the constraint condition. Thus, in this case, the semi-stable period T75 having the maximum period length may be selected as the second period, among the semi-stable periods T75, T74, and T73 which satisfy the constraint condition.

Most of the above descriptions relates to the transitional period of a movement or a posture which has a stable period. However, the above descriptions may be similarly applied for a movement (for example, standing action) which substantially does not have the stable period. In this case, in the above descriptions, the "stable period" may be replaced with a "strong action period" and the "semi-stable period" may be replaced with a "weak action period".

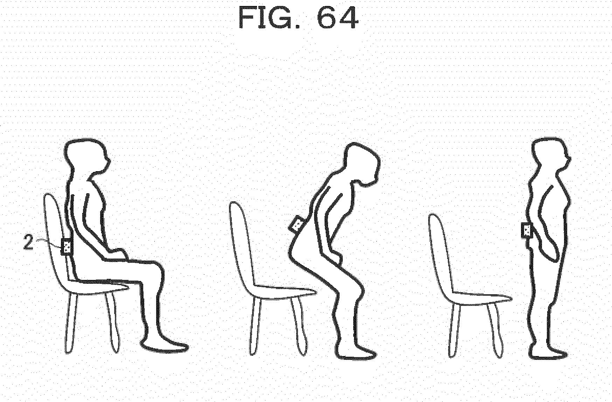

An application example for the standing action (example of a movement which substantially does not have the stable period) will be described with reference to FIGs. 64 to 66.

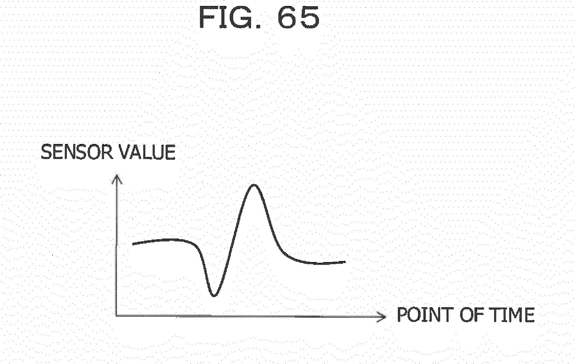

FIG. 64 is a diagram illustrating a transitional state for the standing action. FIG. 64 schematically illustrates a movement of a person from a state (sitting position) of sitting on a chair to the standing position. The sensor 2 is illustrated in FIG. 64. Here, as the sensor 2, a gyro sensor which is attached to the vicinity of the waist of the person is used. FIG. 65 is a diagram illustrating an example of the time series data of the sensor 2 obtained at a time of the movement illustrated in FIG. 64.

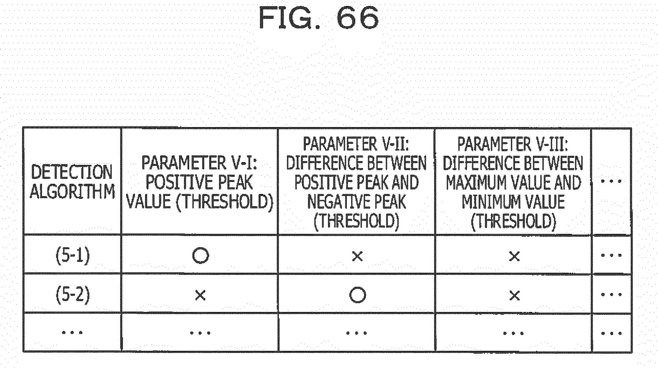

FIG. 66 is a diagram illustrating an example of a detection algorithm of the strong action period in the standing action. The detection algorithm of the strong action period in the standing action may be stored in the stable period detection algorithm database 20. In the example illustrated in FIG. 66, a first threshold for detecting the strong action period in the standing action includes a threshold for a positive peak value of sensor values, a threshold relating to a difference value between a positive peak and a negative peak, a threshold relating to a difference between the maximum value and the minimum value of the sensor values, and the like. A specific value of the first threshold may be adapted based on the real data relating to the standing action, similarly to other movement or posture such as the gait.

In a case of an action (movement which substantially does not have the stable period) which is not stably repeated, such as the standing action, detecting of a transitional state by also including a movement having a fluctuating tendency, such as reactions before and after the action is useful. For example, detecting of the transitional state by also including a forward protruding action and the like just before the standing action is useful. The movement such as the reaction is proximate to movements on both sides before and after that point of time. In the following descriptions, an example of a specifying method of the transitional period relating to the action which is not stably repeated will be described by using the similar characteristics. In the following descriptions, processing that the measuring apparatus 10A according to the example illustrated in FIG. 11 specifies the transitional period relating to the action which is not stably repeated will be described. However, the following descriptions may be also applied similarly in a case of the measuring apparatus 10B according to the example illustrated in FIG. 49.

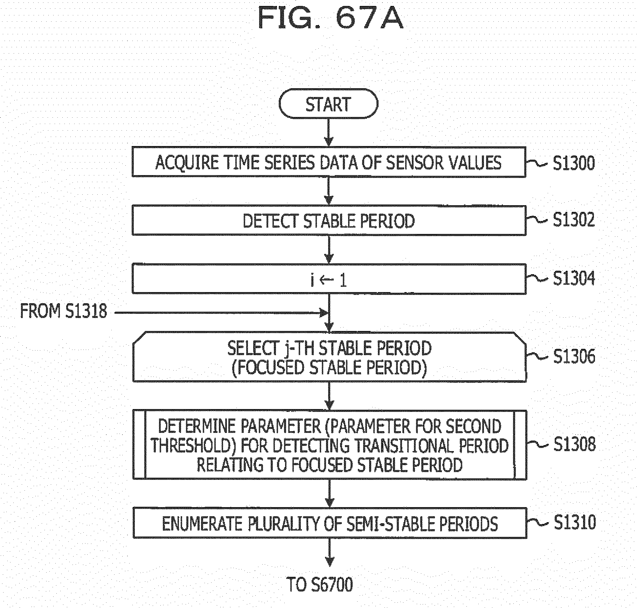

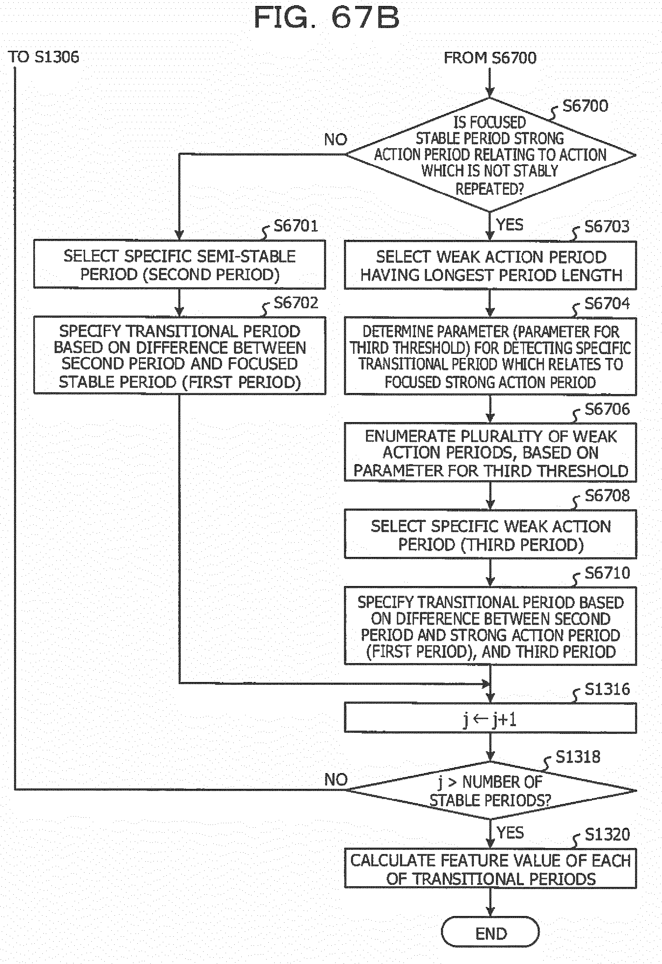

FIGs. 67A and 67B are flowcharts illustrating an example of processing performed by the measuring apparatus 10A when the transitional period relating to the action which is not stably repeated.

The processing illustrated in FIG. 67 is different from the processing illustrated in FIG. 13 in that the processing illustrated in FIG. 67 does not include the processes of Steps S1312 and S1314 and the processes of Steps S6700 to S6710 are added between Step S1310 and Step S1316. In the following descriptions, the processes of Steps S6700 to S6710 will be described mostly.

In Step S6700, the semi-stable period-detecting parameter determination unit 12 determines whether or not the focused stable period is a strong action period relating to an action (movement which substantially does not have the stable period) which is not stably repeated. When the focused stable period is the strong action period, the process proceeds to Step S6703. In other cases, the process proceeds to Step S6701. When the focused stable period is the strong action period, the periods enumerated in Step S1310 are "weak action periods". In the following descriptions, here, an action which is not stably repeated is set to be the standing action, as an example. When the focused stable period is the strong action period, the focused stable period is also referred to as a "focused strong action period".

In Step S6701, similarly to Step S1312 in the processing illustrated in FIG. 13, the semi-stable period selection unit 14 selects one semi-stable period from a plurality of semi-stable periods which are enumerated in Step S1310, with reference to the semi-stable period selection index database 22.

In Step S6702, similarly to Step S1314 in the processing illustrated in FIG. 13, the transitional period detection unit 15 specifies a transitional period based on a difference between the second period obtained in Step S1312 and the focused stable period (first period) selected in Step S1306.

In Step S6703, the semi-stable period selection unit 14 selects a weak action period having the longest period length from a plurality of weak action period which are enumerated in Step S1310, as the second period. The semi-stable period selection unit 14 may ease a value of the second threshold candidate until the period length of a period before the strong action period is not long, and may deduce a weak action period which is extended to an extent that the period length is not long before the strong action period, as the second period.

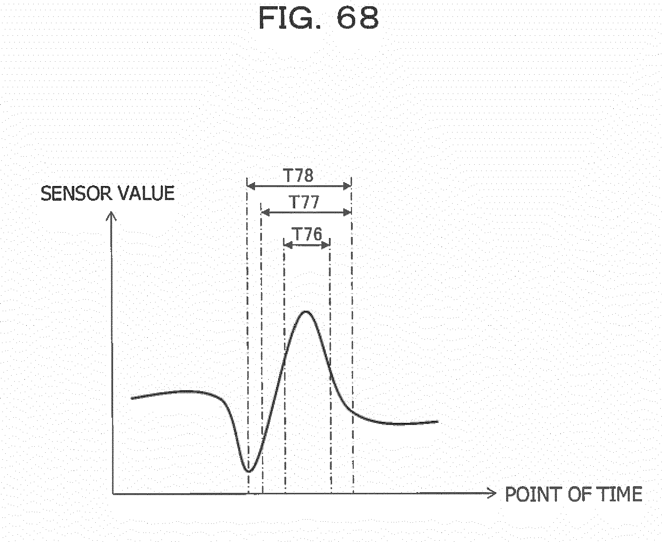

FIG. 68 is a diagram illustrating an example of the plurality of enumerated weak action periods. The weak action period may be detected based on the plurality of eased values of the second threshold candidate relating to a parameter (see FIG. 66) for detecting the strong action period in the standing action, as described above. In the example illustrated in FIG. 68, a strong action period T76 is detected by a first threshold relating to a positive peak value of sensor values. In the example illustrated in FIG. 68, weak action periods T77 and T78 are detected based on the plurality of eased values of the second threshold candidate relating to a difference between the maximum value and the minimum value of the sensor values. The weak action periods T77 and T78 relating to the standing action are typically detected in a form of including a period (candidate of the transitional period) which is not included in the strong action period T76, before and after the strong action period T76, as illustrated in FIG. 68. In the example illustrated in FIG. 68, the weak action period T78 is selected as the second period.

In Step S6704, the semi-stable period-detecting parameter determination unit 12 determines a parameter appropriate for detecting a specific weak action period which relates to the focused strong action period, as a parameter for a third threshold with reference to a third threshold candidate database (not illustrated). The parameter for the third threshold is a parameter for detecting a specific transitional period (for example, period of the forward protruding action just after the standing action) of which specifying is difficult only by using the eased values of the second threshold candidate. Thus, the parameter for the third threshold is typically a parameter of a type which is different from that of the parameter for the second threshold. The semi-stable period-detecting parameter determination unit 12 determines a parameter having a tendency (similarity increase tendency) that a value based on the time series data of the sensor 2 becomes closer to a value in a predetermined period with being close to the predetermined period which has the initiation time of the second period as the center, as the parameter for the third threshold. For example, the semi-stable period-detecting parameter determination unit 12 determines a parameter of which the period length is extended before and after the initiation time of the second period with an increase of the degree of mitigation, as the parameter for the third threshold.

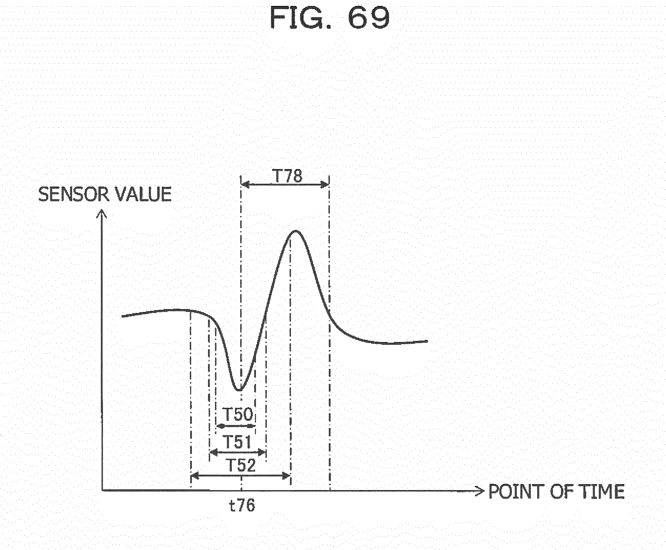

In Step S6706, the semi-stable period enumeration unit 13 enumerates a plurality of weak action periods, based on a plurality of eased values of a third threshold candidate relating to the parameter for the third threshold which is determined in Step S6704. An enumeration method of the weak action periods is as described above, except that the used eased value is different. FIG. 69 is a diagram illustrating an example of the plurality of enumerated weak action periods based on the plurality of eased values of the third threshold candidate. In the example illustrated in FIG. 69, weak action periods T50, T51, and T52 are detected based on the plurality of eased values (eased value having a defined range) of the third threshold candidate which relates to the difference between the maximum value and the minimum value of the sensor values. The weak action periods T50, T51, and T52 have a period length which is extended before and after the initiation time t7 of the second period and the period length becomes longer as the degree of mitigation is increased. A eased value relating to the weak action period T52 has the degree of mitigation which is greater than that of a eased value relating to the weak action period T51. The eased value relating to the weak action period T51 has the degree of mitigation which is greater than that of a eased value relating to the weak action period T50.

In Step S6708, the semi-stable period selection unit 14 selects one weak action period from the plurality of weak action periods which are enumerated in Step S6706, as a third period. A specifying method (selection method) of the third period is similar to the above-described specifying method (selection method) of the second period, and may be any method. As an example, the semi-stable period selection unit 14 selects a weak action period having the longest period length, as the third period. In the example illustrated in FIG. 69, the weak action period T52 is selected as the third period. In this case, a eased value of the third threshold candidate, which is used in obtaining of the weak action period T52 which is selected as the third period is employed as the third threshold.

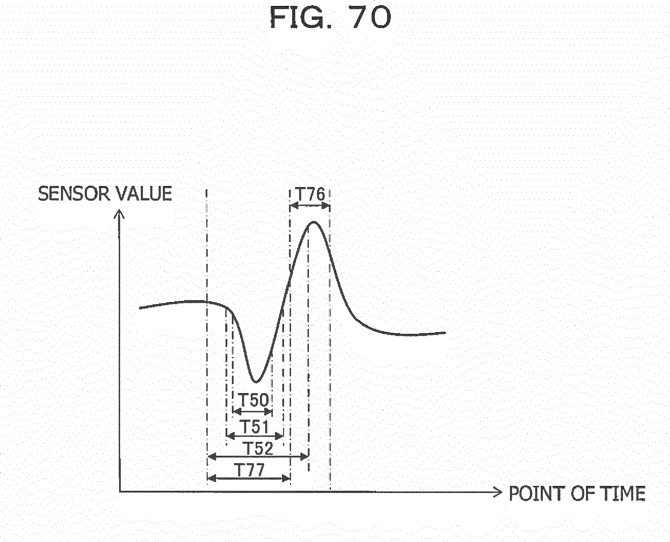

In Step S6710, the transitional period detection unit 15 specifies a transitional period based on the difference between the second period and the focused strong action period (first period), and a difference between the same second period and the third period. For example, the transitional period detection unit 15 specifies a period obtained by subtracting an overlapping portion of the focused strong action period (first period) which is selected in Step S1306, from the second period obtained in Step S6703, as a first period of the transitional period. The transitional period detection unit 15 specifies a period obtained by subtracting an overlapping portion of the second period which is obtained in Step S6703, from the third period obtained in Step S6708, as a second period of the transitional period. The transitional period detection unit 15 specifies a period obtained by linking the first period and the second period, as the transitional period. The second period of the transitional period may include the whole first period of the transitional period by using the specifying method of the third period. In this case, the transitional period detection unit 15 may specify a period obtained by subtracting an overlapping portion of the focused strong action period (first period) which is selected in Step S1306, from the third period obtained in Step S6708, as the transitional period. For example, in the example illustrated in FIG. 70, a period obtained by subtracting the focused strong action period (first period) T76 from a third period T52 is specified as the transitional period.

In the example illustrated in FIGs. 64 to 66, the method of specifying the transitional period ahead of the standing action is described. However, a method of specifying the transitional period after a movement which is not stably repeated, such as the standing action may be also applied.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

Hitherto, details of the example are described, but it is not limited to the specific example, and various deformations and various changes may be made in a range described in claims. All or some of the components of the above-described example may be combined.

For example, in the above-described specifying method of the transitional period, the transitional period is specified separately from the stable period. However, the specifying method of the transitional period may be used for detecting an action period obtained by combining the transitional period and the stable period (or the strong action period). In this case, the transitional period may be also specified with high accuracy by using the above-described specifying method of the transitional period. Thus, it is possible to detect the action period with high accuracy.

REFERENCE SIGNS LIST

1 MEASUREMENT SYSTEM
10, 10A, 10B MEASURING APPARATUS
11, 11B STABLE PERIOD DETECTION UNIT
12, 12B SLIGHTLY-STABLE PERIOD-DETECTING PARAMETER DETERMINATION UNIT
13, 13B SLIGHTLY-STABLE PERIOD ENUMERATION UNIT
14 SLIGHTLY-STABLE PERIOD SELECTION UNIT
15 TRANSITIONAL PERIOD DETECTION UNIT
16 FEATURE QUANTITY CALCULATION UNIT
20, 20B STABLE PERIOD DETECTION ALGORITHM DATABASE
21 SECOND THRESHOLD CANDIDATE DATABASE
22 SLIGHTLY-STABLE PERIOD SELECTION INDEX DATABASE
23 FEATURE QUANTITY DATABASE
24 SLIGHTLY-STABLE PERIOD DETECTION PARAMETER DATABASE
32 SENSOR DATA ACQUISITION UNIT
34 SENSOR DATA STORAGE UNIT

**Claims**

1. A specifying method executed by a processor, the specifying method comprising:

specifying a first period (S1302) for which it is determined that a specific movement or a specific posture is being performed by applying a first threshold relating to a given type of parameter to time series data obtained from a sensor for detecting a movement of a person;
specifying a second period (S1310, S1312) for which it is determined that the specific movement or the specific posture is being performed by applying a second threshold to the time series data, the second threshold relating to the same type of parameter as the first threshold or relating to a different type of parameter from that of the first threshold; and
specifying a transitional period (S1314) based on a difference between the second period and the first period, the transitional period corresponding to one of a period of a transition to the specific movement or the specific posture, and another period of another transition from the specific movement or the specific posture to another movement or another posture,

wherein:

when the first and second thresholds relate to the same type of parameter, the second threshold is set relative to the first threshold such that the second period includes the whole first period and a period which is not included in the first period; and
when the first and second thresholds relate to different types of parameter, the second threshold is set relative to the first threshold such that the second period includes at least a portion of the first period and a period which is not included in the first period; and
wherein the specifying of the second period includes selecting one second period candidate as the second period, from second period candidates for which it is determined that the specific movement or posture is being performed, when second threshold candidates are changed based on the parameter for the second threshold and the changed second threshold candidates are applied to the time series data, the selection of the one second period candidate as the second period being based on a specific rule, the specific rule including whether or not characteristics of the time series data in the second period candidate satisfy a given specific constraint condition based on features during gait initiation, the specific constraint condition including that a changing quantity of an angle between the leg and the orthogonal to the ground at a first step during the second period candidate is smaller than a changing quantity of said angle at a second step during the same second period candidate.

2.  The specifying method according to claim 1, further comprising:

    determining (S1308; S1400 - S1406) a parameter, the value of which in the time series data becomes closer to the values of the same parameter in the first period as the time of the time series data becomes closer to the time of the first period; and
    selecting the determined parameter as the parameter used to specify the second threshold,
    wherein the second threshold is set based on that determined parameter for the second threshold.

3.  The specifying method according to claim 1, further comprising:

    determining a parameter for specifying the second period which is longer than the first period, as a parameter for the second threshold,
    wherein the second threshold is set based on the parameter for the second threshold.

4.  The specifying method according to claim 2 or 3,
    wherein the determining of the parameter for the second threshold includes selecting a parameter (S1406) from plural types of parameters.

5.  The specifying method according to claim 1,
    wherein the specific rule relates to at least one of the period length of the second period candidate, whether or not the second period candidate includes another first period, whether or not the second period candidate includes a time when the sensor detects noise related to a change in the surrounding environment of the person, whether or not the second period candidate includes a second period detected based on a sensor which is different from the sensor, and whether or not characteristics of the time series data in the second period candidate satisfy a given specific constraint condition.

6.  The specifying method according to any preceding claim,
    wherein the specific movement or posture is gait.

7.  The specifying method according to claim 1,
    wherein the specific movement or posture is a standing position or a sitting position,
    wherein the specific rule includes whether or not the characteristics of time series data in an extension period satisfy the specific constraint condition, when a period obtained by extending the second period candidate due to a change for which the second threshold candidate is changed in the mitigation direction is set as the extension period,
    wherein the specific constraint condition includes that a difference is equal to or less than a specific value, the difference being between:

    characteristics relating to the extension period; and
    characteristics in the extension period which is changed by a change occurring when the second threshold candidate relating to the extension period is further changed.

8.  The specifying method according to any of claims 1 to 7,
    wherein the specific movement follows a stable movement which is periodically repeated.

9.  The specifying method according to any of claims 1 to 5,
    wherein the specific movement or posture is a movement which is not stably repeated.

10. The specifying method according to claim 9, further comprising:

    determining a parameter having a tendency of the value of the time series data becoming closer to a value in a period of which an initiation time of the second period is set as the center, with being close to the initiation time of the second period, as a parameter for a third threshold; and
    specifying a third period for which it is determined that the specific movement is being performed, when a third threshold which is based on the parameter for the third threshold and has a type different from the type of the second threshold is applied to the time series data,
    wherein the transitional period is specified based on the difference, and a difference between the second period and the third period.

**11.** A specifying apparatus for specifying a transitional period when transition to a specific movement or a specific posture is performed by a person or when transition is performed from the specific movement or the specific posture to another movement or another posture by the person, the specifying apparatus comprising:

an acquiring unit (32) configured to acquire time series data including a value, from a sensor for detecting the value in accordance with various movements of a person;

a first specifying unit (11) configured to specify a first period for which it is determined that the specific movement or the specific posture is being performed by applying a first threshold relating to a given type of parameter to the time series data;

a second specifying unit (12, 13, 14) configured to specify a second period for which it is determined that the specific movement or the specific posture is being performed by applying a second threshold to the time series data, the second threshold relating to the same type of parameter as the first threshold or relating to a different type of parameter from that of the first threshold;

a third specifying unit (15) configured to specify the transitional period based on a difference between the second period and the first period; and

an outputting unit (16) configured to output information regarding the transitional period,

wherein:

when the first and second thresholds relate to the same type of parameter, the second threshold is set relative to the first threshold such that the second period includes the whole first period and a period which is not included in the first period; and

when the first and second thresholds relate to different types of parameter, the second threshold is set such that the second period includes at least a portion of the first period and a period which is not included in the first period; and

wherein second specifying unit (12, 13, 14) is configured to specify the second period by selecting one second period candidate as the second period, from second period candidates for which it is determined that the specific movement or posture is being performed, when second threshold candidates are changed based on the parameter for the second threshold and the changed second threshold candidates are applied to the time series data, the selection of the one second period candidate as the second period being based on a specific rule, the specific rule including whether or not characteristics of the time series data in the second period candidate satisfy a given specific constraint condition based on features during gait initiation, the specific constraint condition including that a changing quantity of an angle between the leg and the orthogonal to the ground at a first step during the second period candidate is smaller than a changing quantity of said angle at a second step during the same second period candidate.

**Patentansprüche**

**1.** Spezifizierungsverfahren, das von einem Prozessor ausgeführt wird, wobei das Spezifizierungsverfahren Folgendes umfasst:

Spezifizieren einer ersten Periode (S1302), für die bestimmt wird, dass eine spezifische Bewegung oder eine spezifische Haltung durchgeführt wird, durch Anwenden eines ersten Schwellwerts, der einen gegebenen Parametertyp betrifft, auf Zeitseriendaten, die von einem Sensor erhalten werden, zum Detektieren einer Bewegung einer Person;

Spezifizieren einer zweiten Periode (S1310, S1312), für die bestimmt wird, dass die spezifische Bewegung oder die spezifische Haltung durchgeführt wird, durch Anwenden eines zweiten Schwellwerts auf die Zeitseriendaten, wobei der zweite Schwellwert denselben Parametertyp betrifft wie der erste Schwellwert oder einen Parametertyp betrifft, der sich vom ersten Schwellwert unterscheidet; und

Spezifizieren einer Übergangsperiode (S1314) auf Basis einer Differenz zwischen der zweiten Periode und der ersten Periode, wobei die Übergangsperiode einer von einer Periode eines Übergangs zur spezifischen Bewegung oder zur spezifischen Haltung und einer anderen Periode eines anderen Übergangs von der spezifischen Bewegung oder der spezifischen Haltung zu einer anderen Bewegung oder einer anderen Haltung entspricht,

wobei:

wenn der erste und der zweite Schwellwert denselben Parametertyp betreffen, der zweite Schwellwert relativ zum ersten Schwellwert derart eingestellt wird, dass die zweite Periode die ganze erste Periode und eine Periode, die in der ersten Periode nicht beinhaltet ist, beinhaltet; und

wenn der erste und der zweite Schwellwert verschiedene Parametertypen betreffen, der zweite Schwellwert relativ zum ersten Schwellwert derart eingestellt wird, dass die zweite Periode mindestens einen Abschnitt der ersten Periode und eine Periode, die in der ersten Periode nicht beinhaltet ist, beinhaltet; und

wobei das Spezifizieren der zweiten Periode das Auswählen eines Zweitperiodenkandidaten als die zweite Periode aus Zweitperiodenkandidaten, für die bestimmt wird, dass die spezifische Bewegung oder Haltung durchgeführt wird, wenn Zweitschwellwertkandidaten auf Basis des Parameters für den zweiten Schwellwert geändert werden und die geänderten Zweitschwellwertkandidaten auf die Zeitseriendaten angewendet werden, beinhaltet, wobei die Auswahl des einen Zweitperiodenkandidaten als die zweite Periode auf einer spezifischen Regel basiert, wobei die spezifische Regel beinhaltet, ob Eigenschaften der Zeitseriendaten im Zweitperiodenkandidaten auf Basis von Merkmalen während einer Ganginitiierung eine gegebene spezifische Einschränkungsbedingung erfüllen oder nicht, wobei die spezifische Einschränkungsbedingung beinhaltet, dass ein sich ändernder Betrag eines Winkels zwischen dem Bein und der Orthogonalen zum Boden bei einem ersten Schritt während des Zweitperiodenkandidaten kleiner ist als ein sich ändernder Betrag des Winkels bei einem zweiten Schritt während desselben Zweitperiodenkandidaten ist.

2. Spezifizierungsverfahren nach Anspruch 1, das ferner Folgendes umfasst:

Bestimmen (S1308; S1400-S1406) eines Parameters, dessen Wert in den Zeitseriendaten sich den Werten desselben Parameters in der ersten Periode annähert, wenn sich die Zeit der Zeitseriendaten der Zeit der ersten Periode annähert; und

Auswählen des bestimmten Parameters als den Parameter, der zum Spezifizieren des zweiten Schwellwerts verwendet wird,

wobei der zweite Schwellwert auf Basis dieses bestimmten Parameters für den zweiten Schwellwert eingestellt wird.

3. Spezifizierungsverfahren nach Anspruch 1, das ferner Folgendes umfasst:

Bestimmen eines Parameters zum Spezifizieren der zweiten Periode, die länger ist als die erste Periode, als einen Parameter für den zweiten Schwellwert,

wobei der zweite Schwellwert auf Basis des Parameters für den zweiten Schwellwert eingestellt wird.

4. Spezifizierungsverfahren nach Anspruch 2 oder 3,

wobei das Bestimmen des Parameters für den zweiten Schwellwert das Auswählen eines Parameters (S1406) aus mehreren Parametertypen beinhaltet.

5. Spezifizierungsverfahren nach Anspruch 1,

wobei die spezifische Regel mindestens eines der Periodenlänge des Zweitperiodenkandidaten, ob der Zweitperiodenkandidat eine weitere erste Periode beinhaltet oder nicht, ob der Zweitperiodenkandidat eine Zeit, zu der der Sensor Geräusche detektiert, die eine Änderung der umliegenden Umgebung der Person betreffen, beinhaltet oder nicht, ob der Zweitperiodenkandidat eine zweite Periode, die auf Basis eines Sensors, der sich vom Sensor unterscheidet, detektiert wird, beinhaltet oder nicht und ob Eigenschaften der Zeitseriendaten im Zweitperiodenkandidaten eine gegebene spezifische Einschränkungsbedingung erfüllen oder nicht, betreffen.

6. Spezifizierungsverfahren nach einem der vorhergehenden Ansprüche,

wobei die spezifische Bewegung oder Haltung ein Gang ist.

7. Spezifizierungsverfahren nach Anspruch 1,

wobei die spezifische Bewegung oder Haltung eine Standposition oder eine Sitzposition ist,

wobei die spezifische Regel beinhaltet, ob die Eigenschaften von Zeitseriendaten in einer Verlängerungsperiode die spezifische Einschränkungsbedingung erfüllen oder nicht, wenn eine Periode, die durch Verlängern des Zweitperiodenkandidaten aufgrund einer Änderung, für die der Zweitschwellwertkandidat geändert wird, in die Minderungsrichtung erhalten wird, als die Verlängerungsperiode eingestellt wird,

wobei die spezifische Einschränkungsbedingung beinhaltet, dass eine Differenz einem spezifischen Wert gleich oder kleiner als dieser ist, wobei die Differenz zwischen Folgendem besteht:

Eigenschaften, die die Verlängerungsperiode betreffen; und

Eigenschaften in der Verlängerungsperiode, die durch eine Änderung geändert wird, die eintritt, wenn der Zweitschwellwertkandidat, der die Verlängerungsperiode betrifft, weiter geändert wird.

8. Spezifizierungsverfahren nach einem der Ansprüche 1 bis 7,
wobei die spezifische Bewegung einer stabilen Bewegung folgt, die regelmäßig wiederholt wird.

9. Spezifizierungsverfahren nach einem der Ansprüche 1 bis 5,
wobei die spezifische Bewegung oder Haltung eine Bewegung ist, die nicht stabil wiederholt wird.

10. Spezifizierungsverfahren nach Anspruch 9, das ferner Folgendes umfasst:

Bestimmen eines Parameters, der eine Tendenz aufweist, dass sich der Wert der Zeitseriendaten einem Wert, bei dem in einer Periode eine Initiierungszeit als die Mitte eingestellt ist, annähert, wobei die Nähe zur Initiierungszeit der zweiten Periode besteht, als einen Parameter für einen dritten Schwellwert und
Spezifizieren einer dritten Periode, für die bestimmt wird, dass die spezifische Bewegung durchgeführt wird, wenn ein dritter Schwellwert, der auf dem Parameter für den dritten Schwellwert basiert und einen Typ aufweist, der sich vom Typ des zweiten Schwellwerts unterscheidet, auf die Zeitseriendaten angewendet wird,
wobei die Übergangsperiode auf Basis der Differenz und einer Differenz zwischen der zweiten Periode und der dritten Periode spezifiziert wird.

11. Spezifizierungsvorrichtung zum Spezifizieren einer Übergangsperiode, wenn ein Übergang zu einer spezifischen Bewegung oder einer spezifischen Haltung von einer Person durchgeführt wird oder wenn ein Übergang von der spezifischen Bewegung oder der spezifischen Haltung zu einer anderen Bewegung oder einer anderen Haltung von der Person durchgeführt wird, wobei die Spezifizierungsvorrichtung Folgendes umfasst:

eine Erfassungseinheit (32), die dazu ausgelegt ist, Zeitseriendaten, die einen Wert beinhalten, zum Detektieren des Wertes gemäß unterschiedlichen Bewegungen einer Person von einem Sensor zu erfassen;
eine erste Spezifizierungseinheit (11), die dazu ausgelegt ist, eine erste Periode, für die bestimmt wird, dass die spezifische Bewegung oder die spezifische Haltung durchgeführt wird, durch Anwenden eines ersten Schwellwerts, der einen gegebenen Parametertyp betrifft, auf Zeitseriendaten zu spezifizieren;
eine zweite Spezifizierungseinheit (12, 13, 14), die dazu ausgelegt ist, eine zweite Periode, für die bestimmt wird, dass die spezifische Bewegung oder die spezifische Haltung durchgeführt wird, durch Anwenden eines zweiten Schwellwerts auf die Zeitseriendaten zu spezifizieren, wobei der zweite Schwellwert denselben Parametertyp betrifft wie der erste Schwellwert oder einen Parametertyp betrifft, der sich vom ersten Schwellwert unterscheidet;
eine dritte Spezifizierungseinheit (15), die dazu ausgelegt ist, die Übergangsperiode auf Basis einer Differenz zwischen der zweiten Periode und der ersten Periode zu spezifizieren; und
eine Ausgabeeinheit (16), die dazu ausgelegt ist, Informationen zur Übergangsperiode auszugeben,

wobei:

wenn der erste und der zweite Schwellwert denselben Parametertyp betreffen, der zweite Schwellwert relativ zum ersten Schwellwert derart eingestellt wird, dass die zweite Periode die ganze erste Periode und eine Periode, die in der ersten Periode nicht beinhaltet ist, beinhaltet; und
wenn der erste und der zweite Schwellwert verschiedene Parametertypen betreffen, der zweite Schwellwert derart eingestellt wird, dass die zweite Periode mindestens einen Abschnitt der ersten Periode und eine Periode, die in der ersten Periode nicht beinhaltet ist, beinhaltet; und
wobei die zweite Spezifizierungseinheit (12, 13, 14) dazu ausgelegt ist, die zweite Periode durch Auswählen eines Zweitperiodenkandidaten als die zweite Periode aus Zweitperiodenkandidaten, für die bestimmt wird, dass die spezifische Bewegung oder Haltung durchgeführt wird, wenn Zweitschwellwertkandidaten auf Basis des Parameters für den zweiten Schwellwert geändert werden und die geänderten Zweitschwellwertkandidaten auf die Zeitseriendaten angewendet werden, zu spezifizieren, wobei die Auswahl des einen Zweitperiodenkandidaten als die zweite Periode auf einer spezifischen Regel basiert, wobei die spezifische Regel beinhaltet, ob Eigenschaften der Zeitseriendaten im Zweitperiodenkandidaten auf Basis von Merkmalen während einer Ganginitiierung eine gegebene spezifische Einschränkungsbedingung erfüllen oder nicht, wobei die spezifische Einschränkungsbedingung beinhaltet, dass ein sich ändernder Betrag eines Winkels zwischen dem Bein und der Orthogonalen zum Boden bei einem ersten Schritt während des Zweitperiodenkandidaten kleiner ist als ein sich ändernder Betrag des Winkels bei einem zweiten Schritt während desselben Zweitperiodenkandidaten ist.

**Revendications**

1.  Procédé de spécification exécuté par un processeur, le procédé de spécification comprenant les étapes consistant à :

    spécifier une première période (S1302) pour laquelle il est déterminé qu'un mouvement spécifique ou une posture spécifique est effectué(e) en appliquant un premier seuil relatif à un type de paramètre donné à des données de série temporelle obtenues à partir d'un capteur pour détecter un mouvement d'une personne ;
    spécifier une deuxième période (S1310, S1312) pour laquelle il est déterminé que le mouvement spécifique ou la posture spécifique est en cours d'exécution en appliquant un deuxième seuil aux données de série temporelle, le deuxième seuil étant relatif au même type de paramètre que le premier seuil ou étant relatif à un type de paramètre différent de celui du premier seuil ; et
    spécifier une période de transition (S1314) sur la base d'une différence entre la deuxième période et la première période, la période de transition correspondant à une parmi une période d'une transition vers le mouvement spécifique ou la posture spécifique, et une autre période d'une autre transition à partir du mouvement spécifique ou de la posture spécifique à un autre mouvement ou une autre posture,

    dans lequel :

    lorsque les premier et deuxième seuils sont relatifs au même type de paramètre, le deuxième seuil est établi par rapport au premier seuil de sorte que la deuxième période comprend toute la première période et une période qui n'est pas comprise dans la première période ; et
    lorsque les premier et deuxième seuils sont relatifs à différents types de paramètre, le deuxième seuil est établi par rapport au premier seuil de sorte que la deuxième période comprend au moins une partie de la première période et une période qui n'est pas incluse dans la première période ; et
    dans lequel la spécification de la deuxième période comprend la sélection d'une candidate de deuxième période en tant que deuxième période, parmi des candidates de deuxième période pour lesquelles il est déterminé que le mouvement ou la posture spécifique est en cours d'exécution, lorsque des candidats de deuxième seuil sont modifiés sur la base du paramètre du deuxième seuil et lorsque les candidats de deuxième seuil modifiés sont appliqués aux données de série temporelle, la sélection de la candidate de deuxième période en tant que deuxième période étant basée sur une règle spécifique, la règle spécifique indiquant si des caractéristiques des données de série temporelle de la candidate de deuxième période satisfont ou non à une condition de contrainte spécifique donnée en fonction des caractéristiques lors de l'initiation de la démarche, la condition de contrainte spécifique comprenant le fait qu'une quantité changeante d'un angle entre la jambe et la perpendiculaire par rapport au sol lors d'une première étape au cours de la candidate de deuxième période est inférieure à une quantité changeante dudit angle lors d'une deuxième étape durant la même candidate de deuxième période.

2.  Procédé de spécification selon la revendication 1, comprenant en outre les étapes consistant à :

    déterminer (S1308 ; S1400- S1406) un paramètre dont la valeur dans la série de données temporelle se rapproche des valeurs du même paramètre dans la première période, lorsque le temps des données de série temporelle se rapproche du temps de la première période ; et
    sélectionner le paramètre déterminé en tant que paramètre utilisé pour spécifier le deuxième seuil,
    dans lequel le deuxième seuil est établi sur la base de ce paramètre déterminé pour le deuxième seuil.

3.  Procédé de spécification selon la revendication 1, comprenant en outre :

    la détermination d'un paramètre pour spécifier la deuxième période qui est plus longue que la première période, en tant que paramètre pour le deuxième seuil,
    dans lequel le deuxième seuil est défini sur la base du paramètre du deuxième seuil.

4.  Procédé de spécification selon la revendication 2 ou 3,
    dans lequel la détermination du paramètre pour le deuxième seuil comprend la sélection d'un paramètre (S1406) parmi plusieurs types de paramètres.

5.  Procédé de spécification selon la revendication 1, dans lequel la règle spécifique concerne au moins une des longueurs de période de la candidate de deuxième période, que la candidate de deuxième période comprenne ou non une autre première période, que la candidate de deuxième période comprenne ou non un temps où le capteur détecte un bruit lié à un changement dans l'environnement de la personne, que la candidate de deuxième période comprenne ou non une deuxième période détectée sur la base d'un capteur différent du capteur, et que des caractéristiques des données de série temporelle de la candidate de deuxième période satisfassent ou non à une condition de contrainte spécificité donnée.

6. Procédé de spécification selon l'une quelconque des revendications précédentes,
   dans lequel le mouvement ou la posture spécifique est une démarche.

7. Procédé de spécification selon la revendication 1, dans lequel le mouvement ou la posture spécifique est une position debout ou une position assise,
   dans lequel la règle spécifique indique si les caractéristiques des données de série temporelle d'une période d'extension satisfont ou non à la condition de contrainte spécifique, lorsqu'une période obtenue en étendant la candidate de deuxième période en raison d'une modification pour laquelle le candidat de deuxième seuil est modifié dans le sens d'atténuation est définie comme période d'extension,
   dans lequel la condition de contrainte spécifique comprend qu'une différence est égale ou inférieure à une valeur spécifique, la différence étant entre :

   des caractéristiques relatives à la période d'extension ; et
   des caractéristiques dans la période de prolongation qui sont modifiées par un changement survenant lorsque le candidat de deuxième seuil relatif à la période de prolongation est encore modifié.

8. Procédé de spécification selon l'une des revendications 1 à 7,
   dans lequel le mouvement spécifique suit un mouvement stable qui se répète périodiquement.

9. Procédé de spécification selon l'une des revendications 1 à 5,
   dans lequel le mouvement ou la posture spécifique est un mouvement qui ne se répète pas de manière stable.

10. Procédé de spécification selon la revendication 9, comprenant en outre les étapes consistant à :

    déterminer un paramètre ayant une tendance à ce que la valeur des données de série temporelle se rapproche d'une valeur dans une période pour laquelle un temps d'initiation de la deuxième période est défini comme centre, tout en étant proche du temps d'initiation de la deuxième période, en tant que paramètre pour un troisième seuil ; et
    spécifier une troisième période pour laquelle il est déterminé que le mouvement spécifique est en cours d'exécution lorsqu'un troisième seuil qui est basé sur le paramètre du troisième seuil et a un type différent du type du deuxième seuil est appliqué aux données de série temporelle,
    dans lequel la période de transition est spécifiée sur la base de la différence, et d'une différence entre la deuxième période et la troisième période.

11. Appareil de spécification pour spécifier une période de transition lorsque la transition vers un mouvement spécifique ou une posture spécifique est effectuée par une personne ou lorsqu'une transition entre le mouvement spécifique ou la posture spécifique vers un autre mouvement ou une autre posture est effectuée par la personne, l'appareil de spécification comprenant :

    une unité d'acquisition (32) configurée pour acquérir des données de série temporelle comprenant une valeur, à partir d'un capteur pour détecter la valeur en fonction de divers mouvements d'une personne ;
    une première unité de spécification (11) configurée pour spécifier une première période pour laquelle il est déterminé que le mouvement spécifique ou la posture spécifique est en cours d'exécution en appliquant un premier seuil relatif à un type donné de paramètre aux données de série temporelle ;
    une deuxième unité de spécification (12, 13, 14) configurée pour spécifier une deuxième période pour laquelle il est déterminé que le mouvement spécifique ou la posture spécifique est en cours d'exécution en appliquant un deuxième seuil aux données de série temporelle, le deuxième seuil étant relatif au même type de paramètre que le premier seuil ou relatif à un type de paramètre différent de celui du premier seuil ;
    une troisième unité de spécification (15) configurée pour spécifier la période de transition sur la base d'une différence entre la deuxième période et la première période ; et
    une unité de délivrance en sortie (16) configurée pour délivrer en sortie des informations concernant la période de transition,

dans lequel :

lorsque les premier et deuxième seuils sont relatifs au même type de paramètre, le deuxième seuil est établi par rapport au premier seuil de sorte que la deuxième période englobe la totalité de la première période et une période qui n'est pas comprise dans la première période ; et

lorsque les premier et deuxième seuils sont relatifs à différents types de paramètres, le deuxième seuil est établi de sorte que la deuxième période comprend au moins une partie de la première période et une période qui n'est pas comprise dans la première période ; et

dans lequel la deuxième unité de spécification (12, 13, 14) est configurée pour spécifier la deuxième période en sélectionnant une candidate de deuxième période en tant que deuxième période, parmi des candidates de deuxième période pour lesquelles il est déterminé que le mouvement ou la posture spécifique est en cours d'exécution, lorsque des candidats de deuxième seuil sont modifiés sur la base du paramètre pour le deuxième seuil et les candidats de deuxième seuil modifiés sont appliqués aux données de série temporelle, la sélection de la candidate de deuxième période en tant que deuxième période étant basée sur une règle spécifique, la règle spécifique indiquant si ou non des caractéristiques des données de série temporelle dans la candidate de deuxième période satisfont à une condition de contrainte spécifique donnée sur la base de caractéristiques lors de l'initiation de la démarche, la condition de contrainte spécifique comprenant qu'une quantité changeante d'un angle entre la jambe et la perpendiculaire par rapport au sol lors d'une première étape pendant la candidate de deuxième période est inférieure à une quantité changeante dudit angle lors d'une deuxième étape pendant la même candidate de deuxième période.

# FIG. 1

1

```
      ⌐2                    ⌐10
┌──────────────┐      ┌──────────────┐
│    SENSOR    │──────│  MEASURING   │
│              │      │  APPARATUS   │
└──────────────┘      └──────────────┘
```

FIG. 2

# FIG. 3

SENSOR VALUE

POINT OF TIME

# FIG. 4

GAIT INITIATION
(TRANSITIONAL STATE)    GAIT (PERIODICAL MOVEMENT)    STOPPING OF GAIT
(TRANSITIONAL STATE)

# FIG. 5A

SENSOR VALUE

POINT
OF TIME

# FIG. 5B

SENSOR VALUE

POINT
OF TIME

# FIG. 6

SENSOR VALUE

X1  X2  X3  X4

POINT OF TIME

Y1

2

Y2

Y3

# FIG. 7

# FIG. 8

# FIG. 9

STANDSTILL (STABLE STATE)

GAIT INITIATION (TRANSITIONAL STATE)

GAIT (STABLE STATE)

INITIATION OF TRANSITIONAL STATE

ENDING OF TRANSITIONAL STATE

# FIG. 10

STANDSTILL
(STABLE STATE)

GAIT
(STABLE STATE)

ENDING OF BEING
STANDSTILL

ENDING OF
TRANSITIONAL STATE

# FIG. 11

# FIG. 12

100

101

PROCESSOR

102

MAIN MEMORY

103

AUXILIARY
MEMORY

104

DRIVE DEVICE

106

NETWORK I/F
DEVICE

107

INPUT DEVICE

105

RECORDING
MEDIUM

# FIG. 13

START

ACQUIRE TIME SERIES DATA OF SENSOR VALUES — S1300

DETECT STABLE PERIOD — S1302

j ← 1 — S1304

SELECT j-TH STABLE PERIOD
(FOCUSED STABLE PERIOD) — S1306

DETERMINE PARAMETER (PARAMETER FOR SECOND
THRESHOLD) FOR DETECTING TRANSITIONAL PERIOD
RELATING TO THE FOCUSED STABLE PERIOD — S1308

ENUMERATE PLURALITY OF SEMI-STABLE PERIODS — S1310

SELECT SPECIFIC SEMI-STABLE PERIOD
(SECOND PERIOD) — S1312

SPECIFY TRANSITIONAL PERIOD BASED ON DIFFERENCE BETWEEN
SECOND PERIOD AND FOCUSED STABLE PERIOD (FIRST PERIOD) — S1314

j ← j+1 — S1316

S1318
j > NUMBER OF STABLE PERIODS?

NO

YES

CALCULATE FEATURE VALUE OF EACH OF TRANSITIONAL
PERIODS — S1320

END

# FIG. 14

```
┌─────────────────────────────────────────┐
│   ACQUIRE EASED VALUES OF SECOND THRESHOLD │
│  CANDIDATE RELATING TO EACH OF ALL TYPES OF│ ∽ S1400
│ PARAMETERS WHICH MAY BE USED IN DETECTING OF│
│         FOCUSED STABLE PERIOD              │
└─────────────────────────────────────────┘
                    │
                    ▼
┌─────────────────────────────────────────┐
│  DETECT SEMI-STABLE PERIOD BASED ON PLURALITY│
│  OF EASED VALUES, FOR EACH OF PLURAL TYPES OF│ ∽ S1402
│            PARAMETERS                      │
└─────────────────────────────────────────┘
                    │
                    ▼
┌─────────────────────────────────────────┐
│  CALCULATE INDEX VALUES INDICATING SIMILARITY│ ∽ S1404
│    INCREASE TENDENCY, FOR EACH PARAMETER   │
└─────────────────────────────────────────┘
                    │
                    ▼
┌─────────────────────────────────────────┐
│     DETERMINE PARAMETER HAVING STRONGEST   │
│ SIMILARITY INCREASE TENDENCY AS PARAMETER FOR│ ∽ S1406
│   SECOND THRESHOLD BASED ON INDEX VALUE    │
└─────────────────────────────────────────┘
```

FIG. 15A

FIG. 15B

# FIG. 16

# FIG. 17

INTERVAL

AMPLITUDE

PARAMETER $\theta$     PARAMETER $\varepsilon$     PARAMETER $\mu$

PARAMETER $\eta$     PARAMETER $\nu$     PARAMETER $\xi$

## FIG. 18

# FIG. 19

| SELECTION INDEX ID | SELECTION INDEX OF 1: MAXIMUM VALUE OF PERIOD LENGTH | SELECTION INDEX OF 2: MINIMUM VALUE OF PERIOD LENGTH | ⋯ | SELECTION INDEX OF 5: POINT OF TIME OF PREVIOUS STABLE PERIOD | SELECTION INDEX OF 6: POINT OF TIME OF SUBSEQUENT STABLE PERIOD | SELECTION INDEX OF 7: POINT OF TIME OF KNOWN STABLE PERIOD | SELECTION INDEX OF 8: POINT OF TIME OF CHANGE OF SURROUNDING ENVIRONMENT | SELECTION INDEX OF 9: CONSTRAINT CONDITION IN GAIT INITIATION | ⋯ |
|---|---|---|---|---|---|---|---|---|---|
| 1 | ◯ | × | ⋯ | × | × | × | × | × | ⋯ |
| 2 | × | ◯ | ⋯ | × | × | × | × | × | ⋯ |
| ⋯ | ⋯ | ⋯ | ⋯ | × | × | × | × | × | ⋯ |
| 3 | × | × | ⋯ | ◯ | ◯ | ⋯ | ⋯ | × | ⋯ |
| 4 | × | × | ⋯ | × | × | ◯ | × | × | ⋯ |
| 5 | × | × | ⋯ | × | × | × | ◯ | × | ⋯ |
| 6 | ◯ | × | ⋯ | × | × | × | × | ◯ | ⋯ |
| ⋯ | ⋯ | ⋯ | ⋯ | ⋯ | ⋯ | ⋯ | ⋯ | × | ⋯ |

EP 3 064 133 B1

FIG. 20A

FIG. 20B

FIG. 20C

FIG. 20D

FIG. 20E

FIG. 20F

FIG. 20G

# FIG. 21

| EASED VALUE ID | EASED VALUE OF PARAMETER $\theta$ | EASED VALUE OF PARAMETER $\varepsilon$ | ... | EASED VALUE OF PARAMETER $\xi$ | ... |
|---|---|---|---|---|---|
| 1 | $\theta_1$ | $\varepsilon_1$ | ... | $\xi_1$ | ... |
| 2 | $\theta_2$ | $\varepsilon_2$ | ... | $\xi_2$ | ... |
| 3 | $\theta_3$ | $\varepsilon_3$ | ... | $\xi_3$ | ... |
| 4 | $\theta_4$ | $\varepsilon_4$ | ... | $\xi_4$ | ... |
| ... | ... | ... | ... | ... | ... |

# FIG. 22A

PARAMETER: $\theta$

$L_{\theta_3}$

$L_{\theta_2}$

$L_{\theta_1}$

T20

SENSOR VALUE

POINT OF TIME

# FIG. 22B

LENGTH OF PERIOD $L_\theta$

$L_{\theta_3}$ $L_{\theta_2}$ $L_{\theta_1}$

$\theta_1$ $\theta_2$ $\theta_3$

$\theta$

# FIG. 23A

# FIG. 23B

# FIG. 24A

# FIG. 24B

FIG. 25A

FIG. 25B

SENSOR VALUE

POINT OF TIME

| POINT OF TIME | SENSOR VALUE |
|---|---|
| 13:00:00 | 0 |
| 13:00:01 | 3.42 |
| 13:00:02 | -1.25 |
| 13:00:03 | 21.2 |
| . . . | . . . |
| 13:00:30 | 22.32 |
| 13:00:31 | 53.74 |
| 13:00:32 | 72.89 |
| . . . | . . . |
| 14:00:00 | 3.4 |
| . . . | . . . |

EP 3 064 133 B1

## FIG. 26

| DETECTION ALGORITHM ID | PARAMETER 1: PEAK VALUE (THRESHOLD) | PARAMETER 2: PEAK INTERVAL (THRESHOLD) | PARAMETER 3: PEAK AMPLITUDE (THRESHOLD) | PARAMETER 4: PEAK GRADIENT (THRESHOLD) | PARAMETER 5: AUTOCORRELATION COEFFICIENT (THRESHOLD) | ... |
|---|---|---|---|---|---|---|
| 1 | ○ (80) | ○ (50) | × (25) | × | × | ... |
| 2 | × | ○ (55) | × | × | × | ... |
| ... | ... | ... | ... | ... | ... | ... |

EP 3 064 133 B1

# FIG. 27

| DETECTION ALGORITHM ID | PARAMETER a: AMPLITUDE (THRESHOLD) | PARAMETER b: DISTRIBUTION (THRESHOLD) | PARAMETER c: AVERAGE VALUE (THRESHOLD) | ⋯ |
|---|---|---|---|---|
| 10 | O (10) | × | × | ⋯ |
| 11 | × | O (5) | × | ⋯ |
| ⋯ | ⋯ | ⋯ | ⋯ | ⋯ |

# FIG. 28

| POINT OF TIME | SENSOR VALUE | DETECTION ALGORITHM ID 1 | | | SENSOR VALUE |
|---|---|---|---|---|---|
| | | IS IT PEAK? | IS PARAMETER 1 (PEAK VALUE) EQUAL TO OR GREATER THAN THRESHOLD (80)? | IS PARAMETER 2 (PEAK INTERVAL) EQUAL TO OR GREATER THAN THRESHOLD (50)? | |
| ... | ... | ... | ... | ... | ... |
| 13:00:03 | 21.2 | ○ | × | × | × |
| ... | ... | ... | ... | ... | ... |
| 13:00:30 | 22.32 | × | − | − | × |
| 13:00:31 | 53.74 | × | − | − | × |
| 13:00:32 | 72.89 | ○ | ○ | ○ | ○ |
| ... | ... | ... | ... | ... | ... |

# FIG. 29

| STABLE PERIOD ID | INITIATION TIME OF STABLE PERIOD | ENDING POINT OF TIME OF STABLE PERIOD |
|---|---|---|
| (1) | 13:00:32 | 13:00:38 |
| (2) | 13:05:01 | 13:05:09 |
| (3) | 13:21:22 | 13:21:36 |
| . . . | . . . | . . . |

# FIG. 30A

| EASED VALUE ID | EASED VALUE OF PARAMETER 1 | EASED VALUE OF PARAMETER 2 | . . . | EASED VALUE OF PARAMETER 10 | . . . |
|---|---|---|---|---|---|
| 1 | 80 | 50 | . . . | 1.0 | . . . |
| 2 | 75 | 45 | . . . | 0.9 | . . . |
| 3 | 70 | 40 | . . . | 0.8 | . . . |
| 4 | 65 | 35 | . . . | 0.7 | . . . |
| . . . | . . . | . . . | . . . | . . . | . . . |

# FIG. 30B

| EASED VALUE ID | PARAMETER a: AMPLITUDE (THRESHOLD) | PARAMETER b: DISTRIBUTION (THRESHOLD) | . . . |
|---|---|---|---|
| 1 | 10 | 5 | . . . |
| 2 | 5 | 4 | . . . |
| . . . | . . . | . . . | . . . |

# FIG. 31A

| EASED VALUE ID | THRESHOLD | INITIATION TIME OF SEMI-STABLE PERIOD | INITIATION TIME OF SEMI-STABLE PERIOD | PERIOD LENGTH |
|---|---|---|---|---|
| 1 | 80 | 13:00:32 | 13:00:38 | 6 |
| 2 | 75 | 13:00:28 | 13:00:41 | 13 |
| 3 | 70 | 13:00:25 | 13:00:41 | 16 |
| 4 | 65 | 13:00:22 | 13:00:44 | 22 |
| . . . | . . . | . . . | . . . | . . . |

# FIG. 31B

| EASED VALUE ID | THRESHOLD | INITIATION TIME OF SEMI-STABLE PERIOD | INITIATION TIME OF SEMI-STABLE PERIOD | PERIOD LENGTH |
|---|---|---|---|---|
| 1 | 50 | 13:00:32 | 13:00:38 | 6 |
| 2 | 45 | 13:00:32 | 13:00:38 | 6 |
| 3 | 40 | 13:00:32 | 13:00:38 | 6 |
| 4 | 35 | 13:00:32 | 13:00:38 | 6 |
| . . . | . . . | . . . | . . . | . . . |

# FIG. 31C

| EASED VALUE ID | THRESHOLD | INITIATION TIME OF SEMI-STABLE PERIOD | INITIATION TIME OF SEMI-STABLE PERIOD | PERIOD LENGTH |
|---|---|---|---|---|
| 1 | 1.0 | 13:00:32 | 13:00:38 | 6 |
| 2 | 0.9 | 13:00:26 | 13:00:38 | 12 |
| 3 | 0.8 | 13:00:31 | 13:00:38 | 7 |
| 4 | 0.7 | 13:00:22 | 13:00:41 | 9 |
| . . . | . . . | . . . | . . . | . . . |

FIG. 32

| PARAMETER | EVALUATION VALUE |
|---|---|
| PARAMETER 1 | 1 |
| PARAMETER 2 | 0 |
| ... | ... |
| PARAMETER 10 | -0.1 |
| ... | ... |

# FIG. 33

| SEMI-STABLE PERIOD ID | INITIATION TIME OF SEMI-STABLE PERIOD | INITIATION TIME OF SEMI-STABLE PERIOD |
|---|---|---|
| ( i ) | 13:00:32 | 13:00:38 |
| ( ii ) | 13:00:28 | 13:00:41 |
| ( iii ) | 13:00:25 | 13:00:41 |
| ( iv ) | 13:00:22 | 13:00:44 |

# FIG. 34

# FIG. 35

FIG. 36

# FIG. 37

# FIG. 38

S3800

IS FOCUSED
STABLE PERIOD STABLE PERIOD
STABLE PERIOD RELATING TO
GAIT?

NO

YES ——— S3804

CALCULATE CHANGING ANGLE OF LEG
AT FIRST STEP IN EACH SEMI-STABLE
PERIOD

S3806

CALCULATE CHANGING ANGLE OF LEG
AT THE SECOND STEP IN EACH SEMI-
STABLE PERIOD

S3808

EXTRACT SEMI-STABLE PERIOD IN
WHICH CHANGING ANGLE OF LEG AT
FIRST STEP IS SMALLER THAN THAT
AT SECOND STEP

S3810

SELECT SEMI-STABLE PERIOD
(SECOND PERIOD) HAVING MAXIMUM
PERIOD LENGTH

S3802

SELECT SEMI-STABLE PERIOD BASED ON
SELECTION INDEX IN ACCORDANCE WITH
MOVEMENT AND THE LIKE RELATING TO
FOCUSED STABLE PERIOD

EP 3 064 133 B1

# FIG. 39

69

# FIG. 40

| SEMI-STABLE PERIOD ID | INITIATION TIME OF SEMI-STABLE PERIOD | INITIATION TIME OF SEMI-STABLE PERIOD | PERIOD LENGTH |
|---|---|---|---|
| 41 | 16:10:38 | 16:10:48 | 10 |
| 42 | 16:10:34 | 16:10:54 | 20 |
| 43 | 16:10:23 | 16:11:02 | 39 |
| 44 | 16:10:15 | 16:11:10 | 55 |
| 45 | 16:10:00 | 16:11:10 | 70 |

# FIG. 41

FIG. 42A

SENSOR VALUE

X1

SEMI-STABLE PERIOD

PERIOD OF ID = 44

POINT OF TIME

FIG. 42B

SENSOR VALUE

X1

SEMI-STABLE PERIOD

PERIOD OF ID = 45

POINT OF TIME

FIG. 43A

SENSOR VALUE

SEMI-STABLE PERIOD

X2

PERIOD OF ID = 44

POINT OF TIME

FIG. 43B

SENSOR VALUE

SEMI-STABLE PERIOD

X2

PERIOD OF ID = 45

POINT OF TIME

# FIG. 44

FIG. 45A

FIG. 45B

FIRST ONE STEP

β

# FIG. 46A          FIG. 46B

### SUBSEQUENT TO SECOND STEP

# FIG. 47

| SEMI-STABLE PERIOD ID | ANGLE OF LEG AT FIRST ONE STEP | ANGLE OF LEG AT SECOND STEP | DETERMINATION OF CONSTRAINT IN GAIT INITIATION |
|---|---|---|---|
| 41 | 23 | 22 | × |
| 42 | 21 | 19 | × |
| 43 | 15 | 16 | ○ |
| 44 | 10 | 13 | ○ |
| 45 | 32 | 10 | × |

# FIG. 48

| SEMI-STABLE PERIOD ID | DETERMINATION OF CONSTRAINT IN GAIT INITIATION | PERIOD LENGTH |
|---|---|---|
| 41 | × | 10 |
| 42 | × | 20 |
| 43 | ○ | 39 |
| 44 | ○ | 55 |
| 45 | × | 70 |

FIG. 49

# FIG. 50

START

DETECT STABLE PERIOD ～ S5002

CLASSIFY PLURALITY OF STABLE PERIODS
BY EACH ATTRIBUTE ～ S5003

$i \leftarrow 1$ ～ S5004

SELECT ALL STABLE PERIODS WHICH
RELATE TO ATTRIBUTE (FOCUSED
ATTRIBUTE) HAVING ATTRIBUTE NUMBER i ～ S5006

DETERMINE PARAMETER (PARAMETER
FOR THE SECOND THRESHOLD) FOR
DETECTING STABLE PERIOD WHICH
RELATES TO FOCUSED ATTRIBUTE ～ S5008

$i \leftarrow i+1$ ～ S5010

S5012

NO

i> NUMBER OF ATTRIBUTES?

YES

STORE PARAMETER FOR SECOND THRESHOLD
FOR EACH ATTRIBUTE IN SEMI-STABLE
PERIOD DETECTION PARAMETER DB ～ S5014

END

# FIG. 51

```
ACQUIRE EASED VALUE OF SECOND THRESHOLD CANDIDATE
RELATING TO EACH OF ALL TYPES OF PARAMETERS WHICH       ~ S5100
MAY BE USED IN DETECTING OF STABLE PERIOD WHICH
RELATES TO FOCUSED ATTRIBUTE
```

$j \leftarrow 1$ ~ S5102

SELECT j-TH STABLE PERIOD (FOCUSED STABLE PERIOD) ~ S5104

DETECT SEMI-STABLE PERIOD BASED ON PLURALITY OF
EASED VALUES, FOR EACH PARAMETER ~ S5106

CALCULATE INDEX VALUE INDICATING STRENGTH OF
SIMILARITY INCREASE TENDENCY, FOR EACH PARAMETER ~ S5108

$j \leftarrow j+1$ ~ S5110

S5112
NO        j> NUMBER OF STABLE PERIODS?

YES

SUM INDEX VALUES (CALCULATE SECOND INDEX VALUE)
FOR ALL OF THE STABLE PERIODS, FOR EACH PARAMETER ~ S5114

DETERMINE PARAMETER OF STRONGEST SIMILARITY
INCREASE TENDENCY AS PARAMETER FOR SECOND ~ S5116
THRESHOLD BASED ON SECOND INDEX VALUE

# FIG. 52

START

ACQUIRE TIME SERIES DATA OF SENSOR VALUES ～ S1300

DETECT STABLE PERIOD ～ S1302

j ← 1 ～ S1304

SELECT j-TH STABLE PERIOD
(FOCUSED STABLE PERIOD) ～ S1306

ACQUIRE PARAMETER FOR SECOND THRESHOLD
CORRESPONDING TO ATTRIBUTE OF FOCUSED STABLE
PERIOD, FROM SEMI-STABLE PERIOD DETECTION
PARAMETER DB ～ S5200

ENUMERATE PLURALITY OF SEMI-STABLE PERIODS ～ S5202

SELECT SPECIFIC SEMI-STABLE PERIOD (SECOND
PERIOD) ～ S1312

SPECIFY TRANSITIONAL PERIOD BASED ON
DIFFERENCE BETWEEN SECOND PERIOD AND
FOCUSED STABLE PERIOD (FIRST PERIOD) ～ S1314

j ← j+1 ～ S1316

NO  j > NUMBER OF STABLE PERIODS? S1318

YES

CALCULATE FEATURE VALUE OF EACH OF
TRANSITIONAL PERIODS ～ S1320

END

FIG. 53

# FIG. 54

SENSOR VALUE

POINT OF TIME

## FIG. 55

| DETECTION ALGORITHM | PARAMETER I: ABSOLUTE VALUE (THRESHOLD) | PARAMETER II: SPEED PEAK (THRESHOLD) | PARAMETER III: DISTRIBUTION (THRESHOLD) | ... |
|---|---|---|---|---|
| ( i ) | ○ | × | × | ... |
| (iii) | × | ○ | × | ... |
| ... | ... | ... | ... | ... |

# FIG. 56

# FIG. 57

FIG. 58

SENSOR VALUE

POINT OF TIME

# FIG. 59

| DETECTION ALGORITHM | PARAMETER A: ABSOLUTE VALUE (THRESHOLD) | PARAMETER BI: SPEED PEAK (THRESHOLD) | PARAMETER C: DISTRIBUTION (THRESHOLD) | ... |
|---|---|---|---|---|
| (a) | ○ | × | × | ... |
| (b) | × | ○ | × | ... |
| ... | ... | ... | ... | ... |

# FIG. 60

FIG. 61A

FIG. 61B

# FIG. 62

# FIG. 63

FIG. 64

# FIG. 65

SENSOR VALUE

POINT OF TIME

# FIG. 66

| DETECTION ALGORITHM | PARAMETER V-I: POSITIVE PEAK VALUE (THRESHOLD) | PARAMETER V-II: DIFFERENCE BETWEEN POSITIVE PEAK AND NEGATIVE PEAK (THRESHOLD) | PARAMETER V-III: DIFFERENCE BETWEEN MAXIMUM VALUE AND MINIMUM VALUE (THRESHOLD) | ・・・ |
|---|---|---|---|---|
| (5-1) | ○ | × | × | ・・・ |
| (5-2) | × | ○ | × | ・・・ |
| ・・・ | ・・・ | ・・・ | ・・・ | ・・・ |

# FIG. 67A

START

ACQUIRE TIME SERIES DATA OF SENSOR VALUES —— S1300

DETECT STABLE PERIOD —— S1302

$i \leftarrow 1$ —— S1304

FROM S1318 ——

SELECT j-TH STABLE PERIOD
(FOCUSED STABLE PERIOD) —— S1306

DETERMINE PARAMETER (PARAMETER FOR SECOND
THRESHOLD) FOR DETECTING TRANSITIONAL PERIOD
RELATING TO FOCUSED STABLE PERIOD —— S1308

ENUMERATE PLURALITY OF SEMI-STABLE PERIODS —— S1310

TO S6700

# FIG. 67B

TO S1306

FROM S6700

```
                                          ┌─────────────────────────┐ ⌐ S6700
                                          │      IS FOCUSED          │
                           NO             │  STABLE PERIOD STRONG    │
                    ◄──────────────────── │ ACTION PERIOD RELATING   │
                    │                     │  TO ACTION WHICH IS NOT  │
                    │                     │    STABLY REPEATED?      │
                    │                     └─────────────────────────┘
                    │                              │ YES
        ┌───────────────────────┐ ⌐ S6701    ┌─────────────────────────┐ ⌐ S6703
        │ SELECT SPECIFIC       │            │ SELECT WEAK ACTION       │
        │ SEMI-STABLE PERIOD    │            │ PERIOD HAVING LONGEST    │
        │ (SECOND PERIOD)       │            │ PERIOD LENGTH            │
        └───────────────────────┘            └─────────────────────────┘
                    │         ⌐ S6702                   │       ⌐ S6704
        ┌───────────────────────┐            ┌─────────────────────────┐
        │ SPECIFY TRANSITIONAL  │            │ DETERMINE PARAMETER      │
        │ PERIOD BASED ON       │            │ (PARAMETER FOR THIRD     │
        │ DIFFERENCE BETWEEN    │            │ THRESHOLD) FOR DETECTING │
        │ SECOND PERIOD AND     │            │ SPECIFIC TRANSITIONAL    │
        │ FOCUSED STABLE PERIOD │            │ PERIOD WHICH RELATES TO  │
        │ (FIRST PERIOD)        │            │ FOCUSED STRONG ACTION    │
        └───────────────────────┘            │ PERIOD                   │
                    │                        └─────────────────────────┘
                    │                                  │       ⌐ S6706
                    │                        ┌─────────────────────────┐
                    │                        │ ENUMERATE PLURALITY OF   │
                    │                        │ WEAK ACTION PERIODS,     │
                    │                        │ BASED ON PARAMETER FOR   │
                    │                        │ THIRD THRESHOLD          │
                    │                        └─────────────────────────┘
                    │                                  │       ⌐ S6708
                    │                        ┌─────────────────────────┐
                    │                        │ SELECT SPECIFIC WEAK     │
                    │                        │ ACTION PERIOD (THIRD     │
                    │                        │ PERIOD)                  │
                    │                        └─────────────────────────┘
                    │                                  │       ⌐ S6710
                    │                        ┌─────────────────────────┐
                    │                        │ SPECIFY TRANSITIONAL     │
                    │                        │ PERIOD BASED ON          │
                    │                        │ DIFFERENCE BETWEEN       │
                    │                        │ SECOND PERIOD AND STRONG │
                    │                        │ ACTION PERIOD (FIRST     │
                    │                        │ PERIOD), AND THIRD       │
                    │                        │ PERIOD                   │
                    │                        └─────────────────────────┘
```

S1316

$$j \leftarrow j+1$$

S1318

NO ◄─── j > NUMBER OF STABLE PERIODS?

│ YES    ⌐ S1320

CALCULATE FEATURE VALUE OF EACH OF TRANSITIONAL PERIODS

END

# FIG. 68

# FIG. 69

# FIG. 70

## REFERENCES CITED IN THE DESCRIPTION

**Patent documents cited in the description**

- JP 2011008612 A
- JP 2012113753 A
- WO 2013046510 A
- JP 2014044510 A
- JP 2005304942 A
- JP 2014036764 A
- US 20140330172 A1
- WO 2011055255 A1

**Non-patent literature cited in the description**

- **FRACCARO et al.** Real-world Gyroscope-based Gait Event Detection and Gait Feature Extraction. *eTELEMED,* 2014
- Analysis of gait initiation action of the young and the elderly. **SHIMAMURA et al.** Congress of the Japanese Physical Therapy Association. Japanese Physical Therapy Association, 2013, vol. 2012, 48101585-48101585